# EUROPÄISCHE PATENTSCHRIFT

(11) **EP 2 393 931 B1**
(45) Veröffentlichungstag und Bekanntmachung des Hinweises auf die Patenterteilung: **12.03.2014**
(21) Anmeldenummer: 10701339.3
(22) Anmeldetag: 13.01.2010
(51) Int. Cl.: C12P 13/00, C12P 17/18, C07D 493/04

(54) **VERFAHREN ZUR AMINIERUNG VON MULTIZYKLISCHEN KETONEN UNTER VERWENDUNG VON TRANSAMINASEN**
PROCESS FOR THE AMINATION OF MULTICYCLIC KETONES EMPLOYING TRANSAMINASES
PROCÉDÉ D'AMINATION DE CÉTONES MULTICYCLIQUES EN UTILISANT DES TRANSAMINASES

(30) Priorität: 04.02.2009 DE 102009000592
(43) Veröffentlichungstag der Anmeldung: 14.12.2011
(73) Patentinhaber: Evonik Degussa GmbH, 45128 Essen (DE)
(72) Erfinder: SIEBER, Volker, 85405 Nandlstadt (DE); GRAMMANN, Katrin, 45739 Oer-Erkenschwick (DE); RÜHMANN, Broder, 94315 Straubing (DE); HAAS, Thomas, 48161 Münster (DE); PFEFFER, Jan, Christoph, 45355 Essen (DE); DODERER, Kai, 63110 Rodgau (DE); ROLLMANN, Claudia, 63755 Alzenau (DE); SKERRA, Arne, 85354 Freising (DE); RAUSCH, Christian, 91575 Windsbach (DE); LERCHNER, Alexandra, 80992 München (DE)
(86) Internationale Anmeldenummer: PCT/EP2010/050331
(87) Internationale Veröffentlichungsnummer: WO 2010/089171

(56) Entgegenhaltungen:
- EP-A1- 2 022 852
- LUCHER, L.A.: "Reactions catalyzed by purified L-glutamine:keto-scyllo-inositol aminotransferase, an enzyme required for biosynthesis of aminocyclitol antibiotics", ANTIMICROBIAL AGENTS AND CHEMOTHERAPY, Bd. 33, Nr. 4, April 1989 (1989-04), Seiten 452-459, XP002622867,
- DATABASE CAPLUS [Online] CHEMICAL ABSTRACTS SERVICE; 2008, Baxter, E.W. & Reitz, A.B.: "Reductive aminations of carbonyl compounds with borohydride and borane reducing agents", XP002622692, Database accession no. 2008:1383655

## Beschreibung

### Gegenstand der Erfindung:

Gegenstand der Erfindung ist ein enzymatisches Verfahren zur Herstellung von Aminogruppen tragenden, multizyklischen Ringsystemen.

### Stand der Technik:

Diamino-dianhydro-dideoxy-hexitole sind als bifunktionelle Amine interessante Zwischenprodukte für chemische Umsetzungen. Vom 2,5-Diamino-1,4:3,6-Dianhydro-2,5-Dideoxy-D-Hexitol sind drei Stereoisomere beschrieben (z.B. Bashford, V. G. and Wiggins, L. F. (1950). Anhydrides of polyhydric alcohols. XIII. The amino derivatives of 1, 4:3, 6-dianhydromannitol, -sorbitol, and L-iditol and their behavior towards nitrous acid. Journal of the Chemical Society 1950 371-374.): 2,5-Diamino-1,4:3,6-Dianhydro-2,5-Dideoxy-D-Mannitol (I), 2,5-Diamino-1,4:3,6-Dianhydro-2,5-Dideoxy-D-Glucitol (II) und 2,5-Diamino-1,4:3,6-Dianhydro-2,5-Dideoxy-L-Iditol (III). Die drei Stereoisomere unterscheiden sich in der Chiralität an der Position 2 und 5. Die Aminogruppen können hier in der endo, endo (I), endo, exo (II) oder exo, exo (III) Position stehen, bezogen auf die Sesselform der annelierten fünfgliedrigen Ringe. 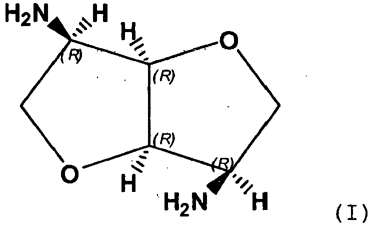 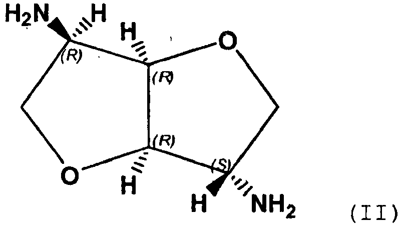 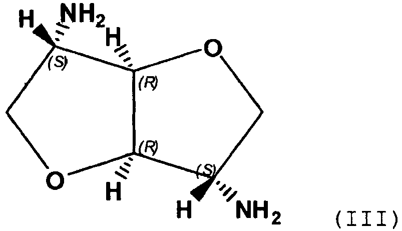 Erfolgreich dargestellt wurden diese Moleküle bisher aus den entsprechenden 1:4,3:6 Dianhydrohexitolen
1,4:3,6-Dianhydro-D-Mannitol, trivial Isomannid (IV) 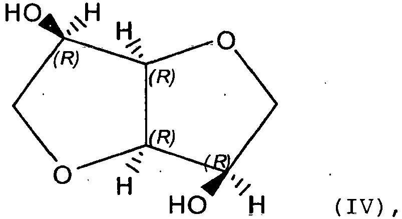 1,4:3,6-Dianhydro-D-Glucitol, trivial Isosorbid (V) 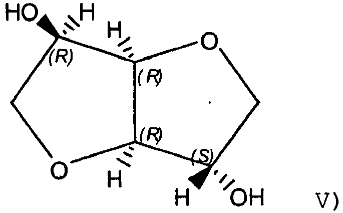 und
1,4:3,6-Dianhydro-L-Iditol, trivial Isoidid (VI) 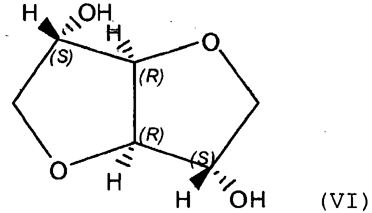 über
1.) Veresterung der Hydroxylgruppen (spez. Mesylierung oder Tosylierung) und
2.) Nucleophile Substitution der Säuregruppen mit Azid und nachfolgender Reduktion zum Diamin.
(Vgl. z.B.: Bashford, V. G. and Wiggins, L. F. (1950). Anhydrides of polyhydric alcohols. XIII. The amino derivatives of 1, 4:3, 6-dianhydromannitol, -sorbitol, and L-iditol and their behavior towards nitrous acid Journal of the Chemical Society 1950 371-374.; Thiem, J. and Bachmann, F. (1991). Synthesis and properties of polyamides derived from anhydro- and dianhydroalditols Makromolekulare Chemie 192 2163-2182.).

Reaktionsschema 1 stellt dies exemplarisch am Beispiel (IV) dar.

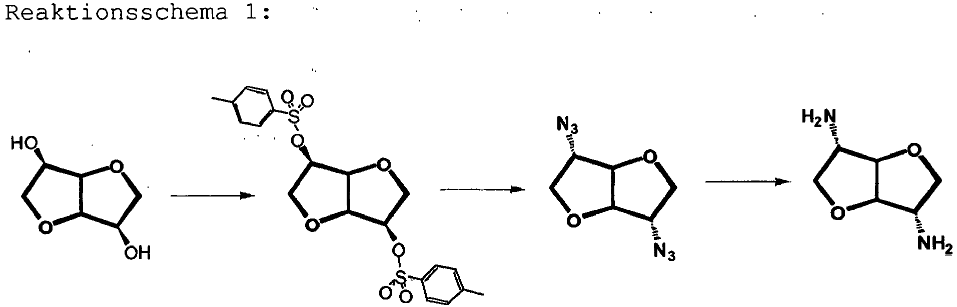

Verschiedene Modifikationen der dargestellten Route wurden getestet, jedoch alle ohne Erfolg:
A) Die Substitution wurde statt mit Azid direkt mit Ammoniak durchgeführt (Montgomery, R. and Wiggins, L. F. (1946). Anhydrides of polyhydric alcohols. V. 2,5-Diamino-1,4,3,6-dianhydromannitol and -sorbitol and their sulfanilamide derivatives Journal of the Chemical Society 1946 393-396.; Klessing, US 4535158; Klessing, DE3028288). Hierbei konnten nur sehr geringe Ausbeuten an Diamin erzielt werden.
B) Die Substitution erfolgte mit primären Aminen statt mit Azid. Auch hier waren die Ausbeuten gering und zudem wurden nur alkylierte Varianten der Diamine dargestellt (Hayashi, H.; Ueno, H.; Suzuki, F. (1992) Synthesis of stereoisomers of 1,4:3,6-dianhydrohexitol nitrate derivative, KF-14124. Bioorganic & Medicinal Chemistry Letters, 2(10), 1187-92.; Klessing, US 4535158; Klessing, DE3028288).
C) Als Alternative zur Reduktion wurde das Diazid mit Ketonen zum Imin umgesetzt und anschließend reduziert (De Coster, G., Vandyck K., Van der Eycken, E., Van der Eycken, J. Elseviers, M. and Röper, H. (2002) D-Isomannide in synthesis: asymmetric Diels-Alder reactions with novel homochiral bis-imine Cu2+-catalysts. Tetrahedron: Asymmetry 13 (2002) 1673-1679). Auch hier wurden allerdings nur alkylierte Varianten der Diamine erhalten.

All diese Routen gehen von den Stereoisomeren-reinen Anhydrohexitolen aus (IV bis VI), die in den einzelnen Reaktionsschritten unterschiedliche Reaktivität zeigen, und führen zu Stereoisomeren-reinen Produkten (I-III). Dabei kommt es über die Azid- Route zu einer Inversion der Stereochemie, aus dem endo, endo Diol (IV) wird zum Beispiel das exo, exo Diamin (III) (Cope, A. C. and Shen, T. Y. (1956). Stereochemistry of 1,4:3,6-dianhydrohexitol derivatives Journal of the American Chemical Society 78, 3177-3182.), so dass in der hier gebrauchten Bezeichnung z.B. zur Synthese des 2,5-Diamino-1,4:3,6-Dianhydro-2,5-Dideoxy-L-Iditols (III) 1,4:3,6-Dianhydro-D-Mannitol (IV) als Ausgangstoff verwendet werden muss.

Die beschriebenen Reaktionswege haben den Nachteil, dass sie entweder sehr ineffizient sind (Substitution des Ditosylats mit Ammoniak, Montgomery, R. and Wiggins, L. F. (1946). Anhydrides of polyhydric alcohols. V. 2,5-Diamino-1,4,3,6-dianhydromannitol and -sorbitol and their sulfanilamide derivatives Journal of the Chemical Society 1946 393-396.) oder sich nur sehr schwer im großtechnischen Maßstab umsetzen lassen, wie z. B. die Substitution mit Azid. Außerdem erhält man ausgehend von einem reinen Stereoisomer als Ausgangsstoff genau nur ein Stereoisomer als Produkt.

Dies ist insofern wichtig, als dass nur das Isosorbid (V) in großen Mengen und billig zur Verfügung steht und sich von diesem über die beschriebene Route nur Produkt (II) darstellen lässt. Interessant wäre es aber von diesem einen verfügbaren Ausgangsstoff (V) ausgehend, alle drei Amine (I-III) großtechnisch darstellen zu können.

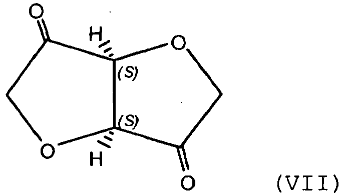

Die Darstellung der Diamine (I) bis (III) aus der Diulose (VII) konnte bisher noch nicht gezeigt werden.

Der CAPLUS Datenbankeintrag mit der Zugriffsnummer 2008:1383655 (BAXTER, E.W. & REITZ, A.B.: Reductive aminations of carbonyl compounds with borohydride and borane reducing agents", ORGANIC REACTIONS, 2002) offenbart ein Verfahren zur Herstellung der Diamine Diaminoisomannid (I), Diaminoisosorbid (II) und Diaminoisoidid (III) durch die chemische Aminierung mindestens einer Ketogruppe in einem entsprechenden mindestens eine Ketogruppe aufweisenden multizyklischen Ringsystem zu einer Aminogruppe.

Dargestellt werden konnte das am Stickstoff alkylierte Derivat des endo/endo-ständigen Diamins(I) (Limberg, G.; Thiem, J. (1994), Synthetic Approach to N-Alkylated 2,5-Diamino-2,5-didesoxy-1,4;3,6-dianhydroalditols by Reductive Alkylation. Synthesis; 1994 (3) 317-321.).

Es gibt in der Natur verschiedene Wege und damit Enzymklassen, Amine zu synthetisieren. Beispiele dieser Enzyme sind Transaminasen (auch Aminotransferasen genannt, EC 2.6.1.x), Aminosäuredehydrogenasen (EC 1.4.1.x) und Ammoniumlyasen (EC 4.3.1.x). Die typischen Produkte der Aminbildung sind dabei α-Aminosäuren ausgehend von α-Ketocarbonsäuren bzw. α-β-ungesättigten Carbonsäuren (Lyasen). In jüngerer Zeit wurden zudem auch sog. Aminoalkoholdehydrogenasen beschrieben (US6432688, WO0023608).

Transaminasen sind Pyridoxalphosphat(PLP)-abhängige Enzyme, die Aminogruppen von einem Molekül unter Bildung einer Oxo-Gruppe auf die Oxo-Gruppe eines zweiten Moleküls unter Bildung einer Aminogruppe übertragen. Damit hat jede Transaminase zumindest zwei Substrate: Den Aminodonor (*in vivo* typischerweise eine α-Aminosäure) und den Aminoakzeptor (siehe Schema). Für diese beiden Substrate besitzen Transaminasen in der Regel aber eine sehr hohe Spezifität.

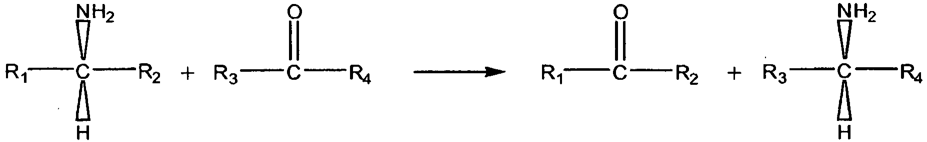

Wenn die Aminogruppe sich an einem chiralen Zentrum befindet, so ergeben die Reaktionen von Transaminasen typischerweise bevorzugt ein Enantiomer. Daher werden Transaminasen häufig zur chiralen Auflösung von racemischen Gemischen verwendet (z.B. Matcham, G. W. and Bowen, A. R. S. Biocatalysis for chiral intermediates: Meeting commercial and technical challenges. Chimica Oggi, 1996, 14(6), 20-24., US6344351, EP404146).

Eine spezielle Gruppe der Transaminasen sind die ω-Transaminasen. Diese haben den Vorzug, Aminogruppen auf Oxo-Gruppen übertragen zu können, die nicht durch benachbarte Carboxylgruppen aktiviert sind. Interessanterweise scheinen diese Enzyme aber vorrangig (S)-spezifisch zu sein (Shin und Kim, 2001, Comparison of the ω-Transaminase from Different Microorganisms and Application to Production of Chiral Amines Biosci. Biotechnol. Biochem. 65 (8): 1782-88).

Transaminasen sind sehr zahlreich und eine Reihe von Substraten und Produkten von Transaminasen sind beschrieben worden. So sind vor allem lineare aliphatische Ketone und aromatische Ketone aminiert worden. Einige wenige Transaminasen sind beschrieben worden, die monozyklische aliphatische Amine bilden können (Lynne A. Lucher, Yu-Ming Chen and James B. Walker (1989) Reactions Catalyzed by Purified L-Glutamine:Keto-Scyllo-Inositol Aminotransferase, an Enzyme Required for Biosynthesis of Aminocyclitol Antibiotics. Antimicrobial Agents and Chemotherapy, 1989, p. 452-459; Bum-Yeol Hwang, Hwa-Jin Lee, Yung-Hun Yang, Hwang-Soo Joo, and Byung-Gee Kim (2004) Characterization and Investigation of Substrate Specificity of the Sugar Aminotransferase WecE from E. coli K12. Chemistry & Biology, Vol. 11, p. 915-925). Es konnte hingegen noch nicht die Aminierung von annelierten multizyklischen aliphatischen Ketonen und speziell nicht von Ketonen in multizyklischen cisverknüpften aliphatischen Ringsystemen erreicht werden.

Aufgabe der Erfindung ist es, ein weiteres breit einsetzbares Verfahren bereitzustellen, mit welchem mindestens eine Aminogruppen tragende, multizyklische Ringsysteme hergestellt werden können. Eine weitere Aufgabe der Erfindung besteht darin, stereochemieunabhängige Synthesen bezüglich Edukten und/oder Produkten durchführen zu können.

### Beschreibung der Erfindung

Überraschenderweise wurde gefunden, dass eine enzymatische, durch Transaminasen katalysierte Umsetzung von multizyklischen Ketonverbindungen zu Enantiomerenmischungen multizyklischer Aminoverbindungen führt.

Dies ist für den Fachmann nicht vorhersehbar und wie oben aufgeführt völlig unerwartet, da diese enzymatischen Reaktionen in der Regel hochgradig stereospezifisch sein sollten und Enzyme in der Regel eine sehr hohe Substratspezifität aufweisen.

Des Weiteren wurde völlig überraschend gefunden, dass dieses Enantiomerenverhältnis durch den pH Wert während der Reaktion beeinflusst werden kann.

Überdies hinaus wurde völlig überraschend gefunden, dass die Transaminasen unterschiedlichste stereochemische Strukturen der multizyklischen Edukte als Substrat akzeptieren.

Mit erfindungsgemäßem Verfahren hergestellte Amine eignen sich insbesondere zur Herstellung von Epoxiden, Polyurethanen oder Polyamiden

Gegenstand der vorliegenden Erfindung ist daher ein Verfahren zur Aminierung mindestens einer Ketogruppe in einem mindestens eine Ketogruppe aufweisenden multizyklischen Ringsystem zu einer Aminogruppe unter Verwendung mindestens eines Enzyms E mit Transaminase-Aktivität, dadurch gekennzeichnet, dass als multizyklisches Ringsystem Verbindungen ausgewählt aus der Gruppe 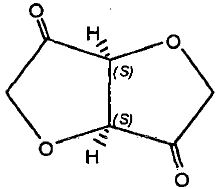 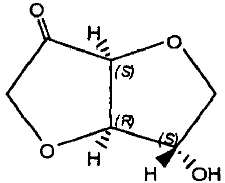 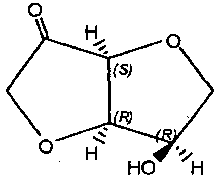 und als Enzym E eine Transaminase
eingesetzt werden.

Das erfindungsgemäße Verfahren zur Herstellung eines Aminogruppen tragenden, multizyklischen Ringsystems wird nachfolgend beispielhaft beschrieben.

Alle angegebenen Prozent (%) sind, wenn nicht anders angegeben, Massenprozent.

Unter einem "*multizyklischen Ringsystem*" sind Substanzen zu verstehen, die aus mindestens zwei miteinander kovalent verbundenen ringförmigen Molekülen aufgebaut sind.

Unter einem "*Wildtyp*" einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "*Wildtyp*" fallen daher insbesondere nicht solche Zellen bzw. solche Gene, deren Gensequenzen zumindest teilweise durch den Menschen mittels rekombinanter Verfahren verändert worden sind.

Der Begriff "*Überexpression*" beschreibt die Erhöhung der intrazellulären Aktivität oder Konzentration eines oder mehrerer Enzyme oder Proteine in einem Organismus, die durch die entsprechende DNA kodiert werden, indem man beispielsweise die Kopienzahl des Gens bzw. der Gene, des offenen Leserahmens (ORFs) bzw. der ORFs um mindestens eine Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert. In *E. coli* werden exemplarisch *lac*, *tac* und *trp* als starke Promotoren,genannt.

Als "*Transaminierung*" soll generell die Umwandlung eines Ketons zu einem Amin verstanden werden; anders als in der herkömmlichen Verwendung sollen nicht nur alpha-Ketonsäuren sondern generell alle Ketone als Edukte verstanden werden.

Als "*homologe Expression*" *oder* "*homologe Überexpression*" soll die Überexpression eines Proteins, welches vorher bereits in dem Mikroorganismus vorhanden war, verstanden werden.

Als "*heterologe Expression*" *oder* "*heterologe Überexpression*" soll die Expression bzw. Überexpression eines Proteins, welches vorher nicht in dem Mikroorganismus vorhanden war, verstanden werden.

Das Ringsystem kann jedes multizyklische Ringsystem sein, welches über mindestens eine Ketogruppe verfügt. Es kann eine Substanz sein, aufgebaut aus mehreren substituierten oder unsubstituierten, gesättigten oder ungesättigten, aliphatischen oder aromatischen Ringen; insbesondere handelt es sich um Substanzen, deren Ringe über eine molekulare Brücke verbunden sind oder in denen mindestens ein Atom am Aufbau von mindestens zwei Ringen gleichzeitig beteiligt ist. Die Ringe können reine Kohlenstoffringe oder Heterozyklen oder Mischungen aus beiden sein. Bevorzugtes Heteroatom in Heterozyklen ist Sauerstoff. Bevorzugt sind die Ringe, die ausschließlich Kohlenstoffatome aufweisen.

Als bevorzugte Substituenten am Ringsystem kommen kurzkettige (1 bis 5 C-Atome), gegebenenfalls weiter substituierte, Alkyreste oder Alkoxyreste in Frage.

Es werden solche multizyklischen Ringsysteme eingesetzt, die cis-verknüpfte Ringe aufweisen.

Die Anzahl der Ringe im Ringsystem liegt bei zwei oder drei Ringen. Es werden Ringsysteme mit zwei Ringen eingesetzt.

Die Ringgröße eines das Ringsystem aufbauenden Einzelringes beträgt 3 bis 9, 4 bis 7 oder 5 Atome.

Die Ketogruppe kann mit einem Ringkohlenstoff oder mit einem Substituentenkohlenstoff, d.h. mit einem Kohlenstoffatom, das nicht Bestanteil eines Ringes im Ringsystem ist, verbunden sein. Die Ketogruppe ist mit einem Ringkohlenstoff verbunden. Des Kohlenstoff der Ketogruppe ist Bestandteil eines der Ringe.

Bevorzugt werden in dem erfindungsgemäßen Verfahren als multizyklisches Ringsystem Verbindungen ausgewählt aus der Gruppe: 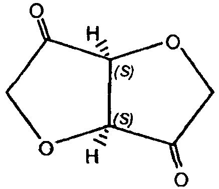 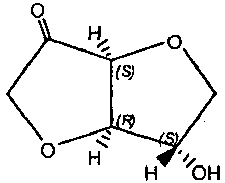 eingesetzt.

Es können Ketogruppen tragende, multizyklische Ringsysteme aller möglichen Herkunft eingesetzt werden.

In einer besonders bevorzugten Ausführungsform des erfindungsgemäßen Verfahrens wird ein Ketogruppen tragendes, multizyklisches Ringsystem eingesetzt, welches durch Oxidation mindestens einer sekundären Hydroxylgruppe eines multizyklischen Ausgangsringsystems zu einem Keton erhalten wird (Verfahrensschritt A).

Dies lässt sich beispielhaft durch Reaktionsschema 2 verbildlichen, wobei mit A gekennzeichnete Verfahrensschritte die Oxidation mindestens einer sekundären Hydroxylgruppe zu einer Ketogruppe darstellen, mit B gekennzeichnete Verfahrensschritte die Transaminierung. Wie gezeigt können Schritte A und B getrennt nacheinander aber auch abwechselnd ablaufen.

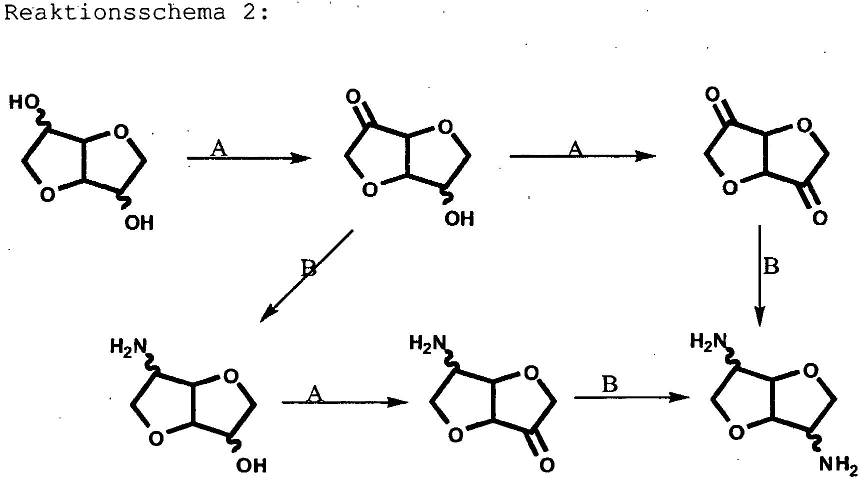

Bevorzugt steht in dem in Verfahrensschritt A eingesetzten mindestens eine sekundäre Hydroxylgruppe aufweisenden multizyklischen Ausgangsringsystem mindestens eine der sekundären Hydroxylgruppen endo-ständig zu dem multizyklischen Ausgangsringsystem. Gleichermaßen bevorzugt steht mindestens eine der sekundären Hydroxylgruppen exo-ständig zu dem multizyklischen Ausgangsringsystem.

Bevorzugt werden in Verfahrensschritt A als multizyklisches Ausgangsringsystem eingesetzt mindestens eine Verbindung ausgewählt aus der Gruppe:
Isomannid,
Isosorbid,
Isoidid,

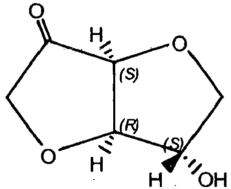

Zur Oxidation der sekundären Hydroxylgruppe in dem multizyklischen Ausgangsringsystem können sämtliche bekannten oxidierenden Verfahren eingesetzt werden, so z.B. elektrochemische, homogen oder heterogen katalytische oder enzymatische. Bevorzugt werden hier Verfahren der heterogen oder homogenen Katalyse eingesetzt.

In einer besonders bevorzugten Ausführungsform des erfindungemäßen Verfahrens wird die Oxidation der sekundären Hydroxylgruppe in dem multizyklischen Ausgangsringsystem durch ein Enzym F mit Alkoholdehydrogenase-Aktivität katalysiert, besonders bevorzugt stammt F aus *Pichia carsonii, Pichia guillermondii* oder *Pichia jadinii.*

In dem Verfahren kann jede Transaminase verwendet werden, die das eingesetzte multizyklische Ringsystem als Substrat akzeptiert.

Vorzugsweise verwendet man bei diesen Verfahren Glutamin-Scyllo-Inositol Transaminasen (EC Nummer: 2.6.1.50), bevorzugt aus Mikroorganismen der Gattungen *Bacillus, Micromonospora* oder *Streptomyces,* besonders bevorzugt *Bacillus circulans* und *Streptomyces griseus.*

Besonders bevorzugte Enzyme mit Transaminase-Aktivität in dem erfindungsgemäßen Verfahren umfassen, bevorzugt bestehend aus, Aminosäuresequenz gemäß SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 und SEQ ID NO: 33.

In einer weiteren besonders bevorzugten Ausführungsform des Verfahrens ist das Enzym mit Transaminase-Aktivität ausgewählt aus der Gruppe der funktionalen Äquivalenten der Gruppe bestehend aus funktionalen Äquivalente der Enzyme ausgewählt aus der Gruppe bestehend aus den Enzymen umfassend, bevorzugt bestehend aus, SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 und SEQ ID NO: 33, bei denen bis zu 25%, bevorzugt bis zu 20%, besonders bevorzugt bis zu 15% insbesondere bis zu 10, 9, 8, 7, 6, 5, 4, 3, 2, 1 % der Aminosäurereste gegenüber der korrespondierenden SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 bzw. SEQ ID NO: 33 durch Deletion, Substitution, eine Insertion oder eine Kombination aus Deletion, Substitution und Insertion verändert worden sind, wobei die funktionalen Äquivalente noch mindestens 50%, bevorzugt 65%, besonders bevorzugt 80% , insbesondere mehr als 90 % der enzymatischen Aktivität des Enzyms umfassend, bevorzugt bestehend aus, SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 bzw. SEQ ID NO: 33 besitzen. In diesem Zusammenhang soll unter enzymatische Aktivität von SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 bzw. SEQ ID NO: 33 die Fähigkeit verstanden werden, die Diulose (VII) zu den entsprechenden Diaminen Diaminoisosorbid (II) und Diaminoisoidid (III) zu transaminieren. Als Transaminasen können im erfindungsgemäßen Verfahren auch "funktionale Äquivalente" oder "funktionale Derivate" eingesetzt werden. "Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die die Diulose (VII) zu den entsprechenden Diaminen transaminieren und die mindestens 50 %, bevorzugt 60 %, besonders bevorzugt 75 %, ganz besonders bevorzugt 90 % der Aktivität eines Enzyms mit der in SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 bzw. SEQ ID NO: 33 aufgeführten Aminosäuresequenz aufweist. Enzym E und seine funktionale Äquivalente sind außerdem vorzugsweise zwischen pH 4 bis 10 stabil und besitzen vorteilhaft ein pH-Aktivitätsoptimum zwischen pH 5 und 8 sowie ein Temperatur-Aktivitätsoptimum im Bereich von 20 °C bis 80 °C Unter "funktionalen Äquivalenten" können insbesondere auch Mutanten verstanden werden, welche in wenigstens einer Sequenzposition der oben genannten Aminosäuresequenzen eine andere als die konkret genannte Aminosäure aufweisen, aber trotzdem eine der oben genannten biologischen Aktivitäten besitzen. "Funktionale Äquivalente" umfassen somit die durch eine oder mehrere Aminosäure-Additionen, -Substitutionen, - Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn; Gln |
| Ile | Leu; Val |
| Leu | Met; Val |
| Lys | Arg; Gln; Glu |
| Met | Leu ; Ile |
| Phe | Met; Leu; Tyr |
| Ser | Thr |
| Thr | Ser |
| Trp | Tyr |
| Tyr | Trp; Phe |
| Val | Ile; Leu |

Unter "funktionalen Äquivalenten" können erfindungsgemäß insbesondere auch Mutanten verstanden werden, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit vorzugsweise auch die durch eine oder mehrere Aminosäure-Additionen, -Substitutionen, -Deletionen und/oder - Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" im obigen Sinne können auch "Präkursoren" der beschriebenen Polypeptide sowie "funktionale Derivate" und "Salze" der Polypeptide sein. "Präkursoren" können dabei natürliche oder synthetische Vorstufen der Polypeptide mit oder ohne der gewünschten biologischen Aktivität sein. Unter dem Ausdruck "Salze" versteht man sowohl Salze von Carboxylgruppen als auch Säureadditionssalze von Aminogruppen der Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" der Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin, N-Acylderivate freier Aminogruppen, hergestellt durch Umsetzung mit Acylgruppen, oder O-Acylderivate freier Hydroxygruppen, hergestellt durch Umsetzung mit Acylgruppen.

"Funktionale Äquivalente" können natürlich auch Polypeptide sein, welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der Polypeptide, welche z.B. die gewünschte biologische Funktion aufweisen.

"Funktionale Äquivalente" können außerdem Fusionsproteine sein, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Erfindungsgemäß können "funktionale Äquivalente" Homologe zu den konkret offenbarten Enzymen sein. Diese besitzen wenigstens 60 %, vorzugsweise wenigstens 75% insbesondere wenigsten 85 %, wie z.B. 90%, 95% oder 99% Homologie zu einer der konkret offenbarten Aminosäuresequenzen, berechnet nach dem Algorithmus von Pearson und Lipman (Pearson WR, Lipman DJ. Improved tools for biological sequence comparison. Proc Natl Acad Sci U S A. 1988 Apr;85(8):2444-8). Eine prozentuale Homologie eines homologen Polypeptids bedeutet insbesondere prozentuale Identität der Aminosäurereste bezogen auf die Gesamtlänge einer der hier konkret beschriebenen Aminosäuresequenzen.

Im Falle einer möglichen Proteinglykosylierung umfassen "funktionale Äquivalente" Enzyme des oben bezeichneten Typs in deglykosylierter bzw. glykosylierter Form sowie durch Veränderung des Glykosylierungsmusters erhältliche abgewandelte Formen.

Homologe der Enzyme können durch Mutagenese erzeugt werden, z.B. durch Punktmutation oder Verkürzung des Enzyms.

Homologe des Enzyms können durch Screening kombinatorischer Banken von Mutanten, wie z.B.

Verkürzungsmutanten, identifiziert werden. Beispielsweise kann eine solche Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Protein-Sequenzen kodieren. Verfahren zur Synthese degenerierter Oligonukleotide sind dem Fachmann bekannt (z.B. Ike Y, Ikuta S, Sato M, Huang T, Itakura K. Solid phase synthesis of polynucleotides. VIII. Synthesis of mixed oligodeoxyribonucleotides by the phosphotriester solid phase method. Nucleic Acids Res. 1983 Jan 25;11(2):477-88). Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Expremieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin AP, Youvan DC. An algorithm for protein engineering: simulations of recursive ensemble mutagenesis. Proc Natl Acad Sci U S A. 1992 Aug 15;89(16):7811-5).

Die in dem erfindungsgemäßen Verfahren eingesetzten Enzyme lassen sich durch die dem Fachmann bekannten Verfahren herstellen, hierzu zählen z.B. die Produktion in Zellkulturen oder Mikroorganismen oder die *in-vitro*-Translation. Hierfür sind insbesondere Nukleinsäuresequenzen (einzel- und doppelsträngige DNA- und RNA-Sequenzen, wie z.B. cDNA und mRNA) nötig. Gegenstand der Erfindung ist die Verwendung der Nukleinsäuresequenzen, die für ein Enzym mit erfindungsgemäßer Transaminase-Aktivität oder eines seiner funktionalen Äquivalente kodieren. Bevorzugt sind Nukleinsäuresequenzen, welche für die Aminosäuresequenz gemäß SEQ ID NO: 2, SEQ ID NO: 4, SEQ ID NO: 7, SEQ ID NO: 9, SEQ ID NO: 11; SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 und SEQ ID NO: 33 oder charakteristische Teilsequenzen davon kodieren. Die entsprechenden Nukleinsäuren lassen sich leicht durch Rückübersetzung der SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 und SEQ ID NO: 33 gemäß dem genetischen Code ermitteln. Hierbei ist eine sogenannte Kodon-Optimierung, d.h. eine Anpassung der Kodons an die häufig benutzten Kodons von insbesondere hoch expremierten Genen des geplanten Wirtsorganismus, in dem die Nukleinsäure exprimiert werden soll. Nukleinsäuresequenzen sind durch chemische Synthese aus den Nukleotidbausteinen, wie z.B. durch Fragmentkondensation einzelner überlappender, komplementärer Nukleinsäurebausteine herstellbar. Die chemische Synthese von Oligonukleotiden kann beispielsweise nach der Phosphoamiditmethode (Koster H, Blocker H, Frank R, Geussenhainer S, Kaiser W. Total synthesis of a structural gene for the human peptide hormone angiotensin II. Hoppe Seylers Z Physiol Chem. 1975 Oct;356(10):1585-93) erfolgen. Auch Nukleinsäuresequenzen kodierend für eines der obigen Polypeptide und deren funktionalen Äquivalenten, welche z.B. unter Verwendung künstlicher Nukleotidanaloga zugänglich sind, können in erfindungemäßem Verfahren eingesetzt werden.

Ebenso können Nukleinsäuren, welchen mit oben genannten kodierenden Sequenzen unter stringenten Bedingungen hybridisieren eingesetzt werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise beim Screening von genomischen oder cDNA-Banken, in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1 % SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z.B. in Ausubel et al., Current Protocols in Molecular Biology, John Wiley & Sons, N.Y. (1989), 6.3.1-6.3.6. beschrieben. Solche Polynukleotide lassen sich gegebenenfalls mit geeigneten Primern mittels PCR vermehren und anschließend isolieren. Darüber hinaus können solche Polynukleotide auch auf chemischem Wege synthetisiert werden.

Besonders bevorzugt wird im erfindungsgemäßen Verfahren zur Bereitstellung des Enzyms E eine Nukleinsäuresequenz umfassend SEQ ID NO: 1 verwendet.

Offenbart sind außerdem Expressionskonstrukte, enthaltend unter der genetischen Kontrolle regulativer Nukleinsäuresequenzen eine für ein Polypeptid kodierende Nukleinsäuresequenz, sowie Vektoren, umfassend wenigstens eines dieser Expressionskonstrukte.

Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem Nukleinsäurekonstrukt sind insbesondere solche zu verstehen, bei welchen das Gen für eine Transaminase mit einem oder mehreren Regulationssignalen zur Steuerung, z.B. Erhöhung, der Genexpression operativ oder funktionell verknüpft wurden.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen insertiert werden, wie weitere regulatorische Elemente oder Terminatoren. Die Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein. Das Nukleinsäurekonstrukt wird zur Expression in einem Wirtsorganismus vorteilhafterweise in einen Vektor, wie beispielsweise einem Plasmid oder einem Phagen insertiert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, und lineare oder zirkuläre DNA zu verstehen. Diese Vektoren können autonom im Wirtsorganismus repliziert oder chromosomal repliziert werden. Weitere Vektoren und Plasmide sind dem Fachmann wohl bekannt und können beispielsweise aus dem Buch Cloning Vectors, A laboratory Manual (Eds. Pouwels P. H. et al. Elsevier, Amsterdam-New York-Oxford, 1985) entnommen werden. In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Bevorzugt werden die in dem erfindungemäßen Verfahren verwendeten Enzyme in Mikroorganismen hergestellt. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

Eine Aufreinigung der Enzyme kann mit bekannten chromatographischen Verfahren erzielt werden. Dazu zählen z.B. die Gelfiltration, die An- und Kationenaustausch-Chromatographie und die hydrophobe Interaktionschromatographie. Es können auch andere übliche Verfahren wie Ultrafiltration, Kristallisation, Aussalzen, Dialyse und native Gelelektrophorese eingesetzt werden. Weitere geeignete Verfahren werden z.B. in Cooper, F. *G., Biochemische Arbeitsmethoden,* Verlag Walter de Gruyter, Berlin, New York beschrieben. Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", die oft auch als Antigene von Antikörpern erkannt werden können, wie z.B. His-Tags, Strep-Tags, myc-Tags, Flag-Tags, MBP-Tags und GST-Tags. Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

In einer bevorzugten Ausführungsform wird das erfindungsgemäße Verfahren in Anwesenheit der zur Herstellung der Enzyme E und/oder F benötigten Organismen durchgeführt, d.h. während der Kultivierung und Wachstum der Organismen, die E und/oder F produzieren, kann die Transaminierung am multizyklischen Ringsystem und/oder gegebenenfalls die Oxidation am Ausgangsringsystem stattfinden. Das Edukt kann direkt zur Anzucht gegeben werden oder nach Anzucht.

Der eingesetzte Organismus zur Herstellung der in dem erfindungsgemäßen Verfahren verwendeten Enzyme kann immobilisiert oder frei in der Kultur beweglich sein. Dieser eingesetzte Organismus kann ein Prokaryont oder Eukaryont sein. Dabei kann es sich um Säugetierzellen (wie etwa Zellen aus dem Menschen), um pflanzliche Zellen oder um Mikroorganismen wie Hefen, Pilze oder Bakterien handeln. Vorzugsweise wird in dem erfindungsgemäßen Verfahren ein Mikroorganismus als Organismus eingesetzt.

Besonders bevorzugt wird in dem erfindungsgemäßen Verfahren ein Organismus eingesetzt, der ausgewählt wird aus der Gruppe:
mindestens einem Mitglied der Gattung *Bacillus,*
mindestens einem Mitglied der Gattung *Lactobacillus,*
mindestens einem Mitglied der Gattung *Pseudomonas,*
mindestens einem Mitglied der Gattung *Streptococcus,*
mindestens einem Mitglied der Gattung *Streptomyces,*
mindestens einem Mitglied der Gattung *Hansenula,*
mindestens einem Mitglied der Gattung *Pichia,*
mindestens einem Mitglied der Gattung *Aspergillus,*
mindestens einem Mitglied der Gattung *Escherichia* und/oder
mindestens einem Mitglied der Gattung *Saccharomyces.*

Vorteilhaft werden gram-positive oder gram-negative Bakterien, bevorzugt Bakterien der Familien *Enterobacteriaceae, Pseudomonadaceae, Rhizobiaceae, Streptomycetaceae* oder *Nocardiaceae,* besonders bevorzugt Bakterien der Gattungen *Escherichia, Corynebacteria, Pseudomonas, Streptomyces, Nocardia, Burkholderia, Salmonella, Agrobacterium oder Rhodococcus* verwendet. Ganz besonders bevorzugt sind die Arten *Escherichia coli* oder *Corynebacterium glutamicum.* Weitere vorteilhafte Bakterien sind darüber hinaus in der Gruppe der Gram-positiven, alpha-Proteobacterien, beta- Proteobacterien oder gamma-Proteobacterien und weitere vorteilhafte Mikroorganismen in der Gattung *Saccharomyces* zu finden. Die Enzyme E und F können in dem erfindungsgemäßen Verfahren getrennt voneinander in mindestens zwei unterschiedlichen Organismen expremiert werden. Bevorzugt ist die gleichzeitige Expression der Enzyme E und F in einem Organismus. Der Organismus kann bereits als Wildtyp das in erfindungsgemäßem Verfahren eingesetzte Enzym auf natürliche Art und Weise exprimieren.

Es kann vorteilhaft sein, dass die Aktivität des eingesetzten Enzyms E und/oder F im Organismus erhöht wird. Dies kann z.B. durch Überexpression des Enzyms durch bekannte oder oben beschriebene rekombinante DNA-Techniken erreicht werden, aber beispielsweise auch durch Mutagenese und anschließende Screening-Verfahren der Organismen auf erhöhte Aktivität.

Es kann im erfindungsgemäßen Verfahren ein Organismus eingesetzt werden, welcher als Wildtyp kein einzusetzendes Enzym exprimiert aber mit Hilfe von rekombinanten Techniken in die Lage versetzt wurde, ein geeignetes Enzym heterolog zu exprimieren.

In dem erfindungsgemäßen Verfahren wird das exprimierte Enzym vorzugsweise homolog überexprimiert, besonders bevorzugt wird das exprimierte Enzym heterolog überexprimiert. Dies kann mit Hilfe einer Expressionskassette erfolgen, in der ein geeigneter Promotor mit einer geeigneten kodierenden Nukleotidsequenz sowie einem Terminator- oder Polyadenylierungssignal fusioniert wurde.

Dazu verwendet man gängige Rekombinations- und Klonierungstechniken, wie sie beispielsweise in T. Maniatis, E. F. Fritsch und J. Sambrook, Molecular Cloning: A Laboratory Manual, Cold Spring Harbor Laboratory, Cold Spring Harbor, NY (1989) sowie in TJ. Silhavy, M. L. Berman und L.W. Enquist, Experiments with Gene Fusions, Cold Spring Harbor Laboratory, Cold Spring Harbor, NY (1984) und in Ausubel, F. M. et al., Current Protocols in Molecular Biology, Greene Publishing Assoc. and Wiley Interscience (1987) beschrieben sind. Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

Die in dem erfindungsgemäßen Verfahren eingesetzten, exprimierten Enzyme können von dem produzierten Organismus sekretiert oder intrazellulär exprimiert werden.

In dem erfindungsgemäßen Verfahren kann die Transaminierung der Ketogruppe und gegebenenfalls die vorherige Oxidation der Hydroxylgruppe während der Kultivierung des Organismus erfolgen oder im Anschluss an die Kultivierung. Der Organismus kann von dem Kulturmedium abgetrennt werden, oder in ihm verbleiben. Im Fall der intrazellulären Expression des eingesetzten Enzyms kann es vorteilhaft sein, die Organismus-Zellen mit den dem Fachmann bekannten Verfahren wie z.B. mit Detergentien, einer French Press oder einer Kugelmühle aufzuschließen.

Aufgrund verfahrenstechnischer Vorteile ist es bevorzugt, dass das Enzym E und/oder F ihre katalytische Funktion in der Zelle ausüben und das Verfahren kontinuierlich in dem Ganzzellkatalysator durchgeführt wird.

In einer weiteren Ausführungsform des erfindungsgemäßen Verfahrens wird das Enzym E und/oder F in aufgereinigter Form eingesetzt. Eine Aufreinigung kann mit bekannten, oben beschriebenen oder chromatographischen Verfahren erzielt werden. Das Enzym E und/oder F kann in dieser Ausführungsform des erfindungsgemäßen Verfahren immobilisiert, z.B. an Latexkugeln gekoppelt, oder frei in Lösung vorliegen.

Bevorzugtes Lösungsmittel in erfindungsgemäßem Verfahren ist Wasser.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 5 und 10, besonders bevorzugt zwischen pH 6 und 9 gehalten.

Das erfindungsgemäße Verfahren kann batchweise, semi-batchweise oder kontinuierlich betrieben werden.

Es kann vorteilhaft sein, die Reaktionsbedingungen, bevorzugt den pH-Wert, während der enzymatischen Aminierung, dem Verfahrensschritt B, zu variieren. Auf diese Weise kann das Verhältnis der gebildeten Enantiomere im Produkt beeinflusst bzw. eingestellt werden. Bevorzugt wird daher das erfindungsgemäße Verfahren dadurch gekennzeichnet, dass durch die Variation der Reaktionsbedingungen, bevorzugt den pH-Wertes, das Verhältnis der gebildeten Enantiomere der mindestens eine Aminogruppe tragenden Ringsysteme beeinflusst wird.

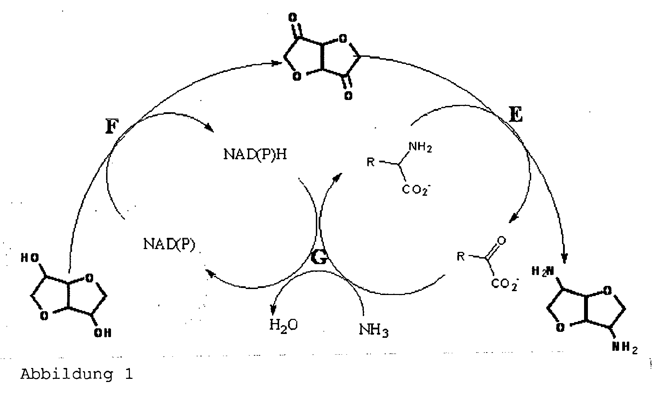

In dem erfindungsgemäßen Verfahren kann das Enzym F und E für die Oxidation der Hydroxylgruppe und die Transaminierung der Ketogruppe des multizyklischen Ringsystems verwendet werden und gleichzeitig ein Enzym G mit Aminosäuredehydrogenase-Aktivität zur gleichzeitigen Regeneration der Reduktionsäquivalente eingesetzt werden. Abbildung 1 verdeutlicht an beispielhaften Substanzen dieses Prinzip der Cofaktorregeneration. Daher werden in einer weiteren, bevorzugten Ausführungsform des erfindungsgemäßen Verfahrens die durch die enzymatischen Reaktionen der Enzyme E und F anfallenden Nebenprodukte, wie beispielsweise Reduktionsäquivalente oder Aminogruppendonoren, durch Verwendung mindestens eines Enzyms G mit Aminosäuredehydrogenase-Aktivität, wie beispielsweise Alanin-Dehydrogenase, Phenylalanin-Dehydrogenase, Aspartat-Dehydrogenase und die Serin-Dehydrogenase, insbesondere der Glutamat-Dehydrogenase regeneriert.

Wird in erfindungsgemäßem Verfahren eine Scyllo-Inositol-Transaminasen eingesetzt, so werden bevorzugt zusätzlich die Enzyme Glutamin-Synthetase und ω-Amidase eingesetzt, um Glutamin, den hier eingesetzten Aminogruppendonor, zu regenerieren.

### Produkte der Transaminierung

Ebenso offenbart sind die mit dem erfindungemäßen Verfahren hergestellten Produkte, also die multizyklischen, mindestens eine Aminogruppe tragenden Ringsysteme.

Bevorzugt ist das mit dem erfindungemäßen Verfahren hergestellte Produkt ein Enantiomeren-Gemisch. Besonders bevorzugt ist das mit dem erfindungemäßen Verfahren hergestellte Produkt ein Enantiomeren-Gemisch enthaltend ein Verhältnis von Diaminoisoidid (III) zu Diaminoisosorbid (II) von 0,5 bis 4, bevorzugt 0,8 bis 3 und besonders bevorzugt von 1 bis 2.

In den nachfolgend aufgeführten Beispielen wird die vorliegende Erfindung beispielhaft beschrieben.

In den nachfolgenden Beispielen 1 bis 3 werden die Darstellung der Diamine (II) - (III) aus der Hexodiulose (VII), die Darstellung der gemischten Monoamine (IX und X) aus dem Monoketon (VIII) 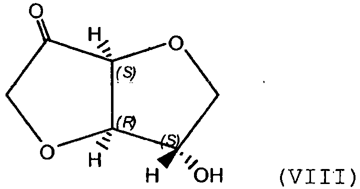 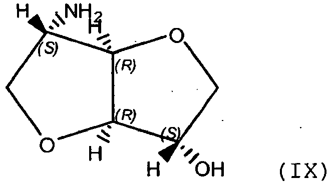 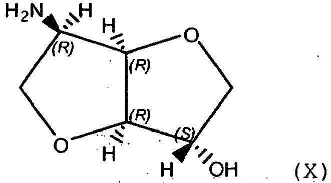 und
die Darstellung der Diamine (II) - (III) direkt aus (V) und (IV) beschrieben.

### Beispiele:

Folgende Abbildungen erläutern den Gegenstand des Beispiels:
Abbildung 2 zeigt das HPLC-Chromatogramm der Standards Diaminoisoidid (III) (0,5 g/l) und Diaminoisosorbid (II) (0,5 g/l) und Diaminoisomannit (I).
Abbildung 3 zeigt das HPLC-Chromatogramm der reduktiven Aminierung mit den Produkten Diaminoisosorbid, Diaminoisoidid und Spuren vom Diaminoisomannid bei pH 7,2.
Abbildung 4 zeigt das HPLC-Chromatogramm der Auftrennung des Reaktionsgemisches aus Aminotransferase BtrR und Diulose (VII) als Substrat bei pH 9.
Abbildung 5 zeigt das HPLC-Chromatogramm der Auftrennung des Reaktionsgemisches aus Aminotransferase BtrR und und Monoketon (VIII) als Substrat bei pH 9.
Abbildung 6 zeigt das HPLC Chromatogramm der Auftrennung des Reaktionsgemisches aus einer Aminotransferase aus *Vibrio fluvialis* und Diulose (VII) als Substrat.
Abbildung 7 zeigt das HPLC Chromatogramm der Auftrennung des Reaktionsgemisches aus einer Aminotransferase aus *Bacillus megaterium* und Diulose (VII) als Substrat.
Abbildung 8 zeigt das HPLC Chromatogramm der Auftrennung des Reaktionsgemisches aus einer Aminotransferase aus *Alcaligenes denitrificans* und Diulose (VII) als Substrat.
Abbildung 9 zeigt das HPLC Chromatogramm der Auftrennung des Reaktionsgemisches aus einer Aminotransferase aus *Chromobacterium violaceum* und Diulose (VII) als Substrat.
Abbildung 10 zeigt das HPLC Chromatogramm der Auftrennung des Reaktionsgemisches aus einer Aminotransferase aus *Anthrobacter sp.* und Diulose (VII) als Substrat.
Abbildung.11 zeigt das HPLC Chromatogramm der Auftrennung des Reaktionsgemisches aus einer Aminotransferase aus *Vibrio fluvialis* und Diulose (VII) als Substrat.
Abbildung 12 zeigt das HPLC Chromatogramm der Auftrennung des Reaktionsgemisches aus einer Aminotransferase aus *Pseudomonas putida* und Diulose (VII) als Substrat.
Abbildung 13 zeigt das HPLC Chromatogramm der Auftrennung des Reaktionsgemisches aus einer Aminotransferase aus *Pseudomonas putida* und Diulose (VII) als Substrat.
Abbildung 14 zeigt das HPLC Chromatogramm der Auftrennung des Reaktionsgemisches aus einer Aminotransferase aus *Pseudomonas putida* und Diulose (VII) als Substrat.
Abbildung 15 zeigt das HPLC Chromatogramm der Auftrennung des Reaktionsgemisches aus einer Aminotransferase aus *Chromobacterium violaceum* und Diulose (VII) als Substrat.
Abbildung 16 zeigt das HPLC Chromatogramm der Auftrennung des Reaktionsgemisches aus einer Aminotransferase aus *Rhodobacter sphaeroides* und Diulose (VII) als Substrat.
Abbildung 17 zeigt das HPLC Chromatogramm der Auftrennung des Reaktionsgemisches aus einer Aminotransferase aus *Paracoccus denitrificans* und Diulose (VII) als Substrat.
Abbildung 18 zeigt das HPLC Chromatogramm der Auftrennung des Reaktionsgemisches aus einer Aminotransferase aus *Mesorhizobium loti* und Diulose (VII) als Substrat.
Abbildung 19 zeigt das HPLC Chromatogramm der Auftrennung des Reaktionsgemisches aus einer Aminotransferase aus *Rhodobacter sphaeroides* und Diulose (VII) als Substrat.

### Beispiel 1: Synthese von Diaminoisosorbid (II) und Diaminoisoidid (III) ausgehend von der Diulose (VII) mit der Aminotransferase BtrR aus Bacillus circulans

Das Gen der Aminotransferase BtrR wurde bei Geneart synthetisiert, dabei wurde die Codonnutzung von *Escherichia coli* berücksichtigt, vgl. SEQ ID NO: 5. Zusätzlich enthielt die synthetisierte Sequenz eine *Eco*RI-Schnittstelle am 5'-Ende und eine *Pst*I-Schnittstelle am 3'-Ende (siehe Sequenz im Anhang). Das Gen wurde integriert in den Vector pGA4 (ampR) geliefert. Für die Klonierung in den Vector pACYC-Duet-1 wurde *btrR* mit den Restriktionsenzymen *Eco*RI und *Pst*I aus pGA4 herausgeschnitten. Der Ligationsansatz von btrR und pACYC-Duet-1 wurde zunächst in *E. coli* DH5α und nach Kontrollsequenzierung des Gens in den Zielorganismus *E. coli* BL21 (DE3) kloniert. Das rekombinante Plasmid trägt die Bezeichnung pACYC-Duet1::btrR Durch die Klonierung von btrR in die MCS1 von pACYC-Duet1 wird eine (His)₆-Tag Fusion am N-Terminus des Proteins eingeführt. Für die Aufreinigung des Proteins BtrR-(His)₆ wurde *E. coli* BL21 in LB-Medium kultiviert. Die Expression von *btrR* wurde in der exponentiellen Wachstumsphase OD 0,4-0,6 durch Zugabe von IPTG (Endkonzentration 1 mM) induziert. Nach einer Inkubationszeit von 6 h wurde die Kultur geerntet und das Zellpellet mit BugBuster-Reagenz aufgeschlossen. Die Aminotransferase BtrR-(His)₆ wurde über Ni-Chelat Affinitätschromatographie (His-bind columns, Novagen) nach Angaben des Säulenherstellers aufgereinigt. Das aufgereinigte Enzym wurde direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Tris-HCl Puffer pH 9 bzw. pH 7,2 | 50 mM |
| Diulose | 50 mM |
| L-Glutamin | 10 mM |
| Pyridoxalphosphat | 0,3 mM |
| aufgereinigtes BtrR in 50 mM Tris-HCl | 80 µg |
| Gesamtvolumen | 100 µl |

Nach einer Inkubationszeit von 72 h wurde die Probe durch eine Filtrationseinheit mit einer Ausschlussgröße von 10 kDa filtriert, um das Protein zu entfernen. In der FMOC-HPLC wurde eine Diaminoisoidid-Konzentration von 8,46 mg/l und eine Diaminosorbid-Konzentration von 6,3 mg/l gemessen. Unter diesen Bedingungen lag das Verhältnis von Diaminoisoidid (III) zu Diaminoisosorbid (II) bei 1,34. Das Verhältnis (III) : (II) konnte durch Erniedrigung des pH-Wertes zur vermehrten Synthese von Diaminoisoidid verschoben werden. Bei einem pH-Wert von 7,2 wurde ein Verhältnis Diaminoisoidid : Diaminoisosorbid von 1,8 erhalten (2,28 g/l vs. 1,27 g/l).

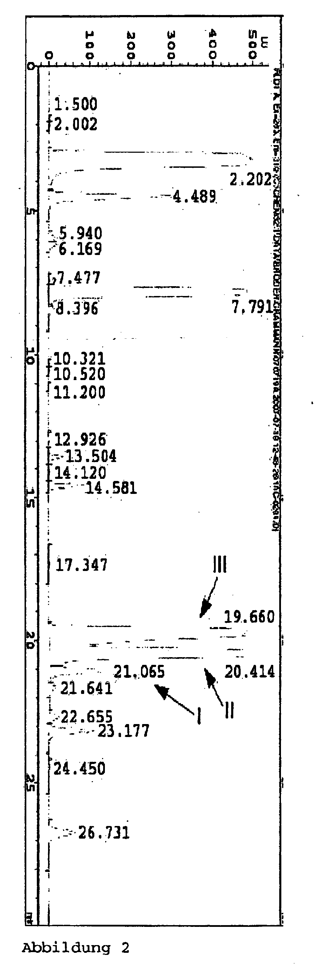

Abbildung 2 zeigt das Chromatogramm der Standards Diaminoisosidid (0,5 g/l) und Diaminoisosorbid (0,5 g/l).

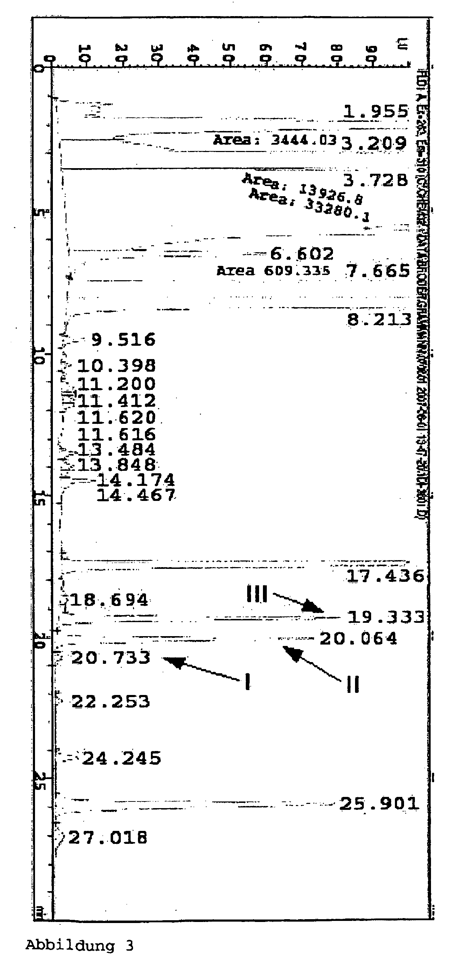

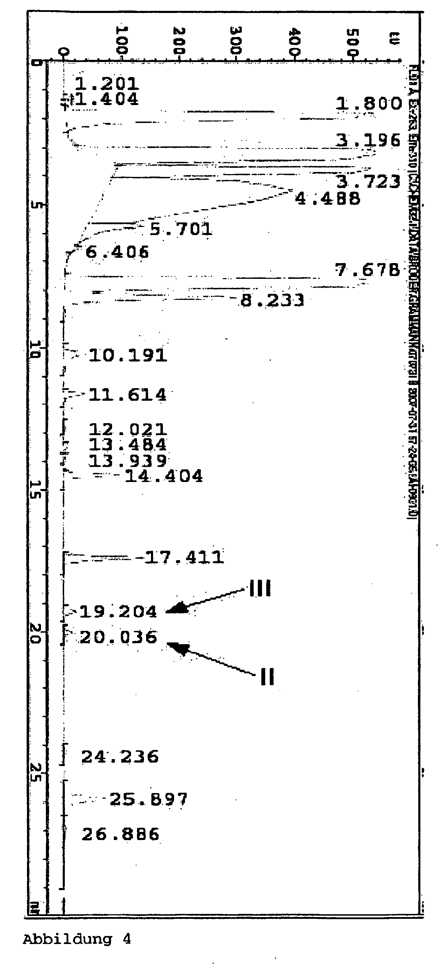

In Abbildung 3 ist die Auftrennung des Reaktionsgemisches aus Aminotransferase BtrR und Diulose (VII) als Substrat bei pH 9, in Abbildung 4 die Auftrennung des Reaktionsgemisches aus Aminotransferase BtrR und Diulose (VII) als Substrat bei pH 7,2 gezeigt.

Bei der Aminierung der Diulose (VII) entstehen zum Grossteil die Diamine (II) und (III) und nur in sehr geringer Menge das Diamin (I).

Durch Variation des pH-Wertes während der enzymatischen Aminierung konnte somit das Verhältnis zwischen Stereoisomer (II) und Stereoisomer (III) verändert werden.

### Beispiel 2: Aminierung des Monoketons (VIII) mit Aminotransferase BtrR aus B. circulans.

Der Enzymtest setzt sich zusammen wie in Beispiel 1 beschrieben. Anstelle der Diulose wird Monoketon (VIII) mit einer Endkonzentration von 50 mM eingesetzt.

**Zusammensetzung des Enzymtests**

| Puffer und Lösungen | Endkonzentration im Ansatz |
|---|---|
| 0,1 M Tris-HCl Puffer pH 9 | 50 mM |
| Monoketon (VIII) | 50 mM |
| L-Glutamin | 10 mM |
| Pyridoxalphosphat | 0,3 mM |
| aufgereinigtes BtrR in 50 mM Tris-HCl | 400 µg |
| Gesamtvolumen | 500 µl |

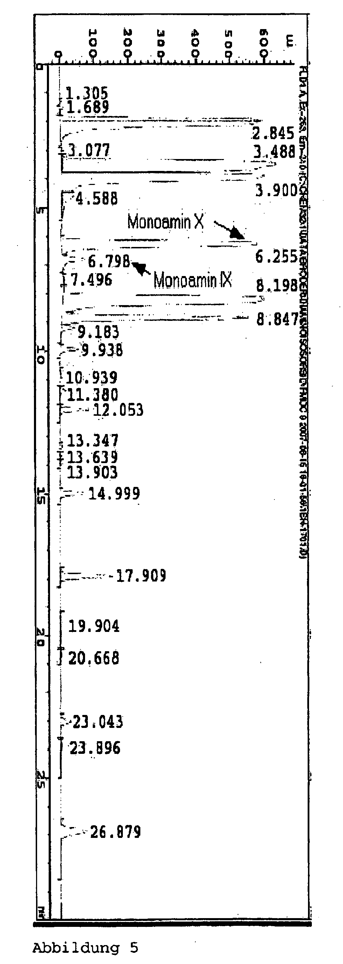

Abbildung 5 zeigt das HPLC-Diagramm der Auftrennung des Reaktionsgemisches aus Aminotransferase BtrR und Monoketon (VIII) als Substrat bei pH 9. Bei der Aminierung des Monoketons (VIII), wird entsprechend des Beispiel 1 mehr (X) als (IX) erhalten.

### Beispiel 3: Umsetzung zum Diamin ausgehend vom Isosorbid mit einer gekoppelten Reaktion

Um eine Umsetzung vom Isosorbid zum Diamin zu erreichen, wird eine Reaktion mit Zellextrakt aus *Pichia guillermondii* und aufgereinigtem BtrR-His Protein angesetzt. Neben der direkten Synthese vom Isosorbid über die Diulose zum Diamin erfolgt die Synthese auch über die Zwischenstufen Monoketon-Monoalkohol, Monoamin-Monoalkohol, Monoamin-Monoketon zum Diamin. Der Zellextrakt von *P*. *guillermondii* wird wie folgt hergestellte: 0,43 g Zellfeuchtmasse wurden in einem Reaktionsgefäß (Füllmenge 1,5 ml) mit 1ml 0,1mol/l Tris / HCl Puffer pH 7,2 resuspendiert. Zu dieser Zellsuspension wurden 1 g Glasperlen (0,25 - 0,3 mm Durchmesser) gegeben und dann 2x 5 min bei einer Frequenz 1/30s in der Kugelmühle (Retsch) aufgeschlossen. Vor dem zweiten 5 min-Lauf wurde das Reaktionsgefäß kurz auf Eis gestellt um Reibungswärme abzuführen. Danach wurde 10min bei 13200xg zentrifugiert und der Überstand vorsichtig abgenommen. Nach einer Inkubationszeit von 72 h werden die Proben über Filtrationseinheiten mit einer Ausschlussgröße von 30 kDa in einer Tischzentrifuge zentrifugiert. Um eine Rückreaktion der "Isosorbid"-Dehydrogenase zu vermeiden und NAD⁺ zu regenerieren, wird Pyruvat und L-Lactatdehydrogenase eingesetzt. Der Enzymtest setzt sich wie folgt zusammen:

| Puffer und Lösungen | Endkonzentration im Ansatz |
|---|---|
| 0,1 M Tris-HCl Puffer pH 8,2 | 50 mM |
| Pyruvat | 20 mM |
| NAD⁺ | 1 mM |
| L-Lactatdehydrogenase | 50 U |
| Isosorbid | 10 mM |
| Enzymextrakt aus P. guilliermondii | 10 % des Endvolumens (100 µl) |
| Pyridoxalphosphat | 0,3 mM |
| aufgereinigtes BtrR in 50 mM Tris-HCl | 800 µg |
| Proteaseinhibitor (Sigma) | 10 µl |
| Gesamtvolumen | 1 ml |

Über HPLC-Analyse können die Monoamin-Zwischenprodukte (IX) und (X) und die Diamine (II) und (III) identifiziert werden. Es konnte die Oxidation von Isosorbid (V) und Isomannid (IV) bis hin zur Diulose (VII) erreicht werden. Mit der rekombinant expremierten Transaminase wurde das Produkt der Oxidation (die Diulose (VII)) zu den Diamin (II) und (III) bzw. die Zwischenprodukte der Oxidation (z.B. das Monoketon (VIII) zu den Monoaminen (IX) und (X)) aminiert.

Somit wurde sowohl die enzymatische Oxidation sowohl der exoständigen als auch der endoständigen sekundären Hydroxylgruppen von Dianhydrohexitolen erreicht als auch die enzymatische Aminierung der Produkte dieser Reaktionen. Dabei konnten Aminogruppen sowohl in exo- als auch in endo-Stellung erhalten werden.

### Beispiel 4: Herstellung einer Aminotransferase aus Vibrio fluvialis und Aminierung der Diulose (VII) mit dieser.

Das Gen der Aminotransferase aus *Vibrio fluvialis* (SEQ ID NO: 6) wurde bei Geneart synthetisiert; dabei wurde die Codonnutzung von *Escherichia coli* berücksichtigt. Zusätzlich enthielt die synthetisierte Sequenz eine *Nde*I-Schnittstelle am 5'-Ende und eine *Xho*I-Schnittstelle am 3'-Ende. Das Gen wurde integriert in den Vector pET21a geliefert.

Der Vektor mit dem Zielgen wurde anschließend in den Zielorganismus *E*. *coli* BL21 (DE3) kloniert.

Die Expression des Aminotransferasegens wurde in der exponentiellen Wachstumsphase bei OD₆₀₀ 0,7 durch Zugabe von IPTG (Endkonzentration 1 mM) induziert. Nach einer Inkubationszeit von 3 h wurde die Kultur geerntet und das Zellpellet lyophilisiert. Lyophilisat aus 20 mg Zellen wurde nach Rehydrierung in 100 mM Natriumphosphatpuffer (pH7) direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 50 mM |
| L-Alanin | 250 mM |
| Pyridoxalphosphat | 1 mM |
| Zell-Lyophilisat mit Aminotransferase | 20 mg |
| Gesamtvolumen | 100 µl |

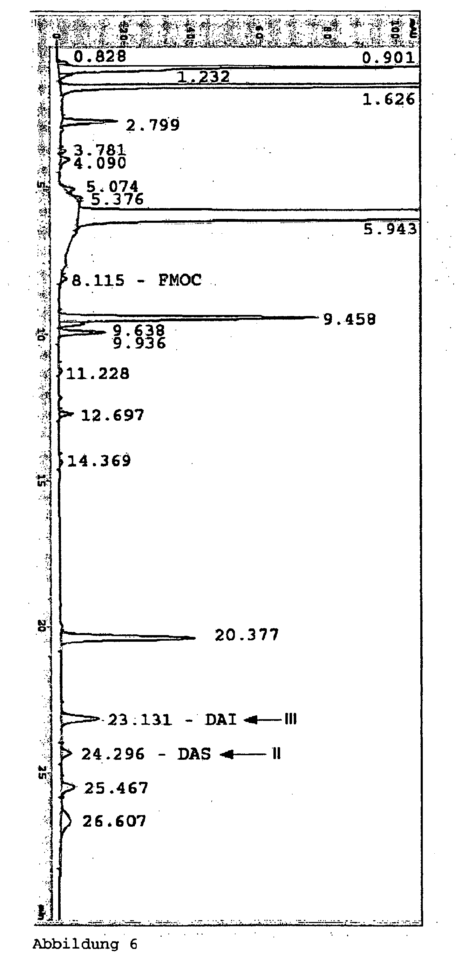

Nach einer Inkubationszeit von 24 h wurde die Probe durch eine Filtrationseinheit mit einer Ausschlussgröße von 10 kDa filtriert, um das Protein und das Zelllysat zu entfernen. Anschließend wird der Ansatz nach FMOC Derivatisierung mittels HPLC vermessen. Abbildung 6 zeigt das HPLC Chromatogramm der Auftrennung des Reaktionsgemisches aus einer Aminotransferase aus *Vibrio fluvialis* und Diulose (VII) als Substrat.

### Beispiel 5: Herstellung einer Aminotransferase aus Bacillus megaterium und Aminierung der Diulose (VII) mit dieser.

Das Gen der (SEQ ID NO: 8) Aminotransferase aus *Bacillus megaterium* wurde bei Geneart synthetisiert; dabei wurde die Codonnutzung von *Escherichia coli* berücksichtigt. Zusätzlich enthielt die synthetisierte Sequenz eine NdeI-Schnittstelle am 5'-Ende und eine *Xho*I-Schnittstelle am 3'-Ende. Das Gen wurde integriert in den Vector pET21a geliefert.

Der Vektor mit dem Zielgen wurde anschließend in den Zielorganismus *E*. *coli* BL21 (DE3) kloniert.

Die Expression des Aminotransferasegens wurde in der exponentiellen Wachstumsphase bei OD₆₀₀ 0,7 durch Zugabe von IPTG (Endkonzentration 1 mM) induziert. Nach einer Inkubationszeit von 3 h wurde die Kultur geerntet und das Zellpellet lyophilisiert. Lyophilisat aus 20 mg Zellen wurde nach Rehydrierung in 100 mM Natriumphosphatpuffer (pH7) direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 50 mM |
| L-Alanin | 250 mM |
| Pyridoxalphosphat | 1 mM |
| Zell-Lyophilisat mit Aminotransferase | 20 mg |
| Gesamtvolumen | 100 µl |

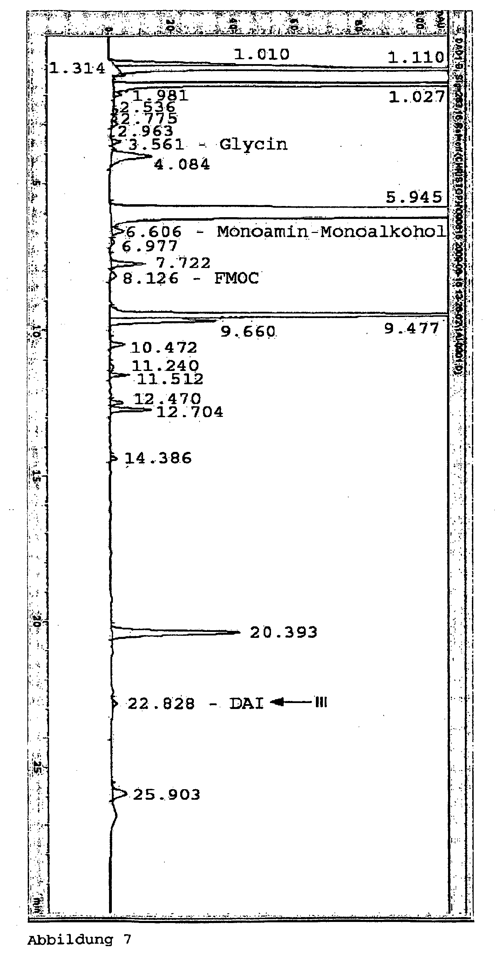

Nach einer Inkubationszeit von 24 h wurde die Probe durch eine Filtrationseinheit mit einer Ausschlussgröße von 10 kDa filtriert, um das Protein und das Zelllysat zu entfernen. Anschließend wird der Ansatz nach FMOC Derivatisierung mittels HPLC vermessen. Abbildung 7 zeigt das HPLC Chromatogramm der Auftrennung des Reaktionsgemisches aus einer Aminotransferase aus *Bacillus megaterium* und Diulose (VII) als Substrat.

### Beispiel 6: Herstellung einer Aminotransferase aus Alcaligenes denitrificans und Aminierung der Diulose (VII) mit dieser.

Das Gen (SEQ ID NO: 10) der Aminotransferase aus *Alcaligenes denitrificans* wurde bei Geneart synthetisiert; dabei wurde die Codonnutzung von *Escherichia coli* berücksichtigt. Zusätzlich enthielt die synthetisierte Sequenz eine *Nde*I-Schnittstelle am 5'-Ende und eine *Xho*I-Schnittstelle am 3'-Ende. Das Gen wurde integriert in den Vector pET21a geliefert.

Der Vektor mit dem Zielgen wurde anschließend in den Zielorganismus *E. coli* BL21 (DE3) kloniert.

Die Expression des Aminotransferasegens wurde in der exponentiellen Wachstumsphase bei OD₆₀₀ 0,7 durch Zugabe von IPTG (Endkonzentration 1 mM) induziert. Nach einer Inkubationszeit von 3 h wurde die Kultur geerntet und das Zellpellet lyophilisiert. Lyophilisat aus 20 mg Zellen wurde nach Rehydrierung in 100 mM Natriumphosphatpuffer (pH7) direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 50 mM |
| L-Alanin | 250 mM |
| Pyridoxalphosphat | 1 mM |
| Zell-Lyophilisat mit Aminotransferase | 20 mg |
| Gesamtvolumen | 100 µl |

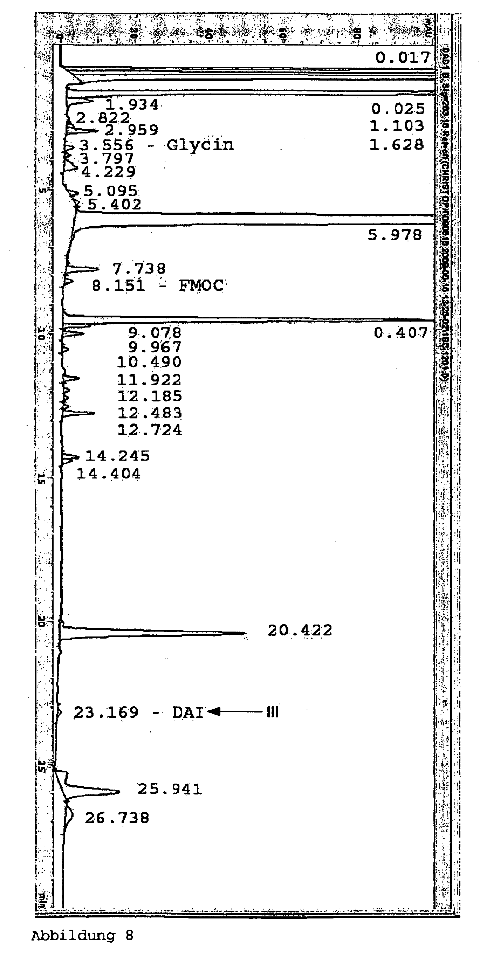

Nach einer Inkubationszeit von 24 h wurde die Probe durch eine Filtrationseinheit mit einer Ausschlussgröße von 10 kDa filtriert, um das Protein und das Zelllysat zu entfernen. Anschließend wird der Ansatz nach FMOC Derivatisierung mittels HPLC vermessen. Abbildung 8 zeigt das HPLC Chromatogramm der Auftrennung des Reaktionsgemisches aus einer Aminotransferase aus *Alcaligenes denitrificans* und Diulose (VII) als Substrat.

### Beispiel 7: Herstellung einer Aminotransferase aus Chromobacter violaceum und Aminierung der Diulose (VII) mit dieser.

Das Gen (SEQ ID NO: 12) der Aminotransferase aus *Chromobacter violaceum* wurde bei Geneart synthetisiert; dabei wurde die Codonnutzung von *Escherichia coli* berücksichtigt. Zusätzlich enthielt die synthetisierte Sequenz eine *Nde*I-Schnittstelle am 5'-Ende und eine *Xho*I-Schnittstelle am 3'-Ende. Das Gen wurde integriert in den Vector pET21a geliefert.

Der Vektor mit dem Zielgen wurde anschließend in den Zielorganismus *E*. *coli* BL21 (DE3) kloniert.

Die Expression des Aminotransferasegens wurde in der exponentiellen Wachstumsphase bei OD₆₀₀ 0,7 durch Zugabe von IPTG (Endkonzentration 1 mM) induziert. Nach einer Inkubationszeit von 3 h wurde die Kultur geerntet und das Zellpellet lyophilisiert. Lyophilisat aus 20 mg Zellen wurde nach Rehydrierung in 100 mM Natriumphosphatpuffer (pH7) direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer ph 7 | 100 mM |
| Diulose | 50 mM |
| L-Alanin | 250 mM |
| Pyridoxalphosphat | 1 mM |
| Zell-Lyophilisat mit Aminotransferase | 20 mg |
| Gesamtvolumen | 100 µl |

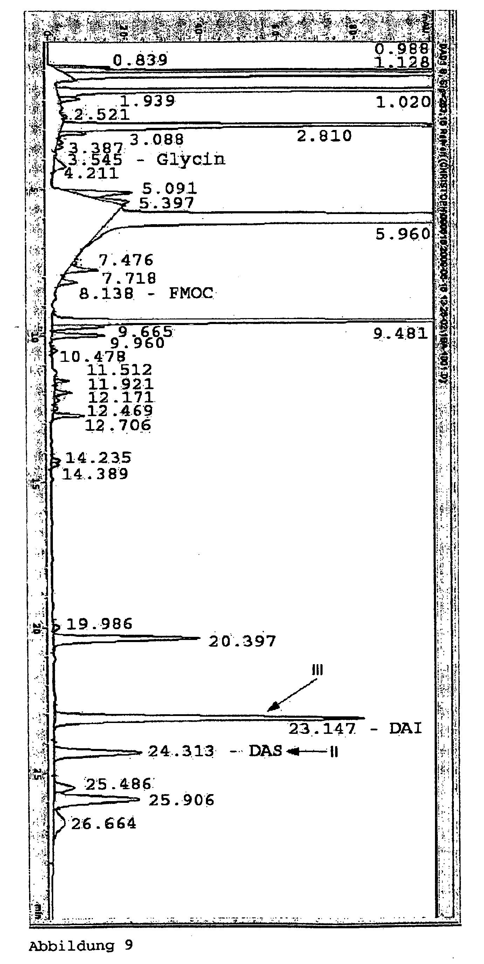

Nach einer Inkubationszeit von 24 h wurde die Probe durch eine Filtrationseinheit mit einer Ausschlussgröße von 10 kDa filtriert, um das Protein und das Zelllysat zu entfernen. Anschließend wird der Ansatz nach FMOC Derivatisierung mittels HPLC vermessen. Abbildung 9 zeigt das HPLC Chromatogramm der Auftrennung des Reaktionsgemisches aus einer Aminotransferase aus *Chromobacterium violaceum* und Diulose (VII) als Substrat.

### Beispiel 8: Herstellung einer Aminotransferase aus Arthrobacter sp. und Aminierung der Diulose (VII) mit dieser.

Das Gen (SEQ ID NO: 14) der Aminotransferase aus *Arthrobacter sp.* wurde bei Geneart synthetisiert; dabei wurde die Codonnutzung von *Escherichia coli* berücksichtigt. Zusätzlich enthielt die synthetisierte Sequenz eine *Nde*I-Schnittstelle am 5'-Ende und eine *Xho*I-Schnittstelle am 3'-Ende. Das Gen wurde integriert in den Vector pET21a geliefert.

Der Vektor mit dem Zielgen wurde anschließend in den Zielorganismus *E. coli* BL21 (DE3) kloniert.

Die Expression des Aminotransferasegens wurde in der exponentiellen Wachstumsphase bei OD₆₀₀ 0,7 durch Zugabe von IPTG (Endkonzentration 1 mM) induziert. Nach einer Inkubationszeit von 3 h wurde die Kultur geerntet und das Zellpellet lyophilisiert. Lyophilisat aus 20 mg Zellen wurde nach Rehydrierung in 100 mM Natriumphosphatpuffer (pH7) direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 50 mM |
| L-Alanin | 250 mM |
| Pyridoxalphosphat | 1 mM |
| Zell-Lyophilisat mit Aminotransferase | 20 mg |
| Gesamtvolumen | 100 µl |

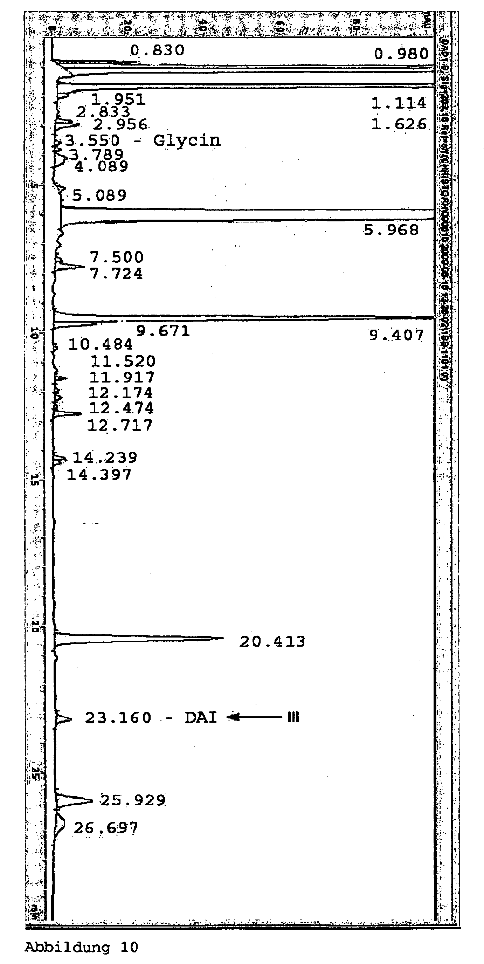

Nach einer Inkubationszeit von 24 h wurde die Probe durch eine Filtrationseinheit mit einer Ausschlussgröße von 10 kDa filtriert, um das Protein und das Zelllysat zu entfernen. Anschließend wird der Ansatz nach FMOC Derivatisierung mittels HPLC vermessen. Abbildung 10 zeigt das HPLC Chromatogramm der Auftrennung des Reaktionsgemisches aus einer Aminotransferase aus *Arthrobacter* sp. und Diulose (VII) als Substrat.

### Beispiel 9: Herstellung einer Aminotransferase aus Vibrio fluvialis und Aminierung der Diulose (VII) mit dieser.

Das Gen (SEQ ID NO: 16) der Aminotransferase aus *Vibrio fluvialis* wurde bei Geneart synthetisiert. Zusätzlich enthielt die synthetisierte Sequenz eine NdeI-Schnittstelle am 5'-Ende und eine *HindIII*-Schnittstelle am 3'-Ende. Das Gen wurde in den Vector pOM17c integriert.

Der Vektor mit dem Zielgen wurde anschließend in den Zielorganismus *E*. *coli* BL21 (DE3) kloniert.

Die Expression des Aminotransferasegens wurde in der exponentiellen Wachstumsphase bei OD₆₀₀ 0,5 durch Zugabe von IPTG (Endkonzentration 1 mM) induziert. Nach einer Inkubationszeit von 5 h wurde die Kultur geerntet und abzentrifugiert. Nachdemm das Zellpellet in 100 mM Natriumphosphatpuffer (pH 7,0) aufgenommen wurde, wurden die Zellen mittels Kugelmühle aufgeschlossen und der Rohextrakt durch anschließende Zentrifugation gewonnen. Der Rohextrakt wurde anschließend direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 5 mM |
| L-Alanin | 45 mM |
| Pyridoxalphosphat | 0,9 mM |
| Rohextrakt | 500 µl |
| Gesamtvolumen | 1500 µl |

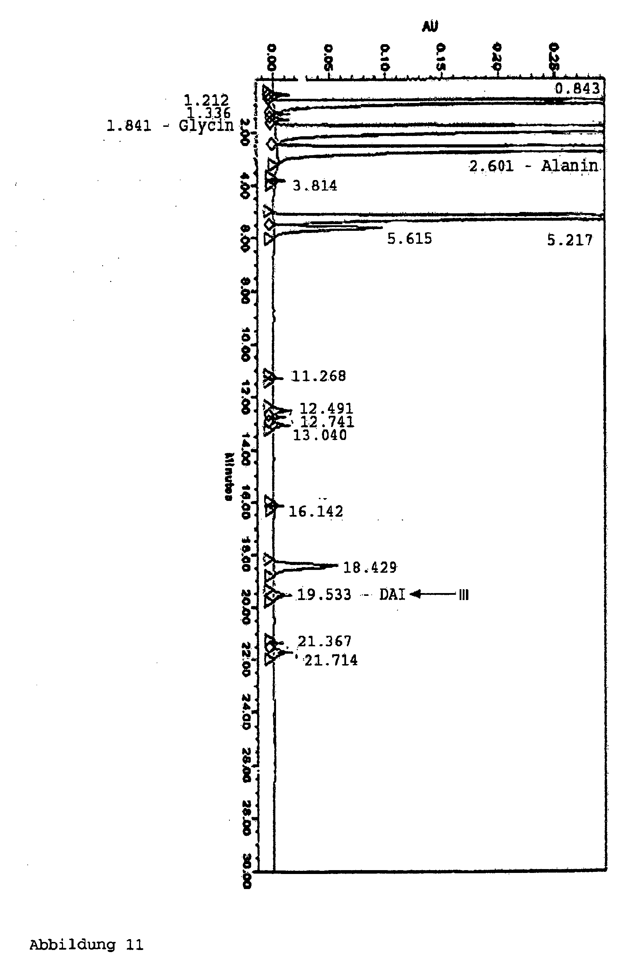

Nach einer Inkubationszeit von 24 h wurde die Reaktion gestoppt und der Ansatz nach FMOC Derivatisierung mittels HPLC vermessen. Abbildung 11 zeigt das HPLC Chromatogramm der Auftrennung des Reaktionsgemisches aus einer Aminotransferase aus *Vibrio fluvialis* und Diulose (VII) als Substrat.

### Beispiel 10: Herstellung einer Aminotransferase aus Pseudomonas putida und Aminierung der Diulose (VII) mit dieser.

Das Gen (SEQ ID NO: 18) der Aminotransferase wurde aus genomischer DNA von *Pseudomonas putida* amplifiziert und nachfolgend in einen pASK-IBA5+ Vektor mit N-terminalem Strep-tag II unter Entfernung des Start-ATGs des Aminotransferasegens kloniert. Dabei wurden die Schnittstellen *Ehe*I und *Hind*III verwendet.

Der Vektor mit dem Zielgen wurde anschließend zur Transformation des Zielorganismus *E. coli* BL21 eingesetzt.

Die Expression der Aminotransferase wurde in der exponentiellen Wachstumsphase brei OD₅₅₀ 0,5 durch Zugabe von 0,2 µg/ml AHT induziert. Nach einer Induktionszeit von 3 h wurde die Kultur geerntet, in 25 mM Hepes/NaOH, pH 8,3 aufgenommen, und die Zellen wurden mittels French-Press aufgeschlossen. Anschließend wurde die mit einen Strep-tag II-fusionierten Aminotransferase mittels Streptavidin-Sepharose gereinigt und über Nacht gegen 25 mM Hepes/NaOH, pH 8,3 dialysiert. Dann wurde das gereinigte Enzyem direkt in dem Enzymtest zur Transaminierung der Diulose eingesetzt.

Der Enzymtest setzte sich wie folgt zusammen:

**Zusammensetzung des Enzymtests**

| Puffer und Lösungen | Endkonzentration im Ansatz |
|---|---|
| Hepes/NaOH-Puffer pH 8,3 | 25 mM |
| Diulose | 25 mM |
| L-Alanin | 8 mM |
| Pyridoxalphosphat | 0,3 mM |
| Aufgereinigtes Enzym | 10 µM |
| Gesamtvolumen | 250 µl |

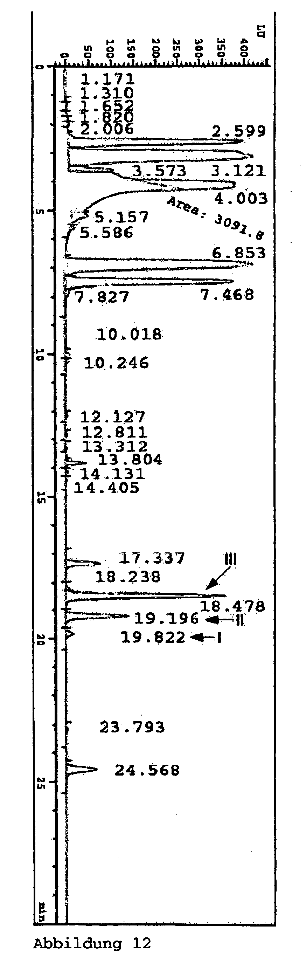

Anschließend wurde der Ansatz nach FMOC Derivatisierung mittels HPLC vermessen. Abbildung 12 zeigt das HPLC Chromatogramm der Auftrennung des Reaktionsgemisches der enzymatischen Umsetzung der Diulose (VII) durch eine Aminotransferase aus *Pseudomonas putida.*

### Beispiel 11: Herstellung einer Aminotransferase aus Pseudomonas putida und Aminierung der Diulose (VII) mit dieser.

Das Gen (SEQ ID NO: 20) der Aminotransferase aus *Pseudomoas putida* wurde aus genomischer DNA kloniert. Zusätzlich enthielt die klonierte Sequenz eine *Nde*I-Schnittstelle am 5'-Ende und eine *XhoI*-Schnittstelle am 3'-Ende. Das Gen wurde in den Vector pET21a(+) integriert.

Der Vektor mit dem Zielgen wurde anschließend in den Zielorganismus *E*. *coli* BL21 (DE3) kloniert.

Die Expression des Aminotransferasegens wurde in der exponentiellen Wachstumsphase bei OD₆₀₀ 0,5 durch Zugabe von IPTG (Endkonzentration 1 mM) induziert. Nach einer Inkubationszeit von 5 h wurde die Kultur geerntet und abzentrifugiert. Nachdemm das Zellpellet in 100 mM Natriumphosphatpuffer (pH 7,0) aufgenommen wurde, wurden die Zellen mittels Kugelmühle aufgeschlossen und der Rohextrakt durch anschließende Zentrifugation gewonnen. Der Rohextrakt wurde anschließend direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 5 mM |
| L-Alanin | 45 mM |
| Pyridoxalphosphat | 0,9 mM |
| Rohextrakt | 500 µl |
| Gesamtvolumen | 1500 µl |

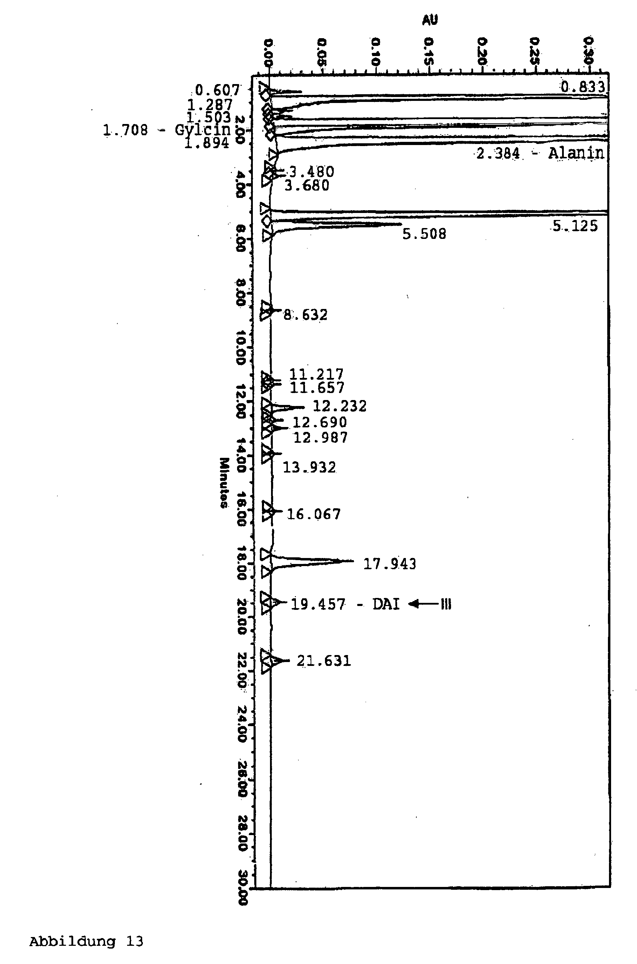

Nach einer Inkubationszeit von 24 h wurde die Reaktion gestoppt und der Ansatz nach FMOC Derivatisierung mittels HPLC vermessen. Abbildung 13 zeigt das HPLC Chromatogramm der Auftrennung des Reaktionsgemisches aus einer Aminotransferase aus *Pseudomoas putida* und Diulose (VII) als Substrat.

### Beispiel 12: Herstellung einer Aminotransferase aus Pseudomonas putida und Aminierung der Diulose (VII) mit dieser.

Das Gen (SEQ ID NO: 22) der Aminotransferase aus *Pseudomoas putida* wurde aus genomischer DNA kloniert. Zusätzlich enthielt die klonierte Sequenz eine *Nde*I-Schnittstelle am 5'-Ende und eine *XhoI*-Schnittstelle am 3'-Ende. Das Gen wurde in den Vector pET21a(+) integriert.

Der Vektor mit dem Zielgen wurde anschließend in den Zielorganismus *E. coli* BL21 (DE3) kloniert.

Die Expression des Aminotransferasegens wurde in der exponentiellen Wachstumsphase bei OD₆₀₀ 0,5 durch Zugabe von IPTG (Endkonzentration 1 mM) induziert. Nach einer Inkubationszeit von 5 h wurde die Kultur geerntet und abzentrifugiert. Nachdemm das Zellpellet in 100 mM Natriumphosphatpuffer (pH 7,0) aufgenommen wurde, wurden die Zellen mittels Kugelmühle aufgeschlossen und der Rohextrakt durch anschließende Zentrifugation gewonnen. Der Rohextrakt wurde anschließend direkt in den Enzymtest zur Umsetzung der Diulose eingesetzt.

Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 5 mM |
| L-Alanin | 45 mM |
| Pyridoxalphosphat | 0 , 9 mM |
| Rohextrakt | 500 µl |
| Gesamtvolumen | 1500 µl |

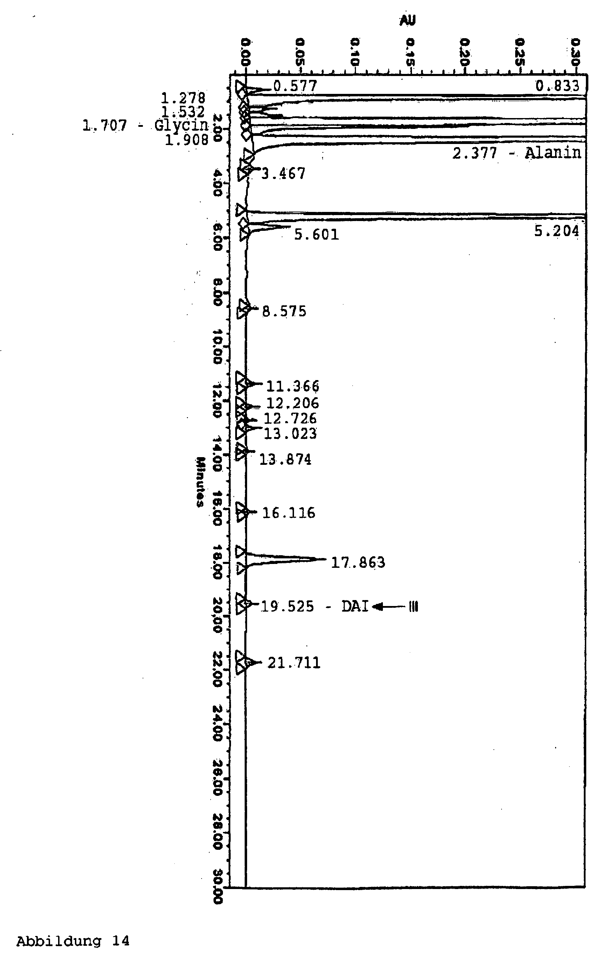

Nach einer Inkubationszeit von 24 h wurde die Reaktion gestoppt und der Ansatz nach FMOC Derivatisierung mittels HPLC vermessen. Abbildung 14 zeigt das HPLC Chromatogramm der Auftrennung des Reaktionsgemisches aus einer Aminotransferase aus *Pseudomoas putida* und Diulose (VII) als Substrat.

### Beispiel 13: Herstellung einer Aminotransferase aus Chromobacterium violaceum und Aminierung der Diulose (VII) mit dieser.

Das Gen (SEQ ID NO: 24) der Aminotransferase aus *Chromobacterium violaceum* wurde aus genomischer DNA kloniert. Zusätzlich enthielt die klonierte Sequenz eine *Nde*I-Schnittstelle am 5'-Ende und eine *XhoI*-Schnittstelle am 3'-Ende. Das Gen wurde in den Vector pET29a integriert.

Der Vektor mit dem Zielgen wurde anschließend in den Zielorganismus *E. coli* BL21 (DE3) kloniert.

Die Expression des Aminotransferasegens wurde in der exponentiellen Wachstumsphase bei OD₆₀₀ 0,5 durch Zugabe von IPTG (Endkonzentration 1 mM) induziert. Nach einer Inkubationszeit von 5 h wurde die Kultur geerntet und abzentrifugiert. Nachdemm das Zellpellet in 100 mM Natriumphosphatpuffer (pH 7,0) aufgenommen wurde, wurden die Zellen mittels Kugelmühle aufgeschlossen und der Rohextrakt durch anschließende Zentrifugation gewonnen. Der Rohextrakt wurde anschließend direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 5 mM |
| L-Alanin | 45 mM |
| Pyridoxalphosphat | 0,9 mM |
| Rohextrakt | 500 µl |
| Gesamtvolumen | 1500 µl |

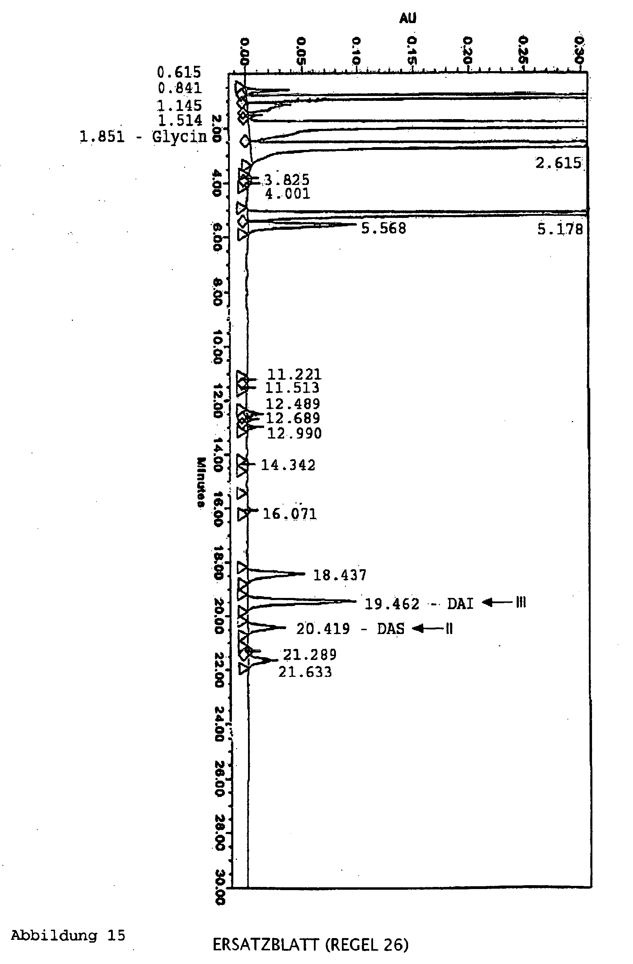

Nach einer Inkubationszeit von 24 h wurde die Reaktion gestoppt und der Ansatz nach FMOC Derivatisierung mittels HPLC vermessen. Abbildung 15 zeigt das HPLC Chromatogramm der Auftrennung des Reaktionsgemisches aus einer Aminotransferase aus *Chromobacterium violaceum* und Diulose (VII) als Substrat.

### Beispiel 14: Herstellung einer Aminotransferase aus Rhodobacter sphaeroides und Aminierung der Diulose (VII) mit dieser.

Das Gen (SEQ ID NO: 26) der Aminotransferase wurde aus genomischer DNA von *Rhodobacter sphaeroides* amplifiziert und nachfolgend in einen pASK-IBA5+ Vektor mit N-terminalem Strep-tag II unter Entfernung des Start-ATGs des Aminotransferasegens kloniert. Dabei wurden die Schnittstellen *Ehe*I und *Hind*III verwendet.

Der Vektor mit dem Zielgen wurde anschließend zur Transformation des Zielorganismus *E*. *coli* BL21 eingesetzt.

Die Expression der Aminotransferase wurde in der exponentiellen Wachstumsphase bei OD₅₅₀ 0,5 durch Zugabe von 0,2 µg/ml AHT induziert. Nach einer Induktionszeit von 3 h wurde die Kultur geerntet, in 25 mM Hepes/NaOH, pH 8,3 aufgenommen, und die Zellen wurden mittels French-Press aufgeschlossen. Anschließend wurde die mit einen Strep-tag II-fusionierten Aminotransferase mittels Streptavidin-Sepharose gereinigt und über Nacht gegen 25 mM Hepes/NaOH, pH 8,3 dialysiert. Dann wurde das gereinigte Enzyem direkt in dem Enzymtest zur Transaminierung der Diulose eingesetzt.

Der Enzymtest setzte sich wie folgt zusammen:

**Zusammensetzung des Enzymtests**

| Puffer und Lösungen | Endkonzentration im Ansatz |
|---|---|
| Hepes/NaOH-Puffer pH 8,3 | 25 mM |
| Diulose | 25 mM |
| L-Alanin | 8 mM |
| Pyridoxalphosphat | 0,3 mM |
| Aufgereinigtes Enzym | 10 µM |
| Gesamtvolumen | 250 µl |

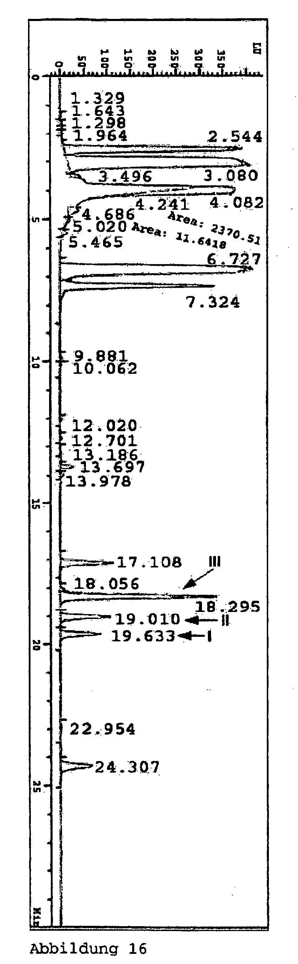

Anschließend wurde der Ansatz nach FMOC Derivatisierung mittels HPLC vermessen. Abbildung 16 zeigt das HPLC Chromatogramm der Auftrennung des Reaktionsgemisches der enzymatischen Umsetzung der Diulose (VII) durch eine Aminotransferase aus *Rhodobacter sphaeroides.*

### Beispiel 15: Herstellung einer Aminotransferase aus Paracoccus denitrificans und Aminierung der Diulose (VII) mit dieser.

Das Gen (SEQ ID NO: 28) der Aminotransferase aus *Paracoccus denitrificans* wurde aus genomischer DNA kloniert. Zusätzlich enthielt die klonierte Sequenz eine *Nde*I-Schnittstelle am 5'-Ende und eine *XhoI*-Schnittstelle am 3'-Ende. Das Gen wurde in den Vector pET21a(+) integriert.

Der Vektor mit dem Zielgen wurde anschließend in den Zielorganismus *E. coli* BL21 (DE3) kloniert.

Die Expression des Aminotransferasegens wurde in der exponentiellen Wachstumsphase bei OD₆₀₀ 0,5 durch Zugabe von IPTG (Endkonzentration 1 mM) induziert. Nach einer Inkubationszeit von 5 h wurde die Kultur geerntet und abzentrifugiert. Nachdem das Zellpellet in 100 mM Natriumphosphatpuffer (pH 7,0) aufgenommen wurde, wurden die Zellen mittels Kugelmühle aufgeschlossen und der Rohextrakt durch anschließende Zentrifugation gewonnen. Der Rohextrakt wurde anschließend direkt in den Enzymtest zur Umsetzung der Diulose eingesetzt. Die Produkte wurden anschließen über HPLC mit FMOC-Derivatisierung nachgewiesen.

Der Enzymtest setzte sich wie folgt zusammen:

**Zusammensetzung des Enzymtests**

| Puffer und Lösungen | Endkonzentration im Ansatz |
|---|---|
| 0,1 M Na-Phosphat Puffer pH 7 | 100 mM |
| Diulose | 5 mM |
| L-Alanin | 45 mM |
| Pyridoxalphosphat | 0,9 mM |
| Rohextrakt | 500 µl |
| Gesamtvolumen | 1500 µl |

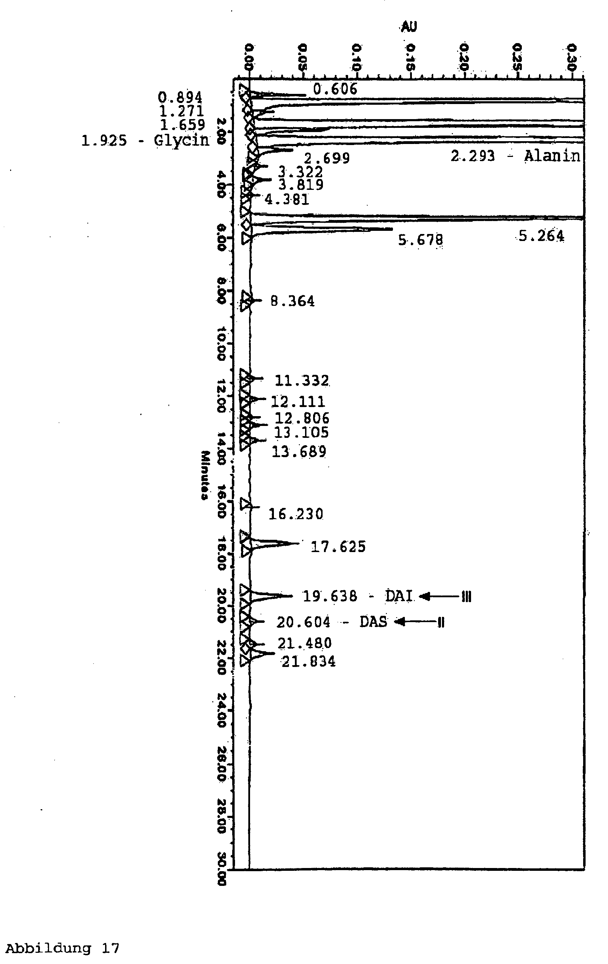

Nach einer Inkubationszeit von 24 h wurde die Reaktion gestoppt und der Ansatz nach FMOC Derivatisierung mittels HPLC vermessen. Abbildung 17 zeigt das HPLC Chromatogramm der Auftrennung des Reaktionsgemisches aus einer Aminotransferase aus *Paracoccus denitrificans* und Diulose (VII) als Substrat.

### Beispiel 16: Herstellung einer Aminotransferase aus Mesorhizobium loti und Aminierung der Diulose (VII) mit dieser.

Das Gen (SEQ ID NO: 30) der Aminotransferase wurde aus genomischer DNA von *Mesorhizobium loti* DSM 2626 amplifiziert und nachfolgend in einen pASK-IBA35+-Vektor mit N-terminalem His₆-tag unter Entfernung des N-terminalen ATG des Aminotransferasegens insertiert. Dabei wurden die Schnittstellen *Ehe*I und *Kpn*I verwendet.

Der Vektor mit dem Zielgen wurde anschließend zur Transfomation des Zielorganismus *E*. *coli* BL21 verwendet.

Die Expression der Aminotransferase wurde in der exponientiellen Wachstumsphase bei OD₅₅₀ 0,5 durch Zugabe von 0,2 µg/ml AHT induziert. Nach einer Induktionszeit von 3 h wurde die Kultur geerntet und die Zellen in 40 mM Hepes/NaOH, pH 7.5 aufgenommen und mittels French Press aufgeschlossen. Anschließend wurde die mit einem His₆-tag-fusionierte Aminotransferase mittels Zn²⁺-aktivierter Iminodiacetic-Sepharose gereinigt und mittels Gelfiltration an Superdex200 in 25 mM Hepes/NaOH, pH 8,3 umgepuffert. Dann wurde das gereinigte Enzym direkt in den Enzymtest zur Transaminierung der Diulose eingesetzt. Der Enzymtest setzte sich wie folgt zusammen:

| Puffer und Lösungen | Endkonzentration im Ansatz |
|---|---|
| Hepes/NaOH-Puffer pH 8,3 | 25 mM |
| Diulose | 25 mM |
| L-Alanin | 8 mM |
| Pyridoxalphosphat | 0,3 mM |
| Aufgereinigtes Enzym | 10 µM |
| Gesamtvolumen | 250 µl |

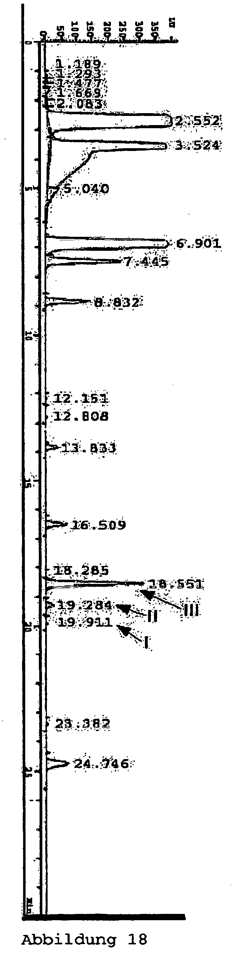

Anschließend wurde der Ansatz nach FMOC Derivatisierung mittels HPLC vermessen. Abbildung 18 zeigt das HPLC Chromatogramm der Auftrennung des Reaktionsgemisches der enzymatischen Umsetzung der Diulose (VII) durch eine Aminotransferase aus *Mesorhizobium loti.*

### Beispiel 17: Herstellung einer Aminotransferase aus Rhodobacter sphaeroides und Aminierung der Diulose (VII) mit dieser.

Das Gen (SEQ ID NO: 32) der Aminotransferase wurde aus genomischer DNA von *Rhodobacter sphaeroides* DSM 158 amplifiziert und nachfolgend in einen pASK-IBA35+-Vektor mit N-terminalem His₆-tag unter Entfernung des N-terminalen ATG des Aminotransferasegens insertiert. Dabei wurden die Schnittstellen *Ehe*I und *Hind*III verwendet.

Der Vektor mit dem Zielgen wurde anschließend zur Transfomation des Zielorganismus *E*. *coli* BL21 verwendet.

Die Expression der Aminotransferase wurde in der exponientiellen Wachstumsphase bei OD₅₅₀ 0,5 durch Zugabe von 0,2 µg/ml AHT induziert. Nach einer Induktionszeit von 3 h wurde die Kultur geerntet und die Zellen in 40 mM Hepes/NaOH, pH 7.5 aufgenommen und mittels French Press aufgeschlossen. Anschließend wurde die mit einem His₆-tag-fusionierte Aminotransferase mittels Zn²⁺-aktivierter Iminodiacetic-Sepharose gereinigt und mittels Gelfiltration an Superdex200 in 25 mM Hepes/NaOH, pH 8,3 umgepuffert. Dann wurde das gereinigte Enzym direkt in den Enzymtest zur Transaminierung der Diulose eingesetzt. Der Enzymtest setzte sich wie folgt zusammen:

| Puffer und Lösungen | Endkonzentration im Ansatz |
|---|---|
| Hepes/NaOH-Puffer pH 8,3 | 25 mM |
| Diulose | 25 mM |
| L-Alanin | 8 mM |
| Pyridoxalphosphat | 0,3 mM |
| Aufgereinigtes Enzym | 10 µM |
| Gesamtvolumen | 250 µl |

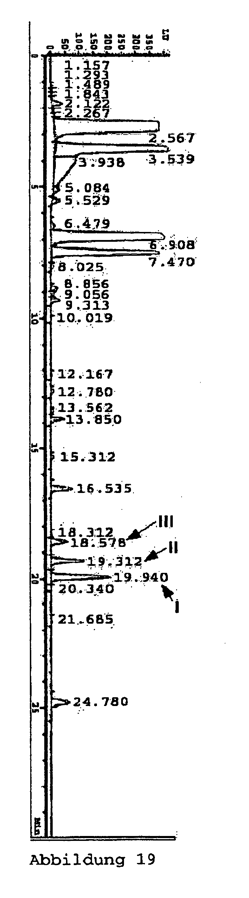

Anschließend wurde der Ansatz nach FMOC Derivatisierung mittels HPLC vermessen. Abbildung 19 zeigt das HPLC Chromatogramm der Auftrennung des Reaktionsgemisches der enzymatischen Umsetzung der Diulose (VII) durch eine Aminotransferase aus *Rhodobacter sphaeroides.*

## Patentansprüche

1. Verfahren zur Aminierung mindestens einer Ketogruppe in einem mindestens eine Ketogruppe aufweisenden multizyklischen Ringsystem zu einer Aminogruppe unter Verwendung mindestens eines Enzyms E mit Transaminase-Aktivität, **dadurch gekennzeichnet, dass** als multizyklische Ringsystem Verbindungen ausgewählt aus der Gruppe 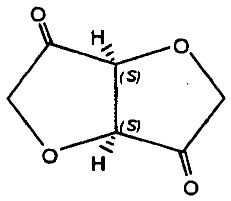 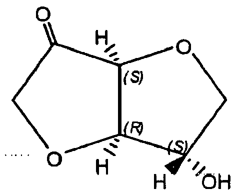 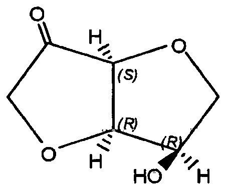 und als Enzym E eine Transaminase
eingesetzt werden.

2. Verfahren gemäß Anspruch 1, **dadurch gekennzeichnet, dass** ein Enzym aus der Klasse der Glutamin-Scyllo-Inositol Transaminasen (EC Nummer: 2.6.1.50) eingesetzt wird.

3. Verfahren gemäß Anspruch 1 oder 2, **dadurch gekennzeichnet, dass** das Enzym E aus Mikroorganismen der Gattungen Bacillus, *Micromonospora* oder *Streptomyces* isoliert werden kann.

4. Verfahren gemäß mindestens einem der Ansprüche 1 bis 3, **dadurch gekennzeichnet, dass** das Enzym E aus Mikroorganismen der Gattungen *Bacillus circulans* oder *Streptomyces griseus* isoliert werden kann.

5. Verfahren gemäß mindestens einem der Ansprüche 1 bis 4, **dadurch gekennzeichnet, dass** das Enzym mit Transaminase-Aktivität eine Polypeptidsequenz gemäß SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 oder SEQ ID NO: 33 umfasst oder ausgewählt wurde aus der Gruppe der funktionalen Äquivalenten des Enzyms Umfassend SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 oder SEQ ID NO: 33, bei denen bis zu 25% der Aminosäurereste gegenüber der korrespondierenden SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 bzw. SEQ ID NO: 33 durch Deletion, Insertion, Substitution, Insertion oder eine Kombination aus Deletion, Substitution und Insertion verändert worden sind und welche noch mindestens 50% der enzymatischen Aktivität des korrespondierenden Enzyms gemäß SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 bzw. SEQ ID NO: 3.3 besitzen.

6. Verfahren gemäß mindestens einem der Ansprüche 1 bis 5, **dadurch gekennzeichnet, dass** ein mindestens eine Ketogruppe aufweisendes multizyklisches Ringsystem eingesetzt wird, welches durch Oxidation mindestens einer sekundären Hydroxylgruppe eines multizyklischen Ausgangsringsystems zu einem Keton erhalten wird, **dadurch gekennzeichnet, dass** als multizyklisches Ausgangsringsystem mindestens eine Verbindung ausgewählt aus der Gruppe:
Isomannid,
Isosorbid,
Isoidid, 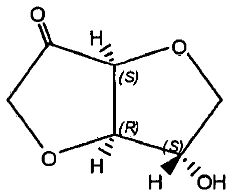 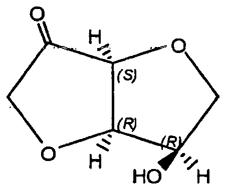
eingesetzt wird.

7. Verfahren gemäß Anspruch 6, **dadurch gekennzeichnet, dass** die Oxidation durch ein Enzym F mit Alkoholdehydrogenase-Aktivität katalysiert wird.

8. Verfahren gemäß Anspruch 7, **dadurch gekennzeichnet, dass** die durch die enzymatischen Reaktionen der Enzyme E und F anfallenden Nebenprodukte durch Verwendung mindestens eines Enzyms G mit Aminosäuredehydrogenase-Aktivität regeneriert werden.

9. Verfahren gemäß mindestens einem der Ansprüche 1 bis 8 **dadurch gekennzeichnet, dass** durch die Variation des pH-Werts das Verhältnis der gebildeten Enantiomere der mindestens eine Aminogruppe tragenden Ringsysteme beeinflusst wird.

## Claims

1. Process for the amination of at least one keto group in a multicyclic ring system bearing at least one keto group to give an amino group, using at least one enzyme E with transaminase activity, **characterized in that** the multicyclic ring system employed are compounds selected from among the group 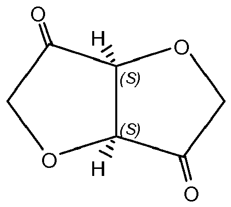 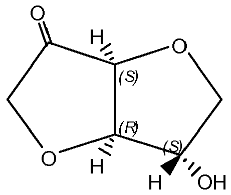 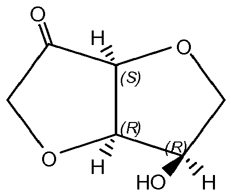 and the enzyme E employed is a transaminase.

2. Process according to Claim 1, **characterized in that** an enzyme from the class of the glutamine-scyllo-inositol transaminases (EC number: 2.6.1.50) is employed.

3. Process according to Claim 1 or 2, **characterized in that** the enzyme E can be isolated from microorganisms of the genera *Bacillus, Micromonospora* or *Streptomyces*.

4. Process according to at least one of Claims 1 to 3, **characterized in that** the enzyme E can be isolated from microorganisms of the species *Bacillus circulans* or *Streptomyces griseus.*

5. Process according to at least one of Claims 1 to 4, **characterized in that** the enzyme with transaminase activity comprises a polypeptide sequence according to SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 or SEQ ID NO: 33 or has been selected from the group of the functional equivalents of the enzyme comprising SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 or SEQ ID NO: 33, where up to 25% of the amino acid residues have been modified over the corresponding SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 and SEQ ID NO: 33, respectively, by deletion, insertion, substitution or a combination of deletion, substitution and insertion and which retain at least 50% of the enzymatic activity of the corresponding enzyme according to SEQ ID NO: 2, SEQ ID NO: 4, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 and SEQ ID NO: 33, respectively.

6. Process according to at least one of Claims 1 to 5, **characterized in that** a multicyclic ring system bearing at least one keto group, which ring system is obtained by oxidation of at least one secondary hydroxyl group of a multicyclic starting ring system to give a ketone, is employed, **characterized in that** the multicyclic starting ring system employed is at least one compound selected from the group:
isomannide,
isosorbide,
isoidide,

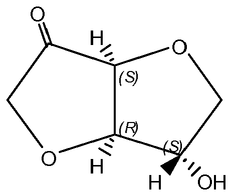

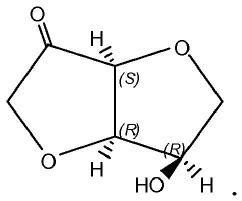

7. Process according to Claim 6, **characterized in that** the oxidation is catalyzed by an enzyme F with alcohol dehydrogenase activity.

8. Process according to Claim 7, **characterized in that** the byproducts generated by the enzymatic reactions of the enzymes E and F are regenerated by using at least one enzyme G with amino acid dehydrogenase activity.

9. Process according to at least one of Claims 1 to 8, **characterized in that** the ratio of the enantiomers formed, of the ring systems bearing at least one amino group, is influenced by varying the pH.

## Revendications

1. Procédé pour l'amination d'au moins un groupe céto dans un système cyclique polycyclique comportant au moins un groupe céto en un groupe amino avec utilisation d'une enzyme E à activité transaminase, **caractérisé en ce qu'**on utilise comme système cyclique polycyclique des composés choisis dans le groupe 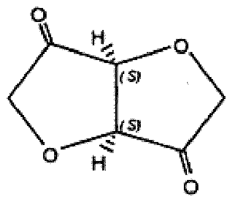 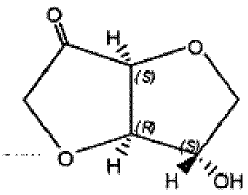 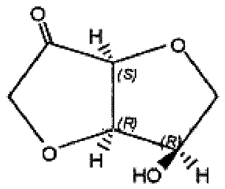 et comme enzyme E une transaminase.

2. Procédé selon la revendication 1, **caractérisé en ce qu'**on utilise une enzyme choisie dans la classe des glutamine-scyllo-inositol transaminases (numéro EC : 2.6.1.50).

3. Procédé selon la revendication 1 ou 2, **caractérisé en ce que** l'enzyme E peut être isolée à partir de micro-organismes appartenant au genre *Bacillus, Micromonospora* ou *Streptomyces.*

4. Procédé selon au moins l'une quelconque des revendications 1 à 3, **caractérisé en ce que** l'enzyme E peut être isolée à partir de micro-organismes appartenant aux espèces *Bacillus circulans* ou *Streptomyces griseus.*

5. Procédé selon au moins l'une quelconque des revendications 1 à 4, caractérisé en ce l'enzyme à activité transaminase comprend une séquence de polypeptide selon SEQ ID N° 2, SEQ ID N° 4, SEQ ID N° 7, SEQ ID N° 9, SEQ ID N° 11, SEQ ID N° 13, SEQ ID N° 15, SEQ ID N° 17, SEQ ID N° 19, SEQ ID N° 21, SEQ ID N° 23, SEQ ID N° 25, SEQ ID N° 27, SEQ ID N° 29, SEQ ID N° 31 ou SEQ ID N° 33 ou a été choisie dans le groupe des équivalents fonctionnels de l'enzyme comprenant SEQ ID N° 2, SEQ ID N° 4, SEQ ID N° 7, SEQ ID N° 9, SEQ ID N° 11, SEQ ID N° 13, SEQ ID N° 15, SEQ ID N° 17, SEQ ID N° 19, SEQ ID N° 21, SEQ ID N° 23, SEQ ID N° 25, SEQ ID N° 27, SEQ ID N° 29, SEQ ID N° 31 ou SEQ ID N° 33, dans lesquels jusqu'à 25 % des résidus acide aminé ont été modifiés par délétion, insertion, remplacement ou une combinaison de délétion, remplacement et insertion, par rapport aux séquences SEQ ID N° 2, SEQ ID N° 4, SEQ ID N° 7, SEQ ID N° 9, SEQ ID N° 11, SEQ ID N° 13, SEQ ID N° 15, SEQ ID N° 17, SEQ ID N° 19, SEQ ID N° 21, SEQ ID N° 23, SEQ ID N° 25, SEQ ID N° 27, SEQ ID N° 29, SEQ ID N° 31 ou SEQ ID N° 33 correspondantes et qui possèdent encore au moins 50 % de l'activité enzymatique de l'enzyme correspondante selon SEQ ID N° 2, SEQ ID N° 4, SEQ ID N° 7, SEQ ID N° 9, SEQ ID N° 11, SEQ ID N° 13, SEQ ID N° 15, SEQ ID N° 17, SEQ ID N° 19, SEQ ID N° 21, SEQ ID N° 23, SEQ ID N° 25, SEQ ID N° 27, SEQ ID N° 29, SEQ ID N° 31 ou SEQ ID N° 33.

6. Procédé selon au moins l'une quelconque des revendications 1 à 5, **caractérisé en ce qu'**on utilise un système cyclique polycyclique comportant au moins un groupe céto, qui est obtenu par oxydation d'au moins un groupe hydroxy secondaire d'un système cyclique polycyclique de départ en une cétone, **caractérisé en ce qu'**on utilise comme système cyclique polycyclique de départ au moins un composé choisi dans le groupe
isomannide,
isosorbide,
isoidide,

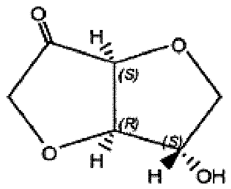

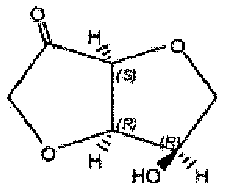

7. Procédé selon la revendication 6, **caractérisé en ce que** l'oxydation est catalysée par une enzyme F à activité alcool déshydrogénase.

8. Procédé selon la revendication 7, **caractérisé en ce qu'**on régénère les sous-produits formés par les réactions enzymatiques des enzymes E et F, en utilisant au moins une enzyme G à activité acide aminé déshydrogénase.

9. Procédé selon au moins l'une quelconque des revendications 1 à 8, **caractérisé en ce que** par la variation du pH on influe sur le rapport des énantiomères formés des systèmes cycliques portant au moins un groupe amino.


### SEQUENCE LISTING

<110> Evonik Degussa
<120> Verfahren zur Herstellung von Aminogruppen tragenden, multizyklischen Ringsystemen
<130> 200900038
<160> 33
<170> PatentIn version 3.4
<210> 1
   <211> 1257
   <212> DNA
   <213> Bacillus circulans
<400> 1
   atgaccattc catttgacca ttggccagaa tggcctcaac actccgatcg tacccgccga      60
   aaaattgaag aagtgtttca gtcgaacaga tgggcgataa gcggatattg gacgggcgag     120
   gaaagcatgg aacggaaatt cgccaaggcg tttgcggatt tcaacggggt cccatactgt     180
   gtgccgacga cgagcggctc cacggcttta atgcttgccc ttgaagcgct cggaataggc     240
   gaaggggacg aagtcattgt tcccagcctc acttggattg caaccgctac cgccgtatta     300
   aacgtgaatg cgcttcctgt ttttgtggac gttgaggcgg atacgtattg tattgatcct     360
   caattgataa agtcggccat tacggataaa acgaaagcca ttatcccggt ccatttgttc     420
   ggaagtatgg cgaatatgga tgaaataaac gaaatcgcgc aagaacacaa cctttttgtg     480
   attgaagact gcgcgcaaag ccacggttcc gtatggaaca accagcgtgc cggaacgata     540
   ggagatatag gggcgttcag ctgtcagcag ggcaaagtgt taacggccgg agaagggggg    600
   attatcgtga cgaaaaatcc ccgtttattt gaactcatcc aacaattgag agcggattcc     660
   agggtttatt gcgatgattc ctccgaattg atgcacggtg acatgcagct ggtaaaaaaa     720
   ggggacattc aaggctccaa ctattgcttg tccgaatttc aatcggccat ccttctggat     780
   caacttcagg agctggatga caagaacgcg atccgggaaa agaacgccat gttcttgaac     840
   gatgccttaa gcaaaattga cggcattaag gtgatgaagc gccctcctca agtcagcaga     900
   caaacttatt acggctatgt attccggttc gatccggtga aattcggcgg gttgaatgcg     960
   gatcagttct gtgaaattct tcgcgaaaag ctcaatatgg ggacgtttta cctgcatccg    1020
   ccgtatctcc ccgtacacaa aaatccgttg ttctgcccct ggacaaagaa ccgttatttg    1080
   aaatcggttc gtaagacaga agcttattgg agagggcttc attacccggt ctccgaacgt    1140
   gcttccgggc aatccatcgt tatccaccat gctattttac tggctgaacc ctcgcatctg    1200
   tcgcttctgg tcgatgccgt agccgaactt gcccggaaat tttgcgtgac tcattaa       1257
<210> 2
   <211> 418
   <212> PRT
   <213> Bacillus circulans
<400> 2
   Met Thr Ile Pro Phe Asp His Trp Pro Glu Trp Pro Gln His Ser Asp
   1               5                   10                  15
   Arg Thr Arg Arg Lys Ile Glu Glu Val Phe Gln Ser Asn Arg Trp Ala
               20                  25                  30
   Ile Ser Gly Tyr Trp Thr Gly Glu Glu Ser Met Glu Arg Lys Phe Ala
           35                  40                  45
   Lys Ala Phe Ala Asp Phe Asn Gly Val Pro Tyr Cys Val Pro Thr Thr
       50                  55                  60
   Ser Gly Ser Thr Ala Leu Met Leu Ala Leu Glu Ala Leu Gly Ile Gly
   65                  70                  75                  80
   Glu Gly Asp Glu Val Ile Val Pro Ser Leu Thr Trp Ile Ala Thr Ala
                   85                  90                  95
   Thr Ala Val Leu Asn Val Asn Ala Leu Pro Val Phe Val Asp Val Glu
               100                 105                 110
   Ala Asp Thr Tyr Cys Ile Asp Pro Gln Leu Ile Lys Ser Ala Ile Thr
           115                 120                 125
   Asp Lys Thr Lys Ala Ile Ile Pro Val His Leu Phe Gly Ser Met Ala
       130                 135                 140
   Asn Met Asp Glu Ile Asn Glu Ile Ala Gln Glu His Asn Leu Phe Val
   145                 150                 155                 160
   Ile Glu Asp Cys Ala Gln Ser His Gly Ser Val Trp Asn Asn Gln Arg
                   165                 170                 175
   Ala Gly Thr Ile Gly Asp Ile Gly Ala Phe Ser Cys Gln Gln Gly Lys
               180                 185                 190
   Val Leu Thr Ala Gly Glu Gly Gly Ile Ile Val Thr Lys Asn Pro Arg
           195                 200                 205
   Leu Phe Glu Leu Ile Gln Gln Leu Arg Ala Asp Ser Arg Val Tyr Cys
       210                 215                 220
   Asp Asp Ser Ser Glu Leu Met His Gly Asp Met Gln Leu Val Lys Lys
   225                 230                 235                 240
   Gly Asp Ile Gln Gly Ser Asn Tyr Cys Leu Ser Glu Phe Gln Ser Ala
                   245                 250                 255
   Ile Leu Leu Asp Gln Leu Gln Glu Leu Asp Asp Lys Asn Ala Ile Arg
               260                 265                 270
   Glu Lys Asn Ala Met Phe Leu Asn Asp Ala Leu Ser Lys Ile Asp Gly
           275                 280                 285
   Ile Lys Val Met Lys Arg Pro Pro Gln Val Ser Arg Gln Thr Tyr Tyr
       290                 295                 300
   Gly Tyr Val Phe Arg Phe Asp Pro Val Lys Phe Gly Gly Leu Asn Ala
   305                 310                 315                 320
   Asp Gln Phe Cys Glu Ile Leu Arg Glu Lys Leu Asn Met Gly Thr Phe
                   325                 330                 335
   Tyr Leu His Pro Pro Tyr Leu Pro Val His Lys Asn Pro Leu Phe Cys
               340                 345                 350
   Pro Trp Thr Lys Asn Arg Tyr Leu Lys Ser Val Arg Lys Thr Glu Ala
           355                 360                 365
   Tyr Trp Arg Gly Leu His Tyr Pro Val Ser Glu Arg Ala Ser Gly Gln
       370                 375                 380
   Ser Ile Val Ile His His Ala Ile Leu Leu Ala Glu Pro Ser His Leu
   385                 390                 395                 400
   Ser Leu Leu Val Asp Ala Val Ala Glu Leu Ala Arg Lys Phe Cys Val
                   405                 410                 415
   Thr His
<210> 3
   <211> 1275
   <212> DNA
   <213> Streptomyces griseus
<400> 3
   atggatagtt cactggcaat ctctggtggg ccacgtctgt ccaaccgcga gtggccacgt      60
   tggccacagc cgggtgaccg cgcgctgaaa agcctcgaag acgtcctgac aagcgggcgt     120
   tggaccatta gctgcgccta tcagggccgg gattcgtatg agcgtcaatt cgcgtccgca     180
   ttcgcggact actgcggctc ggcgatgtgc gtgccgatat cgacgggaac cgcttcgctg 240
   gccatcgccc tggaggcctg cggggtcggc gccggcgacg aggtgatcgt ccccggactg 300
   agctgggtgg cctccgcctc ggccgttctc ggcatcaacg ccgtccccgt gctggtggac 360
   gtcgacccgg ccacgtactg cctggacccg gcggccaccg aggcggcgat cacggagcgg 420
   acccgggcca tcaccgtggt gcacgcctac tcggccgtcg cggacctgga cgcgctcctg 480
   gacatcgccc ggcggcacgg cctgccgctg atcgaggact gcgcacacgc gcacggcgcc 540
   gggttccgcg gccgtccggt gggggcgcac ggtgccgcgg gagtcttcag catgcagggc 600
   agcaagctgc tgacctgcgg cgagggcggt gcgctggtga ccgacgacgc ggacgtcgcg 660
   ctccgtgcgg agcacctgcg cgcggacgga cgggtggtgc ggcgcgagcc ggtgggcgtg 720
   ggcgagatgg agctggagga gaccggccgg atgatgggca gcaacgcctg cctgtcggag 780
   ttccacgcgg cggtactgct ggaccagctc gaactgctcg acgggcagaa cgcccgccgg 840
   acccgggcgg ccgaccacct caccgaccgg ttgagcgagc tggggatgac ggcccaggcg 900
   acggccccgg ggacgaccgc ccgcgcctac taccgctatc tggtgaggct gcccgacgaa 960
   gtgctggccg tggcgccggt ggaacggttc gcgcacgcgc tcacggccga actgggcttc 1020
   gcggtgaccc agacccaccg tccgctgaac gacaacccgc tgaaccgccc ctcgtcgcgc 1080
   aggcggttcg ccaccgacgc ccgctacctg gagcgggtgg acccgtcccg gttcgacctt 1140
   ccggccgcca agcgcgccca cgagagcgtt gtgagcttca gccacgaagt gctcctggct 1200
   ccgctcgacg ccatcgacga catcgcccgg gcgttccgga aggtcctgga caacgtccgg 1260
   gaggtttccc gctga 1275
<210> 4
   <211> 424
   <212> PRT
   <213> Streptomyces griseus
<400> 4
   Met Asp Ser Ser Leu Ala Ile Ser Gly Gly Pro Arg Leu Ser Asn Arg
   1               5                   10                  15
   Glu Trp Pro Arg Trp Pro Gln Pro Gly Asp Arg Ala Leu Lys Ser Leu
               20                  25                  30
   Glu Asp Val Leu Thr Ser Gly Arg Trp Thr Ile Ser Cys Ala Tyr Gln
           35                  40                  45
   Gly Arg Asp Ser Tyr Glu Arg Gln Phe Ala Ser Ala Phe Ala Asp Tyr
       50                  55                  60
   Cys Gly Ser Ala Met Cys Val Pro Ile Ser Thr Gly Thr Ala Ser Leu
   65 70 75 80
   Ala Ile Ala Leu Glu Ala Cys Gly Val Gly Ala Gly Asp Glu Val Ile
   85 90 95
   Val Pro Gly Leu Ser Trp Val Ala Ser Ala Ser Ala Val Leu Gly Ile
   100 105 110
   Asn Ala Val Pro Val Leu Val Asp Val Asp Pro Ala Thr Tyr Cys Leu
   115 120 125
   Asp Pro Ala Ala Thr Glu Ala Ala Ile Thr Glu Arg Thr Arg Ala Ile
   130 135 140
   Thr Val Val His Ala Tyr Ser Ala Val Ala Asp Leu Asp Ala Leu Leu
   145 150 155 160
   Asp Ile Ala Arg Arg His Gly Leu Pro Leu Ile Glu Asp Cys Ala His
   165 170 175
   Ala His Gly Ala Gly Phe Arg Gly Arg Pro Val Gly Ala His Gly Ala
   180 185 190
   Ala Gly Val Phe Ser Met Gln Gly Ser Lys Leu Leu Thr Cys Gly Glu
   195 200 205
   Gly Gly Ala Leu Val Thr Asp Asp Ala Asp Val Ala Leu Arg Ala Glu
   210 215 220
   His Leu Arg Ala Asp Gly Arg Val Val Arg Arg Glu Pro Val Gly Val
   225 230 235 240
   Gly Glu Met Glu Leu Glu Glu Thr Gly Arg Met Met Gly Ser Asn Ala
   245 250 255
   Cys Leu Ser Glu Phe His Ala Ala Val Leu Leu Asp Gln Leu Glu Leu
   260 265 270
   Leu Asp Gly Gln Asn Ala Arg Arg Thr Arg Ala Ala Asp His Leu Thr
   275 280 285
   Asp Arg Leu Ser Glu Leu Gly Met Thr Ala Gln Ala Thr Ala Pro Gly
   290 295 300
   Thr Thr Ala Arg Ala Tyr Tyr Arg Tyr Leu Val Arg Leu Pro Asp Glu
   305 310 315 320
   Val Leu Ala Val Ala Pro Val Glu Arg Phe Ala His Ala Leu Thr Ala
   325 330 335
   Glu Leu Gly Phe Ala Val Thr Gln Thr His Arg Pro Leu Asn Asp Asn
   340 345 350
   Pro Leu Asn Arg Pro Ser Ser Arg Arg Arg Phe Ala Thr Asp Ala Arg
   355 360 365
   Tyr Leu Glu Arg Val Asp Pro Ser Arg Phe Asp Leu Pro Ala Ala Lys
   370 375 380
   Arg Ala His Glu Ser Val Val Ser Phe Ser His Glu Val Leu Leu Ala
   385 390 395 400
   Pro Leu Asp Ala Ile Asp Asp Ile Ala Arg Ala Phe Arg Lys Val Leu
   405 410 415
   Asp Asn Val Arg Glu Val Ser Arg
   420
<210> 5
   <211> 1305
   <212> DNA
   <213> Artificial
<220>
   <223> Codon Optimized gen
<400> 5
   atgggcagca gccatcacca tcatcaccac agccaggatc cgaattccat gaccatcccg 60
   tttgatcatt ggccggaatg gccgcagcat agcgatcgta cccgtcgtaa aatcgaagaa 120
   gtgtttcaga gcaaccgttg ggcgattagc ggctattgga ccggcgaaga aagcatggaa 180
   cgcaaatttg cgaaagcgtt tgcggatttt aacggcgtgc cgtattgcgt tccgaccacc 240
   tctggtagca ccgcgctgat gctggccctg gaagcgctgg gcattggcga aggcgatgaa 300
   gtgattgtgc cgagcctgac ctggattgcg accgcgaccg cggtgctgaa cgtgaatgcg 360
   ctgccggtgt ttgtggatgt ggaagcggat acctattgca ttgatccgca gctgattaaa 420
   agcgcgatta ccgataaaac caaagcgatt attccggtgc acctgtttgg cagcatggcg 480
   aacatggatg aaattaacga aatcgcgcag gaacataacc tgttcgttat tgaagattgc 540
   gcgcagagcc atggcagcgt gtggaacaac cagcgtgcgg gcaccattgg cgatattggc 600
   gcgtttagct gccagcaggg caaagtgctg accgcgggtg aaggcggcat tattgtgacc 660
   aaaaatccgc gtctgtttga actgattcag cagctgcgtg cggatagccg tgtgtattgc 720
   gatgatagca gcgaactgat gcatggcgat atgcagctgg tgaaaaaagg cgatattcag 780
   ggcagcaact attgcctgag cgaatttcag agcgcgattc tgctggatca gctgcaagaa 840
   ctggacgata aaaacgcgat ccgtgaaaaa aacgcgatgt ttctgaacga tgccctgagc 900
   aaaattgatg gcatcaaagt gatgaaacgt ccgccgcagg tgagccgtca gacctattat 960
   ggctatgtgt ttcgttttga tccggtgaaa tttggcggcc tgaacgcgga tcagttttgc 1020
   gaaattctgc gcgaaaaact gaacatgggc accttttatc tgcatccgcc gtatctgccg 1080
   gtgcataaaa atccgctgtt ttgcccgtgg accaaaaacc gttatctgaa aagcgtgcgt 1140
   aaaaccgaag cgtattggcg tggcctgcat tatccggtga gcgaacgtgc gagcggccag 1200
   agcattgtga ttcatcatgc gatcctgctg gccgaaccga gccatctgag cctgctggtt 1260
   gatgcggtgg cggaactggc ccgtaaattt tgcgtgaccc attaa 1305
<210> 6
   <211> 1362
   <212> DNA
   <213> Vibrio fluvialis
<400> 6
   atgaacaaac cgcagagctg ggaagcgcgt gcggaaacct atagcctgta tggctttacc 60
   gatatgccga gcctgcatca gcgtggcacc gttgttgtta cccatggcga aggcccgtat 120
   atcgttgatg ttaacggccg tcgttatctg gatgcgaata gcggcctgtg gaatatggtt 180
   gcgggctttg atcataaagg cctgatcgat gcggcgaaag cgcagtatga acgttttccg 240
   ggctatcatg cgttttttgg ccgtatgagc gatcagaccg tgatgctgag cgaaaaactg 300
   gttgaagtga gcccgtttga tagcggccgt gtgttttata ccaacagcgg cagcgaagcg 360
   aatgatacca tggttaaaat gctgtggttt ctgcatgcgg cggaaggtaa accgcagaaa 420
   cgtaaaattc tgacccgctg gaacgcgtat catggcgtta ccgcggttag cgcgagcatg 480
   accggcaaac cgtataacag cgtgtttggt ctgccgctgc cgggttttgt tcatctgacc 540
   tgcccgcatt attggcgtta tggcgaagaa ggcgaaaccg aagaacagtt tgttgcgcgt 600
   ctggcgcgtg aactggaaga aaccattcag cgcgaaggcg cggataccat tgcgggtttt 660
   tttgcggaac cggttatggg tgcgggtggt gttattccgc cggcgaaagg ttattttcag 720
   gcgattctgc cgatcctgcg caaatatgat attccggtga tcagcgatga agtgatttgc 780
   ggctttggtc gtaccggtaa tacctggggt tgcgtgacct atgattttac cccggatgcg 840
   atcattagca gcaaaaacct gaccgcgggc ttttttccga tgggtgcggt tattctgggt 900
   ccggaactga gcaaacgtct ggaaaccgcg attgaagcga tcgaagaatt tccgcatggt 960
   tttaccgcga gcggtcatcc ggttggttgt gcgattgcgc tgaaagcgat cgatgtggtt 1020
   atgaacgaag gcctggcgga aaatgttcgt cgtctggcgc cgcgttttga agaacgcctg 1080
   aaacatattg cggaacgtcc gaacattggc gaatatcgcg gcattggttt tatgtgggcg 1140
   ctggaagcgg ttaaagataa agcgagcaaa accccgtttg atggcaatct gagcgtgagc 1200
   gaacgtattg cgaatacctg caccgatctg ggtctgattt gtcgtccgct gggtcagagc 1260
   gttgttctgt gcccgccgtt tattctgacc gaagcgcaga tggatgaaat gttcgataaa 1320
   ctggaaaaag cgctggataa agttttcgcg gaagttgcgt aa 1362
<210> 7
   <211> 453
   <212> PRT
   <213> Vibrio fluvialis
<400> 7
   Met Asn Lys Pro Gln Ser Trp Glu Ala Arg Ala Glu Thr Tyr Ser Leu
   1               5                   10                  15
   Tyr Gly Phe Thr Asp Met Pro Ser Leu His Gln Arg Gly Thr Val Val
               20                  25                  30
   Val Thr His Gly Glu Gly Pro Tyr Ile Val Asp Val Asn Gly Arg Arg
           35                  40                  45
   Tyr Leu Asp Ala Asn Ser Gly Leu Trp Asn Met Val Ala Gly Phe Asp
       50                  55                  60
   His Lys Gly Leu Ile Asp Ala Ala Lys Ala Gln Tyr Glu Arg Phe Pro
   65                  70                  75                  80
   Gly Tyr His Ala Phe Phe Gly Arg Met Ser Asp Gln Thr Val Met Leu
                   85                  90                  95
   Ser Glu Lys Leu Val Glu Val Ser Pro Phe Asp Ser Gly Arg Val Phe
               100                 105                 110
   Tyr Thr Asn Ser Gly Ser Glu Ala Asn Asp Thr Met Val Lys Met Leu
           115                 120                 125
   Trp Phe Leu His Ala Ala Glu Gly Lys Pro Gln Lys Arg Lys Ile Leu
       130                 135                 140
   Thr Arg Trp Asn Ala Tyr His Gly Val Thr Ala Val Ser Ala Ser Met
   145                 150                 155                 160
   Thr Gly Lys Pro Tyr Asn Ser Val Phe Gly Leu Pro Leu Pro Gly Phe
                   165                 170                 175
   Val His Leu Thr Cys Pro His Tyr Trp Arg Tyr Gly Glu Glu Gly Glu
               180                 185                 190
   Thr Glu Glu Gln Phe Val Ala Arg Leu Ala Arg Glu Leu Glu Glu Thr
   195 200 205
   Ile Gln Arg Glu Gly Ala Asp Thr Ile Ala Gly Phe Phe Ala Glu Pro
   210 215 220
   Val Met Gly Ala Gly Gly Val Ile Pro Pro Ala Lys Gly Tyr Phe Gln
   225 230 235 240
   Ala Ile Leu Pro Ile Leu Arg Lys Tyr Asp Ile Pro Val Ile Ser Asp
   245 250 255
   Glu Val Ile Cys Gly Phe Gly Arg Thr Gly Asn Thr Trp Gly Cys Val
   260 265 270
   Thr Tyr Asp Phe Thr Pro Asp Ala Ile Ile Ser Ser Lys Asn Leu Thr
   275 280 285
   Ala Gly Phe Phe Pro Met Gly Ala Val Ile Leu Gly Pro Glu Leu Ser
   290 295 300
   Lys Arg Leu Glu Thr Ala Ile Glu Ala Ile Glu Glu Phe Pro His Gly
   305 310 315 320
   Phe Thr Ala Ser Gly His Pro Val Gly Cys Ala Ile Ala Leu Lys Ala
   325 330 335
   Ile Asp Val Val Met Asn Glu Gly Leu Ala Glu Asn Val Arg Arg Leu
   340 345 350
   Ala Pro Arg Phe Glu Glu Arg Leu Lys His Ile Ala Glu Arg Pro Asn
   355 360 365
   Ile Gly Glu Tyr Arg Gly Ile Gly Phe Met Trp Ala Leu Glu Ala Val
   370 375 380
   Lys Asp Lys Ala Ser Lys Thr Pro Phe Asp Gly Asn Leu Ser Val Ser
   385 390 395 400
   Glu Arg Ile Ala Asn Thr Cys Thr Asp Leu Gly Leu Ile Cys Arg Pro
   405 410 415
   Leu Gly Gln Ser Val Val Leu Cys Pro Pro Phe Ile Leu Thr Glu Ala
   420 425 430
   Gln Met Asp Glu Met Phe Asp Lys Leu Glu Lys Ala Leu Asp Lys Val
   435 440 445
   Phe Ala Glu Val Ala
   450
<210> 8
   <211> 1425
   <212> DNA
   <213> Bacillus megaterium
<400> 8
   atgagcctga ccgtgcagaa aattaattgg gaacaggtga aagaatggga tcgcaaatat      60
   ctgatgcgta cctttagcac ccagaatgaa tatcagccgg ttccgattga aagcaccgaa     120
   ggcgattatc tgattatgcc ggatggcacc cgtctgctgg atttttttaa tcagctgtat     180
   tgcgttaatc tgggccagaa aaaccagaaa gtgaacgcag caattaaaga agcactggat     240
   cgttacggtt ttgtgtggga tacctatgcc accgattata aagcaaaagc cgcaaaaatt     300
   attattgaag atattctggg cgatgaagat tggcctggta aagttcgttt tgttagcacc     360
   ggtagcgaag cagttgaaac cgcactgaat attgcacgtc tgtataccaa tcgtccgctg     420
   gttgttaccc gtgaacatga ttatcatggt tggaccggtg gtgcagcaac cgttacccgt     480
   ctgcgtagct atcgtagcgg tctggttggt gaaaatagcg aaagctttag cgcacagatt     540
   ccgggtagca gctataatag cgcagttctg atggcaccga gcccgaatat gtttcaggat     600
   tccgatggta atctgctgaa agatgaaaat ggtgaactgc tgtccgttaa atatacccgt     660
   cgcatgattg aaaattatgg tccggaacag gttgcagcag ttattaccga agttagccag     720
   ggtgcaggta gcgcaatgcc tccgtatgaa tatattccgc agattcgtaa aatgaccaaa     780
   gaactgggtg ttctgtggat taatgatgaa gtgctgaccg gttttggtcg taccggtaaa     840
   tggtttggct atcagcatta tggtgttcag ccggatatta ttaccatggg taaaggtctg     900
   agcagcagca gcctgcctgc aggtgcagtt ctggttagca aagaaatcgc agcctttatg     960
   gataaacatc gttgggaaag cgttagcacc tatgcaggtc atccggttgc aatggcagca    1020
   gtttgtgcaa atctggaagt gatgatggaa gaaaattttg tggaacaggc caaagatagc    1080
   ggtgaatata tccgtagcaa actggaactg ctgcaggaaa aacataaaag cattggcaat    1140
   tttgatggtt atggcctgct gtggattgtt gatattgtga atgccaaaac caaaaccccg    1200
   tatgttaaac tggatcgcaa ttttacccat ggcatgaatc cgaatcagat tccgacccag    1260
   attatcatga aaaaagccct ggaaaaaggt gttctgattg gtggtgttat gccgaatacc    1320
   atgcgtattg gtgcaagcct gaatgttagc cgtggcgata ttgataaagc aatggatgca    1380
   ctggattatg ccctggatta tctggaaagc ggtgaatggc agtaa                    1425
<210> 9
   <211> 474
   <212> PRT
   <213> Bacillus megaterium
<400> 9
   Met Ser Leu Thr Val Gln Lys Ile Asn Trp Glu Gln Val Lys Glu Trp
   1               5                   10                  15
   Asp Arg Lys Tyr Leu Met Arg Thr Phe Ser Thr Gln Asn Glu Tyr Gln
               20                  25                  30
   Pro Val Pro Ile Glu Ser Thr Glu Gly Asp Tyr Leu Ile Met Pro Asp
           35                  40                  45
   Gly Thr Arg Leu Leu Asp Phe Phe Asn Gln Leu Tyr Cys Val Asn Leu
       50                  55                  60
   Gly Gln Lys Asn Gln Lys Val Asn Ala Ala Ile Lys Glu Ala Leu Asp
   65                  70                  75                  80
   Arg Tyr Gly Phe Val Trp Asp Thr Tyr Ala Thr Asp Tyr Lys Ala Lys
                   85                  90                  95
   Ala Ala Lys Ile Ile Ile Glu Asp Ile Leu Gly Asp Glu Asp Trp Pro
               100                 105                 110
   Gly Lys Val Arg Phe Val Ser Thr Gly Ser Glu Ala Val Glu Thr Ala
           115                 120                 125
   Leu Asn Ile Ala Arg Leu Tyr Thr Asn Arg Pro Leu Val Val Thr Arg
       130                 135                 140
   Glu His Asp Tyr His Gly Trp Thr Gly Gly Ala Ala Thr Val Thr Arg
   145                 150                 155                 160
   Leu Arg Ser Tyr Arg Ser Gly Leu Val Gly Glu Asn Ser Glu Ser Phe
                   165                 170                 175
   Ser Ala Gln Ile Pro Gly Ser Ser Tyr Asn Ser Ala Val Leu Met Ala
               180                 185                 190
   Pro Ser Pro Asn Met Phe Gln Asp Ser Asp Gly Asn Leu Leu Lys Asp
           195                 200                 205
   Glu Asn Gly Glu Leu Leu Ser Val Lys Tyr Thr Arg Arg Met Ile Glu
       210                 215                 220
   Asn Tyr Gly Pro Glu Gln Val Ala Ala Val Ile Thr Glu Val Ser Gln
   225                 230                 235                 240
   Gly Ala Gly Ser Ala Met Pro Pro Tyr Glu Tyr Ile Pro Gln Ile Arg
                   245                 250                 255
   Lys Met Thr Lys Glu Leu Gly Val Leu Trp Ile Asn Asp Glu Val Leu
               260                 265                 270
   Thr Gly Phe Gly Arg Thr Gly Lys Trp Phe Gly Tyr Gln His Tyr Gly
           275                 280                 285
   Val Gln Pro Asp Ile Ile Thr Met Gly Lys Gly Leu Ser Ser Ser Ser
       290                 295                 300
   Leu Pro Ala Gly Ala Val Leu Val Ser Lys Glu Ile Ala Ala Phe Met
   305                 310                 315                 320
   Asp Lys His Arg Trp Glu Ser Val Ser Thr Tyr Ala Gly His Pro Val
                   325                 330                 335
   Ala Met Ala Ala Val Cys Ala Asn Leu Glu Val Met Met Glu Glu Asn
               340                 345                 350
   Phe Val Glu Gln Ala Lys Asp Ser Gly Glu Tyr Ile Arg Ser Lys Leu
           355                 360                 365
   Glu Leu Leu Gln Glu Lys His Lys Ser Ile Gly Asn Phe Asp Gly Tyr
       370                 375                 380
   Gly Leu Leu Trp Ile Val Asp Ile Val Asn Ala Lys Thr Lys Thr Pro
   385                 390                 395                 400
   Tyr Val Lys Leu Asp Arg Asn Phe Thr His Gly Met Asn Pro Asn Gln
                   405                 410                 415
   Ile Pro Thr Gln Ile Ile Met Lys Lys Ala Leu Glu Lys Gly Val Leu
               420                 425                 430
   Ile Gly Gly Val Met Pro Asn Thr Met Arg Ile Gly Ala Ser Leu Asn
           435                 440                 445
   Val Ser Arg Gly Asp Ile Asp Lys Ala Met Asp Ala Leu Asp Tyr Ala
       450                 455                 460
   Leu Asp Tyr Leu Glu Ser Gly Glu Trp Gln
   465                 470
<210> 10
   <211> 1323
   <212> DNA
   <213> Alcaligenes denitrificans Y2k-2
<400> 10
   atgagcgcag caaaactgcc ggatctgagc catctgtgga tgccgtttac cgcaaatcgt      60
   cagtttaaag caaatccgcg tctgttagct tctgcaaaag gtatgtatta taccagcttt     120
   gatggtcgcc agattctgga tggcaccgca ggtctgtggt gtgttaatgc aggtcattgc     180
   cgtgaagaaa ttgttagcgc aattgcaagc caggcaggtg ttatggatta tgcaccgggt     240
   tttcagctgg gtcatccgct ggcctttgaa gcagcaaccg cagttgcagg tctgatgccg     300
   cagggtctgg atcgtgtttt ttttaccaat agcggtagcg aaagcgttga taccgcactg     360
   aaaattgcac tggcctatca tcgtgcacgt ggtgaagcac agcgtacccg tctgatcggt     420
   cgtgaacgtg gttatcatgg tgttggtttt ggtggtattt ctgtgggtgg tattagcccg     480
   aatcgtaaaa cctttagcgg tgcactgctg cctgcagttg atcatctgcc gcatacccat     540
   agcctggaac ataatgcatt tacccgtggc cagccggaat ggggtgcaca tctggccgat     600
   gaactggaac gtattattgc actgcatgat gcaagcacca ttgcagcagt tattgttgaa     660
   ccgatggcag gtagcaccgg tgttctggtt ccgcctaaag gttatctgga aaaactgcgt     720
   gaaattaccg cacgtcatgg tattctgctg atttttgatg aagtgattac cgcctatggt     780
   cgtctgggtg aagcaaccgc agcagcatat tttggtgtta caccggatct gattaccatg     840
   gcaaaaggtg ttagcaatgc agcagttccg gcaggtgcag ttgcagttcg tcgtgaagtt     900
   catgatgcca ttgttaatgg tccgcagggt ggcattgaat tttttcatgg ctatacctat     960
   agcgcacatc cgctggctgc agcagcagtt ctggccaccc tggatattta tcgtcgcgag    1020
   gacctgttcg ctcgtgctcg taaactgagc gcagcatttg aagaagcagc ccattctctg    1080
   aaaggtgcac cgcatgttat tgatgtgcgt aatattggtc tggttgccgg tattgaactg    1140
   tctccgcgtg aaggcgctcc tggcgctcgt gcagctgagg ctttccaaaa atgctttgat    1200
   accggtctga tggttcgtta taccggtgat attctggccg ttagccctcc gctgattgtg    1260
   gatgaaaatc agatcggcca gatttttgaa ggtattggca aagtgctgaa agaagtggcc    1320
   taa                                                                  1323
<210> 11
   <211> 440
   <212> PRT
   <213> Alcaligenes denitrificans Y2k-2
<400> 11
   Met Ser Ala Ala Lys Leu Pro Asp Leu Ser His Leu Trp Met Pro Phe
   1               5                   10                  15
   Thr Ala Asn Arg Gln Phe Lys Ala Asn Pro Arg Leu Leu Ala Ser Ala
               20                  25                  30
   Lys Gly Met Tyr Tyr Thr Ser Phe Asp Gly Arg Gln Ile Leu Asp Gly
           35                  40                  45
   Thr Ala Gly Leu Trp Cys Val Asn Ala Gly His Cys Arg Glu Glu Ile
       50                  55                  60
   Val Ser Ala Ile Ala Ser Gln Ala Gly Val Met Asp Tyr Ala Pro Gly
   65                  70                  75                  80
   Phe Gln Leu Gly His Pro Leu Ala Phe Glu Ala Ala Thr Ala Val Ala
                   85                  90                  95
   Gly Leu Met Pro Gln Gly Leu Asp Arg Val Phe Phe Thr Asn Ser Gly
               100                 105                 110
   Ser Glu Ser Val Asp Thr Ala Leu Lys Ile Ala Leu Ala Tyr His Arg
           115                 120                 125
   Ala Arg Gly Glu Ala Gln Arg Thr Arg Leu Ile Gly Arg Glu Arg Gly
       130                 135                 140
   Tyr His Gly Val Gly Phe Gly Gly Ile Ser Val Gly Gly Ile Ser Pro
   145                 150                 155                 160
   Asn Arg Lys Thr Phe Ser Gly Ala Leu Leu Pro Ala Val Asp His Leu
                   165                 170                 175
   Pro His Thr His Ser Leu Glu His Asn Ala Phe Thr Arg Gly Gln Pro
               180                 185                 190
   Glu Trp Gly Ala His Leu Ala Asp Glu Leu Glu Arg Ile Ile Ala Leu
           195                 200                 205
   His Asp Ala Ser Thr Ile Ala Ala Val Ile Val Glu Pro Met Ala Gly
       210                 215                 220
   Ser Thr Gly Val Leu Val Pro Pro Lys Gly Tyr Leu Glu Lys Leu Arg
   225                 230                 235                 240
   Glu Ile Thr Ala Arg His Gly Ile Leu Leu Ile Phe Asp Glu Val Ile
                   245                 250                 255
   Thr Ala Tyr Gly Arg Leu Gly Glu Ala Thr Ala Ala Ala Tyr Phe Gly
               260                 265                 270
   Val Thr Pro Asp Leu Ile Thr Met Ala Lys Gly Val Ser Asn Ala Ala
   275 280 285
   Val Pro Ala Gly Ala Val Ala Val Arg Arg Glu Val His Asp Ala Ile
   290 295 300
   Val Asn Gly Pro Gln Gly Gly Ile Glu Phe Phe His Gly Tyr Thr Tyr
   305 310 315 320
   Ser Ala His Pro Leu Ala Ala Ala Ala Val Leu Ala Thr Leu Asp Ile
   325 330 335
   Tyr Arg Arg Glu Asp Leu Phe Ala Arg Ala Arg Lys Leu Ser Ala Ala
   340 345 350
   Phe Glu Glu Ala Ala His Ser Leu Lys Gly Ala Pro His Val Ile Asp
   355 360 365
   Val Arg Asn Ile Gly Leu Val Ala Gly Ile Glu Leu Ser Pro Arg Glu
   370 375 380
   Gly Ala Pro Gly Ala Arg Ala Ala Glu Ala Phe Gln Lys Cys Phe Asp
   385 390 395 400
   Thr Gly Leu Met Val Arg Tyr Thr Gly Asp Ile Leu Ala Val Ser Pro
   405 410 415
   Pro Leu Ile Val Asp Glu Asn Gln Ile Gly Gln Ile Phe Glu Gly Ile
   420 425 430
   Gly Lys Val Leu Lys Glu Val Ala
   435 440
<210> 12
   <211> 1380
   <212> DNA
   <213> Chromobacterium violaceum
<400> 12
   atgcagaaac agcgtaccac ctctcagtgg cgtgaactgg atgcagcaca tcatctgcat     60
   ccgtttaccg ataccgcaag cctgaatcag gcaggtgcac gtgttatgac ccgtggtgaa    120
   ggtgtttatc tgtgggatag cgaaggcaac aaaattattg atggtatggc aggtctgtgg    180
   tgtgttaatg ttggttatgg tcgcaaagat tttgcagaag cagcacgtcg tcagatggaa    240
   gaactgccgt tttataatac cttttttaaa accacccatc cggcagttgt tgaactgagc   300
   agcctgctgg ccgaagttac accggcaggt tttgatcgtg tgttttatac caatagcggt    360
   agcgaaagcg ttgataccat gattcgcatg gttcgtcgtt attgggatgt tcagggcaaa    420
   ccggaaaaaa aaaccctgat cggtcgttgg aatggttatc atggtagcac cattggtggt    480
   gccagcctgg gtggtatgaa atatatgcat gaacagggtg atctgccgat tccgggtatg    540
   gcacatattg aacagccgtg gtggtataaa catggcaaag atatgacacc ggatgaattt    600
   ggtgttgttg cagcacgttg gctggaagaa aaaattctgg aaattggtgc cgataaagtt    660
   gcagcatttg tgggtgaacc gattcagggt gcaggtggtg ttattgttcc gcctgcaacc    720
   tattggcctg aaattgaacg tatctgccgc aaatatgatg ttctgctggt tgccgatgaa    780
   gttatttgtg gttttggtcg taccggtgaa tggtttggtc atcagcattt tggttttcag    840
   ccggacctgt ttaccgcagc caaaggctta tcttctggct atctgccgat tggtgcagtt    900
   tttgttggta aacgtgttgc agaaggtctg attgcaggcg gtgattttaa tcatggcttt    960
   acctatagcg gtcatccggt ttgtgcagca gttgcacatg caaatgttgc agcactgcgt   1020
   gatgaaggta ttgttcagcg cgtgaaagat gatattggtc cgtatatgca gaaacgttgg   1080
   cgtgaaacct ttagccgttt tgaacatgtt gatgatgttc gtggtgttgg tatggttcag   1140
   gcatttaccc tggtgaaaaa caaagcaaaa cgcgaactgt ttccggattt tggtgaaatt   1200
   ggcaccctgt gccgtgatat tttttttcgc aataatctga ttatgcgtgc ctgtggtgat   1260
   cacattgtta gcgcaccgcc tctggtgatg acccgtgccg aagttgatga aatgctggcc   1320
   gttgcagaac gctgtctgga agaatttgaa cagaccctga aagcacgtgg tctggcctaa   1380
<210> 13
   <211> 459
   <212> PRT
   <213> Chromobacterium violaceum
<400> 13
   Met Gln Lys Gln Arg Thr Thr Ser Gln Trp Arg Glu Leu Asp Ala Ala
   1               5                   10                  15
   His His Leu His Pro Phe Thr Asp Thr Ala Ser Leu Asn Gln Ala Gly
               20                  25                  30
   Ala Arg Val Met Thr Arg Gly Glu Gly Val Tyr Leu Trp Asp Ser Glu
           35                  40                  45
   Gly Asn Lys Ile Ile Asp Gly Met Ala Gly Leu Trp Cys Val Asn Val
       50                  55                  60
   Gly Tyr Gly Arg Lys Asp Phe Ala Glu Ala Ala Arg Arg Gln Met Glu
   65                  70                  75                  80
   Glu Leu Pro Phe Tyr Asn Thr Phe Phe Lys Thr Thr His Pro Ala Val
   85 90 95
   Val Glu Leu Ser Ser Leu Leu Ala Glu Val Thr Pro Ala Gly Phe Asp
   100 105 110
   Arg Val Phe Tyr Thr Asn Ser Gly Ser Glu Ser Val Asp Thr Met Ile
   115 120 125
   Arg Met Val Arg Arg Tyr Trp Asp Val Gln Gly Lys Pro Glu Lys Lys
   130 135 140
   Thr Leu Ile Gly Arg Trp Asn Gly Tyr His Gly Ser Thr Ile Gly Gly
   145 150 155 160
   Ala Ser Leu Gly Gly Met Lys Tyr Met His Glu Gln Gly Asp Leu Pro
   165 170 175
   Ile Pro Gly Met Ala His Ile Glu Gln Pro Trp Trp Tyr Lys His Gly
   180 185 190
   Lys Asp Met Thr Pro Asp Glu Phe Gly Val Val Ala Ala Arg Trp Leu
   195 200 205
   Glu Glu Lys Ile Leu Glu Ile Gly Ala Asp Lys Val Ala Ala Phe Val
   210 215 220
   Gly Glu Pro Ile Gln Gly Ala Gly Gly Val Ile Val Pro Pro Ala Thr
   225 230 235 240
   Tyr Trp Pro Glu Ile Glu Arg Ile Cys Arg Lys Tyr Asp Val Leu Leu
   245 250 255
   Val Ala Asp Glu Val Ile Cys Gly Phe Gly Arg Thr Gly Glu Trp Phe
   260 265 270
   Gly His Gln His Phe Gly Phe Gln Pro Asp Leu Phe Thr Ala Ala Lys
   275 280 285
   Gly Leu Ser Ser Gly Tyr Leu Pro Ile Gly Ala Val Phe Val Gly Lys
   290 295 300
   Arg Val Ala Glu Gly Leu Ile Ala Gly Gly Asp Phe Asn His Gly Phe
   305 310 315 320
   Thr Tyr Ser Gly His Pro Val Cys Ala Ala Val Ala His Ala Asn Val
   325 330 335
   Ala Ala Leu Arg Asp Glu Gly Ile Val Gln Arg Val Lys Asp Asp Ile
   340 345 350
   Gly Pro Tyr Met Gln Lys Arg Trp Arg Glu Thr Phe Ser Arg Phe Glu
   355 360 365
   His Val Asp Asp Val Arg Gly Val Gly Met Val Gln Ala Phe Thr Leu
   370 375 380
   Val Lys Asn Lys Ala Lys Arg Glu Leu Phe Pro Asp Phe Gly Glu Ile
   385 390 395 400
   Gly Thr Leu Cys Arg Asp Ile Phe Phe Arg Asn Asn Leu Ile Met Arg
   405 410 415
   Ala Cys Gly Asp His Ile Val Ser Ala Pro Pro Leu Val Met Thr Arg
   420 425 430
   Ala Glu Val Asp Glu Met Leu Ala Val Ala Glu Arg Cys Leu Glu Glu
   435 440 445
   Phe Glu Gln Thr Leu Lys Ala Arg Gly Leu Ala
   450 455
<210> 14
   <211> 1431
   <212> DNA
   <213> Arthrobacter sp.
<400> 14
   atgggtctga ccgtgcagaa aattaattgg gaacaggtga aagaatggga tcgcaaatat      60
   ctgatgcgta cctttagcac ccagaatgaa tatcagccgg ttccgattga aagcaccgaa     120
   ggcgattatc tgattacacc gggtggtaca cgtctgctgg atttttttaa tcagctgtgc     180
   tgtgttaatc tgggtcagaa aaatcagaaa gtgaatgcag ccattaaaga agcactggat     240
   cgttacggtt ttgtgtggga tacctatgcc accgattata aagcaaaagc cgcaaaaatt     300
   attattgaag atattctggg agatgaagat tggcctggta aagttcgttt tgttagcacc     360
   ggtagcgaag cagttgaaac cgcactgaat attgcacgtc tgtataccaa tcgtccgctg     420
   gttgttaccc gtgaacatga ttatcatggt tggaccggtg gtgcagcaac cgttacccgt     480
   ctgcgtagct ttcgtagcgg tctggttggt gaaaatagcg aaagctttag cgcacagatt     540
   ccgggtagca gctgtagcag cgcagttctg atggcaccga gcagcaatac ctttcaggat     600
   agcaatggca attatctgaa agatgaaaac ggtgaactgc tgtctgttaa atatacccgt     660
   cgcatgattg aaaattatgg tccggaacag gttgcagcag ttattaccga agttagccag     720
   ggtgttggta gcaccatgcc tccgtatgaa tatgttccgc agattcgcaa aatgaccaaa     780
   gaactgggtg ttctgtggat ttctgatgaa gttctgaccg gttttggtcg taccggtaaa     840
   tggtttggct atcagcatta tggtgttcag ccggatatta ttaccatggg taaaggtctg     900
   agcagcagca gcctgcctgc aggtgcagtt gttgtgagca aagaaatcgc agcctttatg     960
   gataaacatc gttgggaaag cgttagcacc tatgcaggtc atcctgtggc tatggctgcc    1020
   gtttgtgcaa atctggaagt gatgatggaa gaaaatctgg ttgaacaggc caaaaatagc    1080
   ggtgaatata tccgtagcaa actggaactg ctgcaggaaa aacataaaag cattggcaat    1140
   tttgatggtt atggcctgct gtggattgtt gatattgtga atgccaaaac caaaaccccg    1200
   tatgttaaac tggatcgcaa ttttcgtcat ggcatgaatc cgaatcagat tccgacccag    1260
   atcattatgg aaaaagccct ggaaaaaggt gttctgattg gtggtgcaat gccgaatacc    1320
   atgcgtattg gtgcaagcct gaatgttagc cgtggcgata ttgataaagc aatggatgca    1380
   ctggattatg ccctggatta tctggaaagc ggtgaatggc agcagagcta a             1431
<210> 15
   <211> 476
   <212> PRT
   <213> Arthrobacter sp.
<400> 15
   Met Gly Leu Thr Val Gln Lys Ile Asn Trp Glu Gln Val Lys Glu Trp
   1               5                   10                  15
   Asp Arg Lys Tyr Leu Met Arg Thr Phe Ser Thr Gln Asn Glu Tyr Gln
               20                  25                  30
   Pro Val Pro Ile Glu Ser Thr Glu Gly Asp Tyr Leu Ile Thr Pro Gly
           35                  40                  45
   Gly Thr Arg Leu Leu Asp Phe Phe Asn Gln Leu Cys Cys Val Asn Leu
       50                  55                  60
   Gly Gln Lys Asn Gln Lys Val Asn Ala Ala Ile Lys Glu Ala Leu Asp
   65                  70                  75                  80
   Arg Tyr Gly Phe Val Trp Asp Thr Tyr Ala Thr Asp Tyr Lys Ala Lys
                   85                  90                  95
   Ala Ala Lys Ile Ile Ile Glu Asp Ile Leu Gly Asp Glu Asp Trp Pro
               100                 105                 110
   Gly Lys Val Arg Phe Val Ser Thr Gly Ser Glu Ala Val Glu Thr Ala
           115                 120                 125
   Leu Asn Ile Ala Arg Leu Tyr Thr Asn Arg Pro Leu Val Val Thr Arg
   130 135 140
   Glu His Asp Tyr His Gly Trp Thr Gly Gly Ala Ala Thr Val Thr Arg
   145 150 155 160
   Leu Arg Ser Phe Arg Ser Gly Leu Val Gly Glu Asn Ser Glu Ser Phe
   165 170 175
   Ser Ala Gln Ile Pro Gly Ser Ser Cys Ser Ser Ala Val Leu Met Ala
   180 185 190
   Pro Ser Ser Asn Thr Phe Gln Asp Ser Asn Gly Asn Tyr Leu Lys Asp
   195 200 205
   Glu Asn Gly Glu Leu Leu Ser Val Lys Tyr Thr Arg Arg Met Ile Glu
   210 215 220
   Asn Tyr Gly Pro Glu Gln Val Ala Ala Val Ile Thr Glu Val Ser Gln
   225 230 235 240
   Gly Val Gly Ser Thr Met Pro Pro Tyr Glu Tyr Val Pro Gln Ile Arg
   245 250 255
   Lys Met Thr Lys Glu Leu Gly Val Leu Trp Ile Ser Asp Glu Val Leu
   260 265 270
   Thr Gly Phe Gly Arg Thr Gly Lys Trp Phe Gly Tyr Gln His Tyr Gly
   275 280 285
   Val Gln Pro Asp Ile Ile Thr Met Gly Lys Gly Leu Ser Ser Ser Ser
   290 295 300
   Leu Pro Ala Gly Ala Val Val Val Ser Lys Glu Ile Ala Ala Phe Met
   305 310 315 320
   Asp Lys His Arg Trp Glu Ser Val Ser Thr Tyr Ala Gly His Pro Val
   325 330 335
   Ala Met Ala Ala Val Cys Ala Asn Leu Glu Val Met Met Glu Glu Asn
   340 345 350
   Leu Val Glu Gln Ala Lys Asn Ser Gly Glu Tyr Ile Arg Ser Lys Leu
   355 360 365
   Glu Leu Leu Gln Glu Lys His Lys Ser Ile Gly Asn Phe Asp Gly Tyr
   370 375 380
   Gly Leu Leu Trp Ile Val Asp Ile Val Asn Ala Lys Thr Lys Thr Pro
   385 390 395 400
   Tyr Val Lys Leu Asp Arg Asn Phe Arg His Gly Met Asn Pro Asn Gln
   405 410 415
   Ile Pro Thr Gln Ile Ile Met Glu Lys Ala Leu Glu Lys Gly Val Leu
   420 425 430
   Ile Gly Gly Ala Met Pro Asn Thr Met Arg Ile Gly Ala Ser Leu Asn
   435 440 445
   Val Ser Arg Gly Asp Ile Asp Lys Ala Met Asp Ala Leu Asp Tyr Ala
   450 455 460
   Leu Asp Tyr Leu Glu Ser Gly Glu Trp Gln Gln Ser
   465 470 475
<210> 16
   <211> 1359
   <212> DNA
   <213> Vibrio fluvialis
<400> 16
   atgaataaac cgcagagctg ggaagcacgt gcagaaacct atagcctgta tggctttacc 60
   gatatgccga gcctgcatca gcgtggcacc gttgttgtta cccatggtga aggtccgtat 120
   atcgtggatg ttaatggtcg tcgttatctg gatgcaaata gcggtctggg taatatggtt 180
   gccggttttg atcacaaagg cttaatcgat gcagcaaaag cacagtatga acgctttccg 240
   ggttatcatg cattttttgg tcgtatgagc gatcagaccg ttatgctgtc tgaaaaactg 300
   gttgaagtga gcccgtttga tagcggtcgt gtgttttatg ataatagcgg cagcgaagcg 360
   aatgatacca tggttaaaat gctgtggttt ctgcatgcag cagaaggtaa accgcagaaa 420
   cgtaaaattc tgacccgttg gaatgcatat catggtgtta ccgcagttag cgcaagcatg 480
   accggtaaac cgtataatag cgtttttggt ctgccgctgc ctggttttgt tcatctgacc 540
   tgtccgcatt attggcgtta tggtgaagaa ggtgaaaccg aagaacagtt tgttgcacgt 600
   ctggctcgtg aactggaaga aaccattcag cgtgaaggtg cagataccat tgcaggtttt 660
   tttgcagaac cggttatggg tgcaggtggt gttattccgc ctgccaaagg ttattttcag 720
   gccattctgc cgattctgcg caaatatgat attccggtga tctccgatga agttatttgt 780
   ggttttggtc gtaccggtaa tacctggggt tgtgtgacct atgattttac accggatgcc 840
   attattagca gcaaaaatct gaccgcaggc ttttttccga tgggtgcagt tattctgggt 900
   ccggaactga gcaaacgtct ggaaaccgca attgaagcca ttgaagaatt tccgcatggt 960
   tttaccgcaa gcggtcatcc ggttggttgc gctatcgctc tgaaagccat tgatgtggtg 1020
   atgaatgaag gtctggccga aaatgttcgt cgtctggctc cgcgttttga agaacgcctg 1080
   aaacatattg cagaacgtcc gaatattggt tatcgtggca ttggttttat gtgggcactg 1140
   gaagccgtta aagataaagc aagcaaaacc ccgtttgatg gtaatctgag cgttagcgaa 1200
   cgtattgcaa atacctgtac cgatctgggt ctgatttgtc gtccgctggg tcagagcgtt 1260
   gttctgtgtc cgccttttat tctgaccgaa gcccagatgg atgaaatgtt tgataaactg 1320
   gaaaaagccc tggataaagt ttttgcagaa gttgcataa 1359
<210> 17
   <211> 452
   <212> PRT
   <213> Vibrio fluvialis
<400> 17
   Met Asn Lys Pro Gln Ser Trp Glu Ala Arg Ala Glu Thr Tyr Ser Leu
   1               5                   10                  15
   Tyr Gly Phe Thr Asp Met Pro Ser Leu His Gln Arg Gly Thr Val Val
               20                  25                  30
   Val Thr His Gly Glu Gly Pro Tyr Ile Val Asp Val Asn Gly Arg Arg
           35                  40                  45
   Tyr Leu Asp Ala Asn Ser Gly Leu Gly Asn Met Val Ala Gly Phe Asp
       50                  55                  60
   His Lys Gly Leu Ile Asp Ala Ala Lys Ala Gln Tyr Glu Arg Phe Pro
   65                  70                  75                  80
   Gly Tyr His Ala Phe Phe Gly Arg Met Ser Asp Gln Thr Val Met Leu
                   85                  90                  95
   Ser Glu Lys Leu Val Glu Val Ser Pro Phe Asp Ser Gly Arg Val Phe
               100                 105                 110
   Tyr Asp Asn Ser Gly Ser Glu Ala Asn Asp Thr Met Val Lys Met Leu
           115                 120                 125
   Trp Phe Leu His Ala Ala Glu Gly Lys Pro Gln Lys Arg Lys Ile Leu
       130                 135                 140
   Thr Arg Trp Asn Ala Tyr His Gly Val Thr Ala Val Ser Ala Ser Met
   145                 150                 155                 160
   Thr Gly Lys Pro Tyr Asn Ser Val Phe Gly Leu Pro Leu Pro Gly Phe
   165 170 175
   Val His Leu Thr Cys Pro His Tyr Trp Arg Tyr Gly Glu Glu Gly Glu
   180 185 190
   Thr Glu Glu Gln Phe Val Ala Arg Leu Ala Arg Glu Leu Glu Glu Thr
   195 200 205
   Ile Gln Arg Glu Gly Ala Asp Thr Ile Ala Gly Phe Phe Ala Glu Pro
   210 215 220
   Val Met Gly Ala Gly Gly Val Ile Pro Pro Ala Lys Gly Tyr Phe Gln
   225 230 235 240
   Ala Ile Leu Pro Ile Leu Arg Lys Tyr Asp Ile Pro Val Ile Ser Asp
   245 250 255
   Glu Val Ile Cys Gly Phe Gly Arg Thr Gly Asn Thr Trp Gly Cys Val
   260 265 270
   Thr Tyr Asp Phe Thr Pro Asp Ala Ile Ile Ser Ser Lys Asn Leu Thr
   275 280 285
   Ala Gly Phe Phe Pro Met Gly Ala Val Ile Leu Gly Pro Glu Leu Ser
   290 295 300
   Lys Arg Leu Glu Thr Ala Ile Glu Ala Ile Glu Glu Phe Pro His Gly
   305 310 315 320
   Phe Thr Ala Ser Gly His Pro Val Gly Cys Ala Ile Ala Leu Lys Ala
   325 330 335
   Ile Asp Val Val Met Asn Glu Gly Leu Ala Glu Asn Val Arg Arg Leu
   340 345 350
   Ala Pro Arg Phe Glu Glu Arg Leu Lys His Ile Ala Glu Arg Pro Asn
   355 360 365
   Ile Gly Tyr Arg Gly Ile Gly Phe Met Trp Ala Leu Glu Ala Val Lys
   370 375 380
   Asp Lys Ala Ser Lys Thr Pro Phe Asp Gly Asn Leu Ser Val Ser Glu
   385 390 395 400
   Arg Ile Ala Asn Thr Cys Thr Asp Leu Gly Leu Ile Cys Arg Pro Leu
   405 410 415
   Gly Gln Ser Val Val Leu Cys Pro Pro Phe Ile Leu Thr Glu Ala Gln
   420 425 430
   Met Asp Glu Met Phe Asp Lys Leu Glu Lys Ala Leu Asp Lys Val Phe
   435 440 445
   Ala Glu Val Ala
   450
<210> 18
   <211> 1470
   <212> DNA
   <213> Pseudomonas putida
<400> 18
   atgagaattt caagcgtgtc atcagctcct gggagcgtga gttcctgctg tttgctgtct      60
   gatcaacttg gtgccgcagg acgcggctgt cgaacaacgg agaagcacat gagcgtcaac     120
   aacccgcaaa cccgtgaatg gcaaaccctg agcggggagc atcacctcgc gcccttcagc     180
   gactacaagc agctgaagga gaaggggccg cgcatcatca ccaaggccca gggtgtgcat     240
   ttgtgggata gcgaggggca caagatcctc gacggcatgg ctggcctgtg gtgcgtggca     300
   gtgggttatg gccgtgaaga actggttcag gcggcagaaa agcagatgcg cgagctgccg     360
   tactacaacc tgttcttcca gactgcccac ccgcctgcgc tggaactggc caaggcaatc     420
   accgatgtcg cgccgcaagg catgacccat gtgttcttca ccggctccgg ctccgaaggc     480
   aacgacaccg tgctgcgcat ggtccgccat tactgggcgc tgaaaggcaa gccacagaag     540
   cagaccatca tcggccgcat caacggctac cacggctcca ccgtggctgg tgccagcctg     600
   ggcggcatga gcggcatgca cgagcagggc ggcctgccga tcccgggcat cgtgcatatc     660
   ccgcagcctt actggttcgg cgaaggcggc gacatgaccc cggatgagtt cggggtgtgg     720
   gcggccgagc aattggagaa aaaaatcctc gaagtcggcg aagacaacgt cgcagccttc     780
   atcgccgagc cgatccaggg cgccggcggc gtgatcatcc cgccggaaac ctactggccg     840
   aaggtgaagg agattctcgc caagtacgac atcctgttcg tcgccgacga agtgatctgc     900
   ggtttcggcc gtaccggcga gtggttcggc tccgactact acgacctcaa gcctgacctg     960
   atgaccatcg ccaagggcct gacttccggt tacatcccca tgggcggtgt gatcgtgcgt    1020
   gacaccgtgg cccaggtact cagcgaaggc ggcgacttca accacggctt cacctactcc    1080
   ggccacccgg tagcggccgc cgtgggcctg gaaaacctgc gcatcctgcg tgacgagaaa    1140
   atcgtcgaga aggcgcgcag cgaagcggca ccgtatttgc aaaagcgttt gcgtgaactg    1200
   caggaccacc ctctggtggg cgaggtacgc ggcctgggcc tgctcggcgc gatcgagctg    1260
   gtcaaggaca aggccacccg cagccgttac gagggcaagg gcgtgggcat gatctgccgc    1320
   accttctgct tcgaaaacgg cctgatcatg cgcgcggtag gtgacaccat gatcattgcg    1380
   ccgccgctgg tgatcagcca tgcagagatc gacgaactgg tggaaaaggc acgtaaatgc    1440
   ctggacctga ccctcgaagc gatccgttga                                     1470
<210> 19
   <211> 489
   <212> PRT
   <213> Pseudomonas putida
<400> 19
   Met Arg Ile Ser Ser Val Ser Ser Ala Pro Gly Ser Val Ser Ser Cys
   1               5                   10                  15
   Cys Leu Leu Ser Asp Gln Leu Gly Ala Ala Gly Arg Gly Cys Arg Thr
               20                  25                  30
   Thr Glu Lys His Met Ser Val Asn Asn Pro Gln Thr Arg Glu Trp Gln
           35                  40                  45
   Thr Leu Ser Gly Glu His His Leu Ala Pro Phe Ser Asp Tyr Lys Gln
       50                  55                  60
   Leu Lys Glu Lys Gly Pro Arg Ile Ile Thr Lys Ala Gln Gly Val His
   65                  70                  75                  80
   Leu Trp Asp Ser Glu Gly His Lys Ile Leu Asp Gly Met Ala Gly Leu
                   85                  90                  95
   Trp Cys Val Ala Val Gly Tyr Gly Arg Glu Glu Leu Val Gln Ala Ala
               100                 105                 110
   Glu Lys Gln Met Arg Glu Leu Pro Tyr Tyr Asn Leu Phe Phe Gln Thr
           115                 120                 125
   Ala His Pro Pro Ala Leu Glu Leu Ala Lys Ala Ile Thr Asp Val Ala
       130                 135                 140
   Pro Gln Gly Met Thr His Val Phe Phe Thr Gly Ser Gly Ser Glu Gly
   145                 150                 155                 160
   Asn Asp Thr Val Leu Arg Met Val Arg His Tyr Trp Ala Leu Lys Gly
                   165                 170                 175
   Lys Pro Gln Lys Gln Thr Ile Ile Gly Arg Ile Asn Gly Tyr His Gly
               180                 185                 190
   Ser Thr Val Ala Gly Ala Ser Leu Gly Gly Met Ser Gly Met His Glu
           195                 200                 205
   Gln Gly Gly Leu Pro Ile Pro Gly Ile Val His Ile Pro Gln Pro Tyr
   210 215 220
   Trp Phe Gly Glu Gly Gly Asp Met Thr Pro Asp Glu Phe Gly Val Trp
   225 230 235 240
   Ala Ala Glu Gln Leu Glu Lys Lys Ile Leu Glu Val Gly Glu Asp Asn
   245 250 255
   Val Ala Ala Phe Ile Ala Glu Pro Ile Gln Gly Ala Gly Gly Val Ile
   260 265 270
   Ile Pro Pro Glu Thr Tyr Trp Pro Lys Val Lys Glu Ile Leu Ala Lys
   275 280 285
   Tyr Asp Ile Leu Phe Val Ala Asp Glu Val Ile Cys Gly Phe Gly Arg
   290 295 300
   Thr Gly Glu Trp Phe Gly Ser Asp Tyr Tyr Asp Leu Lys Pro Asp Leu
   305 310 315 320
   Met Thr Ile Ala Lys Gly Leu Thr Ser Gly Tyr Ile Pro Met Gly Gly
   325 330 335
   Val Ile Val Arg Asp Thr Val Ala Gln Val Leu Ser Glu Gly Gly Asp
   340 345 350
   Phe Asn His Gly Phe Thr Tyr Ser Gly His Pro Val Ala Ala Ala Val
   355 360 365
   Gly Leu Glu Asn Leu Arg Ile Leu Arg Asp Glu Lys Ile Val Glu Lys
   370 375 380
   Ala Arg Ser Glu Ala Ala Pro Tyr Leu Gln Lys Arg Leu Arg Glu Leu
   385 390 395 400
   Gln Asp His Pro Leu Val Gly Glu Val Arg Gly Leu Gly Leu Leu Gly
   405 410 415
   Ala Ile Glu Leu Val Lys Asp Lys Ala Thr Arg Ser Arg Tyr Glu Gly
   420 425 430
   Lys Gly Val Gly Met Ile Cys Arg Thr Phe Cys Phe Glu Asn Gly Leu
   435 440 445
   Ile Met Arg Ala Val Gly Asp Thr Met Ile Ile Ala Pro Pro Leu Val
   450 455 460
   Ile Ser His Ala Glu Ile Asp Glu Leu Val Glu Lys Ala Arg Lys Cys
   465 470 475 480
   Leu Asp Leu Thr Leu Glu Ala Ile Arg
   485
<210> 20
   <211> 1362
   <212> DNA
   <213> Pseudomonas putida
<400> 20
   atgagcgtca acaacccgca aacccgtgaa tggcaaaccc tgagcgggga gcaccatctc      60
   gcacctttca gtgactacaa gcagctgaag gagaaggggc cgcgcatcat caccaaggcc     120
   cagggggtgc atttgtggga cagcgagggg cacaagatcc tcgacggcat ggcgggcctg      180
   tggtgcgtgg cggtgggtta cggccgtgaa gagctggttc aggcagcaga aaagcagatg     240
   cgcgagctgc cgtactacaa cctgttcttc cagacggccc acccgcctgc actggaactg     300
   gccaaggcca ttaccgatgt ggcgcccgag ggcatgaccc atgtgttctt caccggctcc     360
   ggctccgaag gcaacgacac cgtgctgcgc atggtgcgcc actactgggc gttgaagggc     420
   aagccgcaca agcagaccat catcggccgt atcaacggct accacggctc caccttcgcc     480
   ggtgcttgcc tgggcggcat gagcggcatg cacgagcagg gcggcctgcc gatcccgggc     540
   atcgtgcaca tcccgcagcc gtactggttc ggcgaaggcg gtgacatgac cccggatgcg     600
   ttcggtatct gggcggccga acagctggag aagaaaatcc tcgaagtcgg cgaagacaac     660
   gtcgccgcct tcatcgccga gcctatccag ggcgcaggcg gcgtgatcat cccgccggaa     720
   acctactggc cgaaggtgaa ggagattctc gccaagtacg acatcctgtt cgttgccgac     780
   gaagtcatct gtggtttcgg ccgtaccggc gagtggttcg gctctgatta ctacgacctc     840
   aagcccgacc tgatgaccat cgccaagggc ctgacctccg gttacatccc catgggcggt     900
   gtgatcgtgc gtgacaaagt ggccaaggtg atcagcgaag gcggtgactt caaccacggc     960
   ttcacctatt cgggccaccc ggtagcggcg gcggtgggcc tggaaaacct gcgcatcctg    1020
   cgcgacgagc aaattgtcga gaaggcgcgt actgaagcgg caccgtattt gcaaaagcgt    1080
   ttgcgtgagc tgcaggacca cccgctggtg ggtgaagtgc gcggccttgg catgcttggc    1140
   gcgatcgagc tggtgaaaga caaggccacc cgcagccgtt acgagggcaa gggcgtgggc    1200
   atgatctgcc gcaccttctg cttcgaaaac ggcctgatca tgcgtgcggt gggtgacacc    1260
   atgatcatcg cgccgccgct ggtcatcagc catgcggaaa tcgacgaact ggtggaaaag    1320
   gcacgcaaat gcctcgacct gacccttgag gcgattcgat aa                       1362
<210> 21
   <211> 453
   <212> PRT
   <213> Pseudomonas putida
<400> 21
   Met Ser Val Asn Asn Pro Gln Thr Arg Glu Trp Gln Thr Leu Ser Gly
   1               5                   10                  15
   Glu His His Leu Ala Pro Phe Ser Asp Tyr Lys Gln Leu Lys Glu Lys
               20                  25                  30
   Gly Pro Arg Ile Ile Thr Lys Ala Gln Gly Val His Leu Trp Asp Ser
           35                  40                  45
   Glu Gly His Lys Ile Leu Asp Gly Met Ala Gly Leu Trp Cys Val Ala
       50                  55                  60
   Val Gly Tyr Gly Arg Glu Glu Leu Val Gln Ala Ala Glu Lys Gln Met
   65                  70                  75                  80
   Arg Glu Leu Pro Tyr Tyr Asn Leu Phe Phe Gln Thr Ala His Pro Pro
                   85                  90                  95
   Ala Leu Glu Leu Ala Lys Ala Ile Thr Asp Val Ala Pro Glu Gly Met
               100                 105                 110
   Thr His Val Phe Phe Thr Gly Ser Gly Ser Glu Gly Asn Asp Thr Val
           115                 120                 125
   Leu Arg Met Val Arg His Tyr Trp Ala Leu Lys Gly Lys Pro His Lys
       130                 135                 140
   Gln Thr Ile Ile Gly Arg Ile Asn Gly Tyr His Gly Ser Thr Phe Ala
   145                 150                 155                 160
   Gly Ala Cys Leu Gly Gly Met Ser Gly Met His Glu Gln Gly Gly Leu
                   165                 170                 175
   Pro Ile Pro Gly Ile Val His Ile Pro Gln Pro Tyr Trp Phe Gly Glu
               180                 185                 190
   Gly Gly Asp Met Thr Pro Asp Ala Phe Gly Ile Trp Ala Ala Glu Gln
           195                 200                 205
   Leu Glu Lys Lys Ile Leu Glu Val Gly Glu Asp Asn Val Ala Ala Phe
       210                 215                 220
   Ile Ala Glu Pro Ile Gln Gly Ala Gly Gly Val Ile Ile Pro Pro Glu
   225 230 235 240
   Thr Tyr Trp Pro Lys Val Lys Glu Ile Leu Ala Lys Tyr Asp Ile Leu
   245 250 255
   Phe Val Ala Asp Glu Val Ile Cys Gly Phe Gly Arg Thr Gly Glu Trp
   260 265 270
   Phe Gly Ser Asp Tyr Tyr Asp Leu Lys Pro Asp Leu Met Thr Ile Ala
   275 280 285
   Lys Gly Leu Thr Ser Gly Tyr Ile Pro Met Gly Gly Val Ile Val Arg
   290 295 300
   Asp Lys Val Ala Lys Val Ile Ser Glu Gly Gly Asp Phe Asn His Gly
   305 310 315 320
   Phe Thr Tyr Ser Gly His Pro Val Ala Ala Ala Val Gly Leu Glu Asn
   325 330 335
   Leu Arg Ile Leu Arg Asp Glu Gln Ile Val Glu Lys Ala Arg Thr Glu
   340 345 350
   Ala Ala Pro Tyr Leu Gln Lys Arg Leu Arg Glu Leu Gln Asp His Pro
   355 360 365
   Leu Val Gly Glu Val Arg Gly Leu Gly Met Leu Gly Ala Ile Glu Leu
   370 375 380
   Val Lys Asp Lys Ala Thr Arg Ser Arg Tyr Glu Gly Lys Gly Val Gly
   385 390 395 400
   Met Ile Cys Arg Thr Phe Cys Phe Glu Asn Gly Leu Ile Met Arg Ala
   405 410 415
   Val Gly Asp Thr Met Ile Ile Ala Pro Pro Leu Val Ile Ser His Ala
   420 425 430
   Glu Ile Asp Glu Leu Val Glu Lys Ala Arg Lys Cys Leu Asp Leu Thr
   435 440 445
   Leu Glu Ala Ile Arg
   450
<210> 22
   <211> 1359
   <212> DNA
   <213> Pseudomonas putida
<400> 22
   atgagtgaac agaattcgca gacccttgcc tggcaatcga tgagccgcga ccaccacctg      60
   gccccgttca gcgatgtcaa acaactggcc gagaaaggcc cgcgcatcat cacctcggcc     120
   aagggcgtgt acctgtggga cagcgagggc aacaagattc ttgatggcat ggcgggcctg     180
   tggtgcgtgg cggtgggcta cggtcgcgat gagctggccg aggttgccag ccagcagatg     240
   aagcagctgc cttactacaa cctgttcttc cagaccgcgc acccgcctgc gctggagctg     300
   gccaaggcaa tcgccgatgt ggcgccgcaa ggcatgaacc atgtgttctt caccggctcc     360
   ggttccgaag gcaacgacac cgtgctgcgc atggtgcgcc attactgggc gctgaagggc     420
   aagaagaaca agaacgtcat cattggccgc atcaacggct accacggctc gactgtggcg     480
   ggggcagcat tgggtggcat gagcggcatg caccagcagg gcggggtgat tccggatatc     540
   gtgcacatcc cgcagccata ttggttcggc gaaggcggcg acatgaccga ggccgacttc     600
   ggcgtgtggg cggccgagca gttggagaag aaaatcctcg aagttggcgt ggacaacgtt     660
   gccgccttca tcgccgagcc gatccagggc gccggtggtg tgatcattcc accgcaaacc     720
   tactggccga aggtgaagga aatcctggcc aggtacgaca tcctgttcgt tgccgacgaa     780
   gtgatttgcg gttttggccg caccggtgag tggttcggta ccgactacta cgacctcaag     840
   cccgacctga tgaccattgc caagggcctg acctctggtt acatccccat gggcggggtg     900
   atcgtgcgtg acgaagtggc caaggtcatc agcgaaggcg gcgacttcaa ccacggcttc     960
   acctattctg gccacccggt ggcggctgcg gtgggcctgg agaacctgcg catcctgcgt    1020
   gatgagcaga tcatccagca ggtgcacgat aaaaccgcgc cttatctgca acaacgcctg    1080
   cgcgaactgg ctgaccaccc gctggtaggc gaagtgcgcg gcctgggcat gctcggcgca    1140
   atcgagctgg tgaaagacaa ggccacccgc gccaggtacg aaggcaaagg tgtcggcatg    1200
   atctgccgcc agcactgctt cgacaacggc ctgatcatgc gcgccgtggg cgacaccatg    1260
   atcatcgcgc cgccgctggt gatcagcatc gaggagatcg acgaactggt tgaaaaagcc    1320
   cgcaagtgcc tggacctgac ctatgaggct gttcggtaa                           1359
<210> 23
   <211> 452
   <212> PRT
   <213> Pseudomonas putida
<400> 23
   Met Ser Glu Gln Asn Ser Gln Thr Leu Ala Trp Gln Ser Met Ser Arg
   1               5                   10                  15
   Asp His His Leu Ala Pro Phe Ser Asp Val Lys Gln Leu Ala Glu Lys
   20 25 30
   Gly Pro Arg Ile Ile Thr Ser Ala Lys Gly Val Tyr Leu Trp Asp Ser
   35 40 45
   Glu Gly Asn Lys Ile Leu Asp Gly Met Ala Gly Leu Trp Cys Val Ala
   50 55 60
   Val Gly Tyr Gly Arg Asp Glu Leu Ala Glu Val Ala Ser Gln Gln Met
   65 70 75 80
   Lys Gln Leu Pro Tyr Tyr Asn Leu Phe Phe Gln Thr Ala His Pro Pro
   85 90 95
   Ala Leu Glu Leu Ala Lys Ala Ile Ala Asp Val Ala Pro Gln Gly Met
   100 105 110
   Asn His Val Phe Phe Thr Gly Ser Gly Ser Glu Gly Asn Asp Thr Val
   115 120 125
   Leu Arg Met Val Arg His Tyr Trp Ala Leu Lys Gly Lys Lys Asn Lys
   130 135 140
   Asn Val Ile Ile Gly Arg Ile Asn Gly Tyr His Gly Ser Thr Val Ala
   145 150 155 160
   Gly Ala Ala Leu Gly Gly Met Ser Gly Met His Gln Gln Gly Gly Val
   165 170 175
   Ile Pro Asp Ile Val His Ile Pro Gln Pro Tyr Trp Phe Gly Glu Gly
   180 185 190
   Gly Asp Met Thr Glu Ala Asp Phe Gly Val Trp Ala Ala Glu Gln Leu
   195 200 205
   Glu Lys Lys Ile Leu Glu Val Gly Val Asp Asn Val Ala Ala Phe Ile
   210 215 220
   Ala Glu Pro Ile Gln Gly Ala Gly Gly Val Ile Ile Pro Pro Gln Thr
   225 230 235 240
   Tyr Trp Pro Lys Val Lys Glu Ile Leu Ala Arg Tyr Asp Ile Leu Phe
   245 250 255
   Val Ala Asp Glu Val Ile Cys Gly Phe Gly Arg Thr Gly Glu Trp Phe
   260 265 270
   Gly Thr Asp Tyr Tyr Asp Leu Lys Pro Asp Leu Met Thr Ile Ala Lys
           275                 280                 285
   Gly Leu Thr Ser Gly Tyr Ile Pro Met Gly Gly Val Ile Val Arg Asp
       290                 295                 300
   Glu Val Ala Lys Val Ile Ser Glu Gly Gly Asp Phe Asn His Gly Phe
   305                 310                 315                 320
   Thr Tyr Ser Gly His Pro Val Ala Ala Ala Val Gly Leu Glu Asn Leu
                   325                 330                 335
   Arg Ile Leu Arg Asp Glu Gln Ile Ile Gln Gln Val His Asp Lys Thr
               340                 345                 350
   Ala Pro Tyr Leu Gln Gln Arg Leu Arg Glu Leu Ala Asp His Pro Leu
           355                 360                 365
   Val Gly Glu Val Arg Gly Leu Gly Met Leu Gly Ala Ile Glu Leu Val
       370                 375                 380
   Lys Asp Lys Ala Thr Arg Ala Arg Tyr Glu Gly Lys Gly Val Gly Met
   385                 390                 395                 400
   Ile Cys Arg Gln His Cys Phe Asp Asn Gly Leu Ile Met Arg Ala Val
                   405                 410                 415
   Gly Asp Thr Met Ile Ile Ala Pro Pro Leu Val Ile Ser Ile Glu Glu
               420                 425                 430
   Ile Asp Glu Leu Val Glu Lys Ala Arg Lys Cys Leu Asp Leu Thr Tyr
           435                 440                 445
   Glu Ala Val Arg
       450
<210> 24
   <211> 1380
   <212> DNA
   <213> Chromobacterium violaceum
<400> 24
   atgcagaaac agcgtaccac ctctcagtgg cgtgaactgg atgcggcgca tcatctgcat     60
   ccgtttaccg ataccgcgag cctgaatcag gcgggtgcgc gtgtgatgac ccgtggcgaa    120
   ggcgtgtatc tgtgggatag cgaaggcaac aaaattattg atggcatggc gggcctgtgg    180
   tgcgtgaacg tgggctatgg ccgtaaagat tttgcggaag cggcgcgtcg tcagatggaa    240
   gaactgccgt tttataacac cttctttaaa accacccatc cggcggtggt ggaactgagc    300
   agcctgctgg ccgaagttac cccggcaggt tttgatcgtg tgttttatac caacagcggc    360
   agcgaaagcg tggataccat gattcgtatg gtgcgtcgtt attgggatgt gcagggcaaa    420
   ccggaaaaaa aaaccctgat tggccgttgg aacggctatc acggcagcac cattggcggt    480
   gcgagcctgg gcggcatgaa atatatgcat gaacagggcg atctgccgat tccgggcatg    540
   gcgcatattg aacagccgtg gtggtataaa catggcaaag atatgacccc ggatgaattt    600
   ggcgtggttg cggcgcgttg gctggaagaa aaaattctgg aaatcggcgc ggataaagtg    660
   gcggcgtttg tgggcgaacc gattcagggt gcgggcggtg tgattgttcc gccggcaacc    720
   tattggccgg aaattgaacg tatttgccgc aaatatgatg tgctgctggt tgcggatgaa    780
   gtgatttgcg gctttggccg taccggcgaa tggtttggcc atcagcattt tggctttcag    840
   ccggacctgt ttaccgcggc gaaaggcctg agcagcggct atctgccgat tggcgcggtg    900
   tttgtgggca aacgtgttgc ggaaggtctg attgcgggcg gtgattttaa ccatggcttt    960
   acctatagcg gccatccggt gtgtgcggcg gtggcgcatg cgaatgttgc ggcgctgcgt   1020
   gatgaaggca ttgtgcagcg tgtgaaagat gatattggcc cgtatatgca gaaacgttgg   1080
   cgtgaaacct ttagccgttt tgaacatgtg gatgatgtgc gtggcgtggg catggtgcag   1140
   gcgtttaccc tggtgaaaaa caaagcgaaa cgtgaactgt ttccggattt tggcgaaatt   1200
   ggcaccctgt gccgcgatat tttttttcgc aacaacctga ttatgcgtgc gtgcggcgat   1260
   cacattgtgt ctgcaccgcc gctggttatg acccgtgcgg aagtggatga aatgctggcc   1320
   gtggcggaac gttgcctgga agaatttgaa cagaccctga aagcgcgtgg cctggcctaa   1380
<210> 25
   <211> 459
   <212> PRT
   <213> Chromobacterium violaceum
<400> 25
   Met Gln Lys Gln Arg Thr Thr Ser Gln Trp Arg Glu Leu Asp Ala Ala
   1               5                   10                  15
   His His Leu His Pro Phe Thr Asp Thr Ala Ser Leu Asn Gln Ala Gly
               20                  25                  30
   Ala Arg Val Met Thr Arg Gly Glu Gly Val Tyr Leu Trp Asp Ser Glu
           35                  40                  45
   Gly Asn Lys Ile Ile Asp Gly Met Ala Gly Leu Trp Cys Val Asn Val
       50                  55                  60
   Gly Tyr Gly Arg Lys Asp Phe Ala Glu Ala Ala Arg Arg Gln Met Glu
   65                  70                  75                  80
   Glu Leu Pro Phe Tyr Asn Thr Phe Phe Lys Thr Thr His Pro Ala Val
                   85                  90                  95
   Val Glu Leu Ser Ser Leu Leu Ala Glu Val Thr Pro Ala Gly Phe Asp
               100                 105                 110
   Arg Val Phe Tyr Thr Asn Ser Gly Ser Glu Ser Val Asp Thr Met Ile
           115                 120                 125
   Arg Met Val Arg Arg Tyr Trp Asp Val Gln Gly Lys Pro Glu Lys Lys
       130                 135                 140
   Thr Leu Ile Gly Arg Trp Asn Gly Tyr His Gly Ser Thr Ile Gly Gly
   145                 150                 155                 160
   Ala Ser Leu Gly Gly Met Lys Tyr Met His Glu Gln Gly Asp Leu Pro
                   165                 170                 175
   Ile Pro Gly Met Ala His Ile Glu Gln Pro Trp Trp Tyr Lys His Gly
               180                 185                 190
   Lys Asp Met Thr Pro Asp Glu Phe Gly Val Val Ala Ala Arg Trp Leu
           195                 200                 205
   Glu Glu Lys Ile Leu Glu Ile Gly Ala Asp Lys Val Ala Ala Phe Val
       210                 215                 220
   Gly Glu Pro Ile Gln Gly Ala Gly Gly Val Ile Val Pro Pro Ala Thr
   225                 230                 235                 240
   Tyr Trp Pro Glu Ile Glu Arg Ile Cys Arg Lys Tyr Asp Val Leu Leu
                   245                 250                 255
   Val Ala Asp Glu Val Ile Cys Gly Phe Gly Arg Thr Gly Glu Trp Phe
               260                 265                 270
   Gly His Gln His Phe Gly Phe Gln Pro Asp Leu Phe Thr Ala Ala Lys
           275                 280                 285
   Gly Leu Ser Ser Gly Tyr Leu Pro Ile Gly Ala Val Phe Val Gly Lys
       290                 295                 300
   Arg Val Ala Glu Gly Leu Ile Ala Gly Gly Asp Phe Asn His Gly Phe
   305                 310                 315                 320
   Thr Tyr Ser Gly His Pro Val Cys Ala Ala Val Ala His Ala Asn Val
                   325                 330                 335
   Ala Ala Leu Arg Asp Glu Gly Ile Val Gln Arg Val Lys Asp Asp Ile
               340                 345                 350
   Gly Pro Tyr Met Gln Lys Arg Trp Arg Glu Thr Phe Ser Arg Phe Glu
           355                 360                 365
   His Val Asp Asp Val Arg Gly Val Gly Met Val Gln Ala Phe Thr Leu
       370                 375                 380
   Val Lys Asn Lys Ala Lys Arg Glu Leu Phe Pro Asp Phe Gly Glu Ile
   385                 390                 395                 400
   Gly Thr Leu Cys Arg Asp Ile Phe Phe Arg Asn Asn Leu Ile Met Arg
                   405                 410                 415
   Ala Cys Gly Asp His Ile Val Ser Ala Pro Pro Leu Val Met Thr Arg
               420                 425                 430
   Ala Glu Val Asp Glu Met Leu Ala Val Ala Glu Arg Cys Leu Glu Glu
           435                 440                 445
   Phe Glu Gln Thr Leu Lys Ala Arg Gly Leu Ala
       450                 455
<210> 26
   <211> 1368
   <212> DNA
   <213> Rhodobacter sphaeroides
<400> 26
   atgcgtgacg atgcgccgaa ttcctgggag tcgcgggccg acgcctcttc cttctacggc     60
   ttcaccgatc tgccctcggt gcaccagcgc gggacggtcg tgctgaccca cggcaagggg    120
   ccctacatct acgacgtgca cgggcgcgcc tatctcgacg cgaactcggg cctctggaac    180
   atggtcgcgg gcttcgatca tccggggctg atcgaggctg ccaaggcgca gtacgagcgc    240
   ttccccggct atcatgcctt tttcgggcgg atgtcggacc agacggtcat gctgagcgag    300
   aagctggtcg aggtctcgcc cttcgcgcgg ggccgggtct tctacaccaa ctccggctcc    360
   gaggcgaacg acacgatggt caagatgctc tggttcctcg gcgcggccga gggccacccg    420
   gagcggcgca agatcatcac ccgcgtgaac agctatcacg gcgtgaccgc cgtctcggcc    480
   tcgatgaccg gcaagcccta caacagtctc ttcgggctgc cgctgccggg cttcatccat    540
   gtgggctgcc cgcattactg gcgcttcggg caggcgggcg agaccgaggc cgagttcacc    600
   cagcggcttg cgcgcgagct cgaggccacc atcatcaagg aaggcccgga cacgatcgcg    660
   ggcttcttcg ccgagccggt gatgggtgcc ggtggggtga tcccgccctc cgagggctat    720
   ttccaggcgg tccagccggt gctgaagcgc tacggcatcc cgctcatcgc cgacgaggtg    780
   atctgcggct tcgggcgcac gggcaacacc tggggctgcc agacctacga tttcatgccc    840
   gacgggatca tcagctcgaa gaacatcacc gcgggcttct tcccgatggg cgcggtgatc    900
   ctcgggccgg agctggccga ccggctgcag gccgcttcgg aagcggtcga ggaattcccg    960
   catgggttca ccgcgagcgg ccatccggtc ggctgcgcca tcgcgctgaa ggccatcgat   1020
   gtggtgatga acgaggggct ggccgagaat gtccgcgccc tcacgccgaa attcgaggcg   1080
   gggctcgcct atctggccga gaacccgaac atcggcgaat ggcgcggcaa ggggctgatg   1140
   ggggcgctgg aagcggtgaa ggacaaggcc accaagaccc ccttcccggg cgacctctcg    1200
   gtgtcggagc ggattgccaa cagctgcacc gaccatgggc tgatctgccg gccgctcggc   1260
   cagtcgatcg tgctctgccc gcccttcatc atgacggagg cgcagatgga cgagatgttc   1320
   gagaagctgg gcgccgcgct aaaaaaggtc ttcgcagagg tcgcctga                1368
<210> 27
   <211> 455
   <212> PRT
   <213> Rhodobacter sphaeroides
<400> 27
   Met Arg Asp Asp Ala Pro Asn Ser Trp Glu Ser Arg Ala Asp Ala Ser
   1               5                   10                  15
   Ser Phe Tyr Gly Phe Thr Asp Leu Pro Ser Val His Gln Arg Gly Thr
               20                  25                  30
   Val Val Leu Thr His Gly Lys Gly Pro Tyr Ile Tyr Asp Val His Gly
           35                  40                  45
   Arg Ala Tyr Leu Asp Ala Asn Ser Gly Leu Trp Asn Met Val Ala Gly
       50                  55                  60
   Phe Asp His Pro Gly Leu Ile Glu Ala Ala Lys Ala Gln Tyr Glu Arg
   65                  70                  75                  80
   Phe Pro Gly Tyr His Ala Phe Phe Gly Arg Met Ser Asp Gln Thr Val
                   85                  90                  95
   Met Leu Ser Glu Lys Leu Val Glu Val Ser Pro Phe Ala Arg Gly Arg
               100                 105                 110
   Val Phe Tyr Thr Asn Ser Gly Ser Glu Ala Asn Asp Thr Met Val Lys
           115                 120                 125
   Met Leu Trp Phe Leu Gly Ala Ala Glu Gly His Pro Glu Arg Arg Lys
       130                 135                 140
   Ile Ile Thr Arg Val Asn Ser Tyr His Gly Val Thr Ala Val Ser Ala
   145                 150                 155                 160
   Ser Met Thr Gly Lys Pro Tyr Asn Ser Leu Phe Gly Leu Pro Leu Pro
                   165                 170                 175
   Gly Phe Ile His Val Gly Cys Pro His Tyr Trp Arg Phe Gly Gln Ala
               180                 185                 190
   Gly Glu Thr Glu Ala Glu Phe Thr Gln Arg Leu Ala Arg Glu Leu Glu
           195                 200                 205
   Ala Thr Ile Ile Lys Glu Gly Pro Asp Thr Ile Ala Gly Phe Phe Ala
       210                 215                 220
   Glu Pro Val Met Gly Ala Gly Gly Val Ile Pro Pro Ser Glu Gly Tyr
   225                 230                 235                 240
   Phe Gln Ala Val Gln Pro Val Leu Lys Arg Tyr Gly Ile Pro Leu Ile
                   245                 250                 255
   Ala Asp Glu Val Ile Cys Gly Phe Gly Arg Thr Gly Asn Thr Trp Gly
               260                 265                 270
   Cys Gln Thr Tyr Asp Phe Met Pro Asp Gly Ile Ile Ser Ser Lys Asn
           275                 280                 285
   Ile Thr Ala Gly Phe Phe Pro Met Gly Ala Val Ile Leu Gly Pro Glu
       290                 295                 300
   Leu Ala Asp Arg Leu Gln Ala Ala Ser Glu Ala Val Glu Glu Phe Pro
   305                 310                 315                 320
   His Gly Phe Thr Ala Ser Gly His Pro Val Gly Cys Ala Ile Ala Leu
                   325                 330                 335
   Lys Ala Ile Asp Val Val Met Asn Glu Gly Leu Ala Glu Asn Val Arg
               340                 345                 350
   Ala Leu Thr Pro Lys Phe Glu Ala Gly Leu Ala Tyr Leu Ala Glu Asn
           355                 360                 365
   Pro Asn Ile Gly Glu Trp Arg Gly Lys Gly Leu Met Gly Ala Leu Glu
       370                 375                 380
   Ala Val Lys Asp Lys Ala Thr Lys Thr Pro Phe Pro Gly Asp Leu Ser
   385 390 395 400
   Val Ser Glu Arg Ile Ala Asn Ser Cys Thr Asp His Gly Leu Ile Cys
   405 410 415
   Arg Pro Leu Gly Gln Ser Ile Val Leu Cys Pro Pro Phe Ile Met Thr
   420 425 430
   Glu Ala Gln Met Asp Glu Met Phe Glu Lys Leu Gly Ala Ala Leu Lys
   435 440 445
   Lys Val Phe Ala Glu Val Ala
   450 455
<210> 28
   <211> 1362
   <212> DNA
   <213> Paracoccus denitrificans
<400> 28
   atgaaccaac cgcaaagctg ggaagcccgg gccgagacct attcgctcta cggtttcacc 60
   gacatgccct cggtccatca gcggggcacg gtcgtcgtga cccatggcga ggggccctat 120
   atcgtcgatg tccatggccg ccgctatctg gatgccaatt cgggcctgtg gaacatggtc 180
   gcgggcttcg accacaaggg cctgatcgag gccgccaagg cgcaatacga ccgctttccc 240
   ggctatcacg cctttttcgg ccgcatgtcc gaccagaccg tgatgctgtc ggaaaagctg 300
   gtcgaggtct cgccattcga caacggccgg gtcttctata ccaattccgg ctccgaggcg 360
   aacgacacca tggtcaagat gctgtggttc ctgcatgccg ccgagggcaa gccgcaaaag 420
   cgcaagatcc tgacgcgctg gaacgcctat cacggcgtga ccgcggtttc ggcctcgatg 480
   accggcaagc cctacaactc ggtcttcggc ctgccgctgc ccggcttcat ccacctgacc 540
   tgcccgcatt actggcgcta tggcgaggaa ggcgagaccg aggcgcaatt cgtcgcccgc 600
   ctggcacgcg agcttgagga taccatcacc cgcgagggcg ccgacaccat cgccggcttc 660
   ttcgccgagc cggtgatggg cgcggggggg gtgatcccgc cggcgaaggg ttatttccag 720
   gccatcctgc cgatcttgcg caagtatgac atcccgatga tctcggacga ggtgatctgc 780
   ggcttcgggc gcaccggcaa cacctggggc tgcctgacct acgacttcat gcccgatgcg 840
   atcatctcgt ccaagaacct gactgcgggc ttcttcccga tgggcgccgt catcctcggg 900
   cccgacctcg ccaagcgggt cgaggccgcg gtcgaggcga tcgaggagtt cccgcacggc 960
   ttcaccgcct cgggccatcc ggtcggctgc gccatcgcgc tgaaggccat cgacgtggtg 1020
   atgaacgagg ggctggccga gaatgtccgc cgcctcgcac cccgcttcga ggcggggctg 1080
   aagcgcatcg ccgaccgccc gaacatcggc gaataccgcg gcatcggctt catgtgggcg 1140
   ctggaggcgg tcaaggacaa gccgaccaag acccccttcg acgccaatct ttcggtcagc 1200
   gagcgcatcg ccaatacctg caccgatctg gggctgatct gccggccgct gggccagtcc 1260
   atcgtgctgt gcccgccctt catcctgacc gaggcgcaga tggacgagat gttcgaaaag 1320
   ctggaaaagg cgctcgacaa ggtctttgcc gaggtggcct ga 1362
<210> 29
   <211> 453
   <212> PRT
   <213> Paracoccus denitrificans
<400> 29
   Met Asn Gln Pro Gln Ser Trp Glu Ala Arg Ala Glu Thr Tyr Ser Leu
   1 5 10 15
   Tyr Gly Phe Thr Asp Met Pro Ser Val His Gln Arg Gly Thr Val Val
   20 25 30
   Val Thr His Gly Glu Gly Pro Tyr Ile Val Asp Val His Gly Arg Arg
   35 40 45
   Tyr Leu Asp Ala Asn Ser Gly Leu Trp Asn Met Val Ala Gly Phe Asp
   50 55 60
   His Lys Gly Leu Ile Glu Ala Ala Lys Ala Gln Tyr Asp Arg Phe Pro
   65 70 75 80
   Gly Tyr His Ala Phe Phe Gly Arg Met Ser Asp Gln Thr Val Met Leu
   85 90 95
   Ser Glu Lys Leu Val Glu Val Ser Pro Phe Asp Asn Gly Arg Val Phe
   100 105 110
   Tyr Thr Asn Ser Gly Ser Glu Ala Asn Asp Thr Met Val Lys Met Leu
   115 120 125
   Trp Phe Leu His Ala Ala Glu Gly Lys Pro Gln Lys Arg Lys Ile Leu
   130 135 140
   Thr Arg Trp Asn Ala Tyr His Gly Val Thr Ala Val Ser Ala Ser Met
   145 150 155 160
   Thr Gly Lys Pro Tyr Asn Ser Val Phe Gly Leu Pro Leu Pro Gly Phe
   165 170 175
   Ile His Leu Thr Cys Pro His Tyr Trp Arg Tyr Gly Glu Glu Gly Glu
   180 185 190
   Thr Glu Ala Gln Phe Val Ala Arg Leu Ala Arg Glu Leu Glu Asp Thr
   195 200 205
   Ile Thr Arg Glu Gly Ala Asp Thr Ile Ala Gly Phe Phe Ala Glu Pro
   210 215 220
   Val Met Gly Ala Gly Gly Val Ile Pro Pro Ala Lys Gly Tyr Phe Gln
   225 230 235 240
   Ala Ile Leu Pro Ile Leu Arg Lys Tyr Asp Ile Pro Met Ile Ser Asp
   245 250 255
   Glu Val Ile Cys Gly Phe Gly Arg Thr Gly Asn Thr Trp Gly Cys Leu
   260 265 270
   Thr Tyr Asp Phe Met Pro Asp Ala Ile Ile Ser Ser Lys Asn Leu Thr
   275 280 285
   Ala Gly Phe Phe Pro Met Gly Ala Val Ile Leu Gly Pro Asp Leu Ala
   290 295 300
   Lys Arg Val Glu Ala Ala Val Glu Ala Ile Glu Glu Phe Pro His Gly
   305 310 315 320
   Phe Thr Ala Ser Gly His Pro Val Gly Cys Ala Ile Ala Leu Lys Ala
   325 330 335
   Ile Asp Val Val Met Asn Glu Gly Leu Ala Glu Asn Val Arg Arg Leu
   340 345 350
   Ala Pro Arg Phe Glu Ala Gly Leu Lys Arg Ile Ala Asp Arg Pro Asn
   355 360 365
   Ile Gly Glu Tyr Arg Gly Ile Gly Phe Met Trp Ala Leu Glu Ala Val
   370 375 380
   Lys Asp Lys Pro Thr Lys Thr Pro Phe Asp Ala Asn Leu Ser Val Ser
   385 390 395 400
   Glu Arg Ile Ala Asn Thr Cys Thr Asp Leu Gly Leu Ile Cys Arg Pro
   405 410 415
   Leu Gly Gln Ser Ile Val Leu Cys Pro Pro Phe Ile Leu Thr Glu Ala
   420 425 430
   Gln Met Asp Glu Met Phe Glu Lys Leu Glu Lys Ala Leu Asp Lys Val
   435 440 445
   Phe Ala Glu Val Ala
   450
<210> 30
   <211> 1380
   <212> DNA
   <213> Mesorhizobium loti
<400> 30
   atgctcaacc agtccaacga actcgccgcc tgggatcgcg accacttctt ccatccctcg 60
   acccatatgg gcacgcacgc acgcggcgaa agcccgacgc gcatcatggc cggcggtgaa 120
   ggcgtcaccg tctgggataa caatggcagg aagagcctgg atgcctttgc cggcctctat 180
   tgcgtcaatg tcggctacgg ccgccagaag atcgctgacg ccatcgccgc ccaggcgaaa 240
   aacctcgctt actaccacgc ctatgtcggc cacggcaccg aggcctcgat aacgctggcc 300
   aagatgatca tcgaccgcgc gccaaagggc atgtcgaggg tctatttcgg cctgtcgggc 360
   tcggatgcca acgaaaccaa catcaagctg atctggtact acaacaatgt gctgggacgg 420
   ccggaaaaga agaagatcat ctcgcgctgg cgcggctatc acggctcggg cgtgatgacc 480
   ggttcactga ccgggctcga cctgttccac aacgccttcg acctgccgcg cgcgccgatc 540
   ctgcataccg aggcgcccta ctatttccgc cgcgccgacc gctcgatgag cgaagagcag 600
   ttctcgcagt attgcgccga caagcttgag gagatgatcc tggccgaggg cccggaaacg 660
   gtcgctgcct tcatcggcga gccgattctc ggcactggcg gtatcgtgcc gccgccggct 720
   ggctactggg agaaaatcca ggcggtgctg aagaagtacg acgtgctgct ggtcgccgac 780
   gaggtggtga cgggcttcgg ccggctgggc accatgttcg gctccgacca ctatggtatc 840
   aagccggacc tgatcaccat cgccaagggc ctgacctcgg cctatgcgcc actgtcgggc 900
   gtcatcgtcg ccgacaagat gtggcaggtg ctggtcgagg gctccgacaa gctcggctcg 960
   ctcggccatg gctggaccta ttcggcgcat ccgatctgcg ttgccgccgg ggtagccaat 1020
   ctcgaactga tcgacgagat ggatctggtg acgaatgccc gcgagaccgg cgcctatttc 1080
   cgcgcagagc tggccaaagc ggtcggcggc cacaaacatg tcggcgacgt gcgcggcgac 1140
   ggcatgctgg cggcggtcga gttcgtcgcc gacaaggacg accgggtgtt cttcgacgcg 1200
   tcgcagaaga tcgggccgca agtggcgacg gcgcttgccg caagcggcgt catcggccgc 1260
   gccatgccgc agggtgacat cctcggcttc gccccgccgc tctgcctgac ccgcgaagaa 1320
   gccgacatcg tcgtttcgaa gacggctgat gcggtgaaga gtgtgtttgc caatctctaa 1380
<210> 31
   <211> 459
   <212> PRT
   <213> Mesorhizobium loti
<400> 31
   Met Leu Asn Gln Ser Asn Glu Leu Ala Ala Trp Asp Arg Asp His Phe
   1 5 10 15
   Phe His Pro Ser Thr His Met Gly Thr His Ala Arg Gly Glu Ser Pro
   20 25 30
   Thr Arg Ile Met Ala Gly Gly Glu Gly Val Thr Val Trp Asp Asn Asn
   35 40 45
   Gly Arg Lys Ser Leu Asp Ala Phe Ala Gly Leu Tyr Cys Val Asn Val
   50 55 60
   Gly Tyr Gly Arg Gln Lys Ile Ala Asp Ala Ile Ala Ala Gln Ala Lys
   65 70 75 80
   Asn Leu Ala Tyr Tyr His Ala Tyr Val Gly His Gly Thr Glu Ala Ser
   85 90 95
   Ile Thr Leu Ala Lys Met Ile Ile Asp Arg Ala Pro Lys Gly Met Ser
   100 105 110
   Arg Val Tyr Phe Gly Leu Ser Gly Ser Asp Ala Asn Glu Thr Asn Ile
   115 120 125
   Lys Leu Ile Trp Tyr Tyr Asn Asn Val Leu Gly Arg Pro Glu Lys Lys
   130 135 140
   Lys Ile Ile Ser Arg Trp Arg Gly Tyr His Gly Ser Gly Val Met Thr
   145 150 155 160
   Gly Ser Leu Thr Gly Leu Asp Leu Phe His Asn Ala Phe Asp Leu Pro
   165 170 175
   Arg Ala Pro Ile Leu His Thr Glu Ala Pro Tyr Tyr Phe Arg Arg Ala
   180 185 190
   Asp Arg Ser Met Ser Glu Glu Gln Phe Ser Gln Tyr Cys Ala Asp Lys
   195 200 205
   Leu Glu Glu Met Ile Leu Ala Glu Gly Pro Glu Thr Val Ala Ala Phe
   210 215 220
   Ile Gly Glu Pro Ile Leu Gly Thr Gly Gly Ile Val Pro Pro Pro Ala
   225 230 235 240
   Gly Tyr Trp Glu Lys Ile Gln Ala Val Leu Lys Lys Tyr Asp Val Leu
   245 250 255
   Leu Val Ala Asp Glu Val Val Thr Gly Phe Gly Arg Leu Gly Thr Met
   260 265 270
   Phe Gly Ser Asp His Tyr Gly Ile Lys Pro Asp Leu Ile Thr Ile Ala
   275 280 285
   Lys Gly Leu Thr Ser Ala Tyr Ala Pro Leu Ser Gly Val Ile Val Ala
   290 295 300
   Asp Lys Met Trp Gln Val Leu Val Glu Gly Ser Asp Lys Leu Gly Ser
   305 310 315 320
   Leu Gly His Gly Trp Thr Tyr Ser Ala His Pro Ile Cys Val Ala Ala
   325 330 335
   Gly Val Ala Asn Leu Glu Leu Ile Asp Glu Met Asp Leu Val Thr Asn
   340 345 350
   Ala Arg Glu Thr Gly Ala Tyr Phe Arg Ala Glu Leu Ala Lys Ala Val
   355 360 365
   Gly Gly His Lys His Val Gly Asp Val Arg Gly Asp Gly Met Leu Ala
   370 375 380
   Ala Val Glu Phe Val Ala Asp Lys Asp Asp Arg Val Phe Phe Asp Ala
   385 390 395 400
   Ser Gln Lys Ile Gly Pro Gln Val Ala Thr Ala Leu Ala Ala Ser Gly
   405 410 415
   Val Ile Gly Arg Ala Met Pro Gln Gly Asp Ile Leu Gly Phe Ala Pro
   420 425 430
   Pro Leu Cys Leu Thr Arg Glu Glu Ala Asp Ile Val Val Ser Lys Thr
   435 440 445
   Ala Asp Ala Val Lys Ser Val Phe Ala Asn Leu
   450 455
<210> 32
   <211> 1404
   <212> DNA
   <213> Rhodobacter sphaeroides
<400> 32
   atgacgcgga atgacgcgac gaatgctgcc ggagcggtgg gcgcggcgat gcgggatcac      60
   atcctcttgc ctgcacagga aatggcgaag ctcggcaagt ccgcgcagcc ggtgctgact     120
   catgccgagg gcatctatgt ccataccgag gacggccgcc gcctgatcga cgggccggcg     180
   ggcatgtggt gcgcgcaggt gggctacggc cgccgcgaga tcgtcgatgc catggcgcat     240
   caggcgatgg tgctgcccta tgcctcgccc tggtatatgg ccacgagccc cgcggcgcgg     300
   ctggcggaga agatcgccac gctgacgccg ggcgatctca accggatctt tttcaccacg     360
   ggcgggtcga ccgcggtgga cagcgcgctg cgcttctcgg aattctacaa caacgtgctg     420
   ggccggccgc agaagaagcg catcatcgtg cgctacgacg gctatcacgg ctcgacggcg     480
   ctcaccgccg cctgcaccgg ccgcaccggc aactggccga acttcgacat cgcgcaggac     540
   cggatctcgt tcctctcgag ccccaatccg cgccacgccg gcaaccgcag ccaggaggcg     600
   ttcctcgacg atctggtgca ggaattcgag gaccggatcg agagcctcgg ccccgacacg     660
   atcgcggcct tcctggccga gccgatcctc gcctcgggcg gcgtcattat tccgcccgca     720
   ggctatcatg cgcgcttcaa ggcgatctgc gagaagcacg acatcctcta tatctcggac     780
   gaggtggtga cgggcttcgg ccgttgcggc gagtggttcg cctcggagaa ggtgttcggg     840
   gtggtgccgg acatcatcac cttcgccaag ggcgtgacct cgggctatgt gccgctcggc     900
   ggccttgcga tctccgaggc ggtgctggcg cggatctcgg gcgagaatgc caagggaagc     960
   tggttcacca acggctatac ctacagcaat cagccggtgg cctgcgccgc ggcgcttgcc    1020
   aacatcgagc tgatggagcg cgagggcatc gtcgatcagg cgcgcgagat ggcggactat    1080
   ttcgccgcgg cgctggcttc gctgcgcgat ctgccgggcg tggcggaaac ccggtcggtg    1140
   ggcctcgtgg gttgcgtgca atgcctgctc gacccgaccc gggcggacgg cacggccgag    1200
   gacaaggcct tcaccctgaa gatcgacgag cgctgcttcg agctcgggct gatcgtgcgc    1260
   ccgctgggcg atctctgcgt gatctcgccg ccgctcatca tctcgcgcgc gcagatcgac    1320
   gagatggtcg cgatcatgcg gcaggccatc accgaagtga gcgccgccca cggtctgacc    1380
   gcgaaagaac cggccgccgt ctaa                                           1404
<210> 33
   <211> 467
   <212> PRT
   <213> Rhodobacter sphaeroides
<400> 33
   Met Thr Arg Asn Asp Ala Thr Asn Ala Ala Gly Ala Val Gly Ala Ala
   1 5 10 15
   Met Arg Asp His Ile Leu Leu Pro Ala Gln Glu Met Ala Lys Leu Gly
   20 25 30
   Lys Ser Ala Gln Pro Val Leu Thr His Ala Glu Gly Ile Tyr Val His
   35 40 45
   Thr Glu Asp Gly Arg Arg Leu Ile Asp Gly Pro Ala Gly Met Trp Cys
   50 55 60
   Ala Gln Val Gly Tyr Gly Arg Arg Glu Ile Val Asp Ala Met Ala His
   65 70 75 80
   Gln Ala Met Val Leu Pro Tyr Ala Ser Pro Trp Tyr Met Ala Thr Ser
   85 90 95
   Pro Ala Ala Arg Leu Ala Glu Lys Ile Ala Thr Leu Thr Pro Gly Asp
   100 105 110
   Leu Asn Arg Ile Phe Phe Thr Thr Gly Gly Ser Thr Ala Val Asp Ser
   115 120 125
   Ala Leu Arg Phe Ser Glu Phe Tyr Asn Asn Val Leu Gly Arg Pro Gln
   130 135 140
   Lys Lys Arg Ile Ile Val Arg Tyr Asp Gly Tyr His Gly Ser Thr Ala
   145 150 155 160
   Leu Thr Ala Ala Cys Thr Gly Arg Thr Gly Asn Trp Pro Asn Phe Asp
   165 170 175
   Ile Ala Gln Asp Arg Ile Ser Phe Leu Ser Ser Pro Asn Pro Arg His
   180 185 190
   Ala Gly Asn Arg Ser Gln Glu Ala Phe Leu Asp Asp Leu Val Gln Glu
   195 200 205
   Phe Glu Asp Arg Ile Glu Ser Leu Gly Pro Asp Thr Ile Ala Ala Phe
   210 215 220
   Leu Ala Glu Pro Ile Leu Ala Ser Gly Gly Val Ile Ile Pro Pro Ala
   225 230 235 240
   Gly Tyr His Ala Arg Phe Lys Ala Ile Cys Glu Lys His Asp Ile Leu
   245 250 255
   Tyr Ile Ser Asp Glu Val Val Thr Gly Phe Gly Arg Cys Gly Glu Trp
   260 265 270
   Phe Ala Ser Glu Lys Val Phe Gly Val Val Pro Asp Ile Ile Thr Phe
       275                 280                 285
   Ala Lys Gly Val Thr Ser Gly Tyr Val Pro Leu Gly Gly Leu Ala Ile
       290                 295                 300
   Ser Glu Ala Val Leu Ala Arg Ile Ser Gly Glu Asn Ala Lys Gly Ser
   305                 310                 315                 320
   Trp Phe Thr Asn Gly Tyr Thr Tyr Ser Asn Gln Pro Val Ala Cys Ala
                   325                 330                 335
   Ala Ala Leu Ala Asn Ile Glu Leu Met Glu Arg Glu Gly Ile Val Asp
               340                 345                 350
   Gln Ala Arg Glu Met Ala Asp Tyr Phe Ala Ala Ala Leu Ala Ser Leu
           355                 360                 365
   Arg Asp Leu Pro Gly Val Ala Glu Thr Arg Ser Val Gly Leu Val Gly
       370                 375                 380
   Cys Val Gln Cys Leu Leu Asp Pro Thr Arg Ala Asp Gly Thr Ala Glu
   385                 390                 395                 400
   Asp Lys Ala Phe Thr Leu Lys Ile Asp Glu Arg Cys Phe Glu Leu Gly
                   405                 410                 415
   Leu Ile Val Arg Pro Leu Gly Asp Leu Cys Val Ile Ser Pro Pro Leu
               420                 425                 430
   Ile Ile Ser Arg Ala Gln Ile Asp Glu Met Val Ala Ile Met Arg Gln
           435                 440                 445
   Ala Ile Thr Glu Val Ser Ala Ala His Gly Leu Thr Ala Lys Glu Pro
       450                 455                 460
   Ala Ala Val
   465